# FASCICULE DE BREVET EUROPEEN

(11) **EP 0 687 259 B1**
(45) Date de publication et mention de la délivrance du brevet: **16.04.1997**
(21) Numéro de dépôt: 94908385.1
(22) Date de dépôt: 28.02.1994
(51) Int. Cl.: C07D 305/14, A61K 31/335

(54) **NOUVEAUX TAXOIDES, LEUR PREPARATION ET LES COMPOSITIONS PHARMACEUTIQUES QUI LES CONTIENNENT**
Taxolderivate, deren Herstellung und diese enthaltende pharmazeutische Zusammenstellungen.
NOVEL TAXOIDS, PREPARATION THEREOF AND PHARMACEUTICAL COMPOSITIONS CONTAINING SAME

(30) Priorité: 02.03.1993 FR 9302370
(43) Date de publication de la demande: 20.12.1995
(73) Titulaire: RHONE-POULENC RORER S.A., 92160 Antony (FR)
(72) Inventeur: BOUCHARD, Hervé, F-94200 Ivry-sur-Seine (FR); BOURZAT, Jean-Dominique, F-94300 Vincennes (FR); COMMERCON, Alain, F-94400 Vitry-sur-Seine (FR); PULICANI, Jean-Pierre, F-92160 Anthony (FR)
(74) Mandataire: Pilard, Jacques
(86) Numéro de dépôt international: FR9400222
(87) Numéro de publication internationale: WO9420484

(56) Documents cités:
- EP-A- 0 253 738

## Description

La présente invention concerne un procédé de préparation de taxoïdes de formule générale : 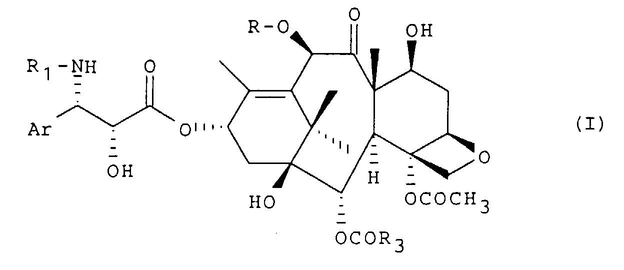 dans laquelle :
Ar représente un radical aryle ou un radical hétérocyclique aromatique éventuellement substitué,
R représente un atome d'hydrogène ou un radical acétyle ou alcoxyacétyle,
R₁ représente un radical benzoyle ou un radical R₂-O-CO- dans lequel R₂ représente :
   - un radical alcoyle droit ou ramifié contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone ou bicycloalcoyle contenant 7 à 11 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle, cyano, carboxy ou alcoyloxycarbonyle dont la partie alcoyle contient 1 à 4 atomes de carbone,
   - ou un radical phényle éventuellement substitué par un ou plusieurs atomes ou radicaux choisis parmi les atomes d'halogène et les radicaux alcoyles contenant 1 à 4 atomes de carbone, alcoyloxy contenant 1 à 4 atomes de carbone,
   - ou un radical hétérocyclyle saturé ou non saturé contenant 4 à 6 chaînons et éventuellement substitué par un ou plusieurs radicaux alcoyles contenant 1 à 4 atomes de carbone, et
R₃ représente
   - un radical alcoyle droit ou ramifié contenant 1 à 8 atomes de carbone, alcényle contenant 2 à 8 atomes de carbone, alcynyle contenant 2 à 8 atomes de carbone, cycloalcoyle contenant 3 à 6 atomes de carbone, cycloalcényle contenant 4 à 6 atomes de carbone ou bicycloalcoyle contenant 7 à 11 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle éventuellement substitué par un ou plusieurs substituants choisis parmi les radicaux cyano, carboxy ou alcoyloxycarbonyle dont la partie alcoyle contient 1 à 4 atomes de carbone,
   - ou un radical aryle éventuellement substitué par un ou plusieurs atomes ou radicaux, étant entendu que R₃ ne peut pas représenter un radical phényle non substitué,
   - ou un radical hétérocyclyle saturé ou non saturé contenant 4 à 6 chaînons éventuellement substitué par un ou plusieurs atomes ou radicaux, étant entendu que les radicaux cycloalcoyles, cycloalcényles ou bicycloalcoyles peuvent être éventuellement substitués par un ou plusieurs radicaux alcoyles contenant 1 à 4 atomes de carbone.

De préférence les radicaux aryles représentés par Ar et R₃ sont des radicaux phényles ou α- ou β-naphtyles éventuellement substitués par un ou plusieurs atomes ou radicaux choisis parmi les atomes d'halogène (fluor, chlore, brome, iode) et les radicaux alcoyles, alcényles, alcynyles, aryles, arylalcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, dialcoylcarbamoyle, cyano, nitro et trifluorométhyle, étant entendu que les radicaux alcoyles et les portions alcoyles des autres radicaux contiennent 1 à 4 atomes de carbone, que les radicaux alcényles et alcynyles contiennent 2 à 8 atomes de carbone et que les radicaux aryles sont des radicaux phényles ou α- ou β-naphtyles, et que le radical R₃ ne peut pas représenter un radical phényle non substitué.

De préférence les radicaux hétérocycliques représentés par Ar et R₃ sont des radicaux hétérocycliques aromatiques ayant 5 chaînons et contenant un ou plusieurs atomes, identiques ou différents, choisis parmi les atomes d'azote, d'oxygène ou de soufre, éventuellement substitués par un ou plusieurs substituants, identiques ou différents, choisis parmi les atomes d'halogène (fluor, chlore, brome, iode) et les radicaux alcoyles contenant 1 à 4 atomes de carbone, aryles contenant 6 à 10 atomes de carbone, alcoxy contenant 1 à 4 atomes de carbone, aryloxy contenant 6 à 10 atomes de carbone, amino, alcoylamino contenant 1 à 4 atomes de carbone, dialcoylamino dont chaque partie alcoyle contient 1 à 4 atomes de carbone, acylamino dont la partie acyle contient 1 à 4 atomes de carbone, alcoxycarbonylamino contenant 1 à 4 atomes de carbone, acyle contenant 1 à 4 atomes de carbone, arylcarbonyle dont la partie aryle contient 6 à 10 atomes de carbone, cyano, nitro, trifluorométhyle, carboxy, carbamoyle, alcoylcarbamoyle dont la partie alcoyle contient 1 à 4 atomes de carbone, dialcoylcarbamoyle dont chaque partie alcoyle contient 1 à 4 atomes de carbone ou alcoxycarbonyle dont la partie alcoxy contient 1 à 4 atomes de carbone.

Plus particulièrement, Ar représente un radical phényle, thiényle-2 ou -3 ou furyle-2 ou -3 ou thiazolyle-2 ou -4 ou -5 éventuellement substitué par un ou plusieurs atomes ou radicaux, identiques ou différents, choisis parmi les atomes d'halogène et les radicaux alcoyles, alcoxy, amino, alcoylamino, dialcoylamino, acylamino, alcoxy-carbonylamino et trifluorométhyle et R₃ représente un radical phényle substitué par un ou plusieurs atomes ou radicaux, identiques ou différents, choisis parmi les atomes d'halogène et les radicaux alcoyles, alcoxy, amino, alcoylamino, dialcoylamino, acylamino, alcoxycarbonylamino, nitro et trifluorométhyle ou un radical thiényle-2 ou -3 ou furyle-2 ou -3 ou thiazolyle-2 ou -4 ou -5 éventuellement substitué par un ou plusieurs atomes ou radicaux, identiques ou différents, choisis parmi les atomes d'halogène et les radicaux alcoyles, alcoxy; amino, alcoylamino, dialcoylamino, acylamino, alcoxycarbonyl-amino et trifluorométhyle.

Plus particulièrement encore, Ar représente un radical phényle éventuellement substitué par un atome de chlore ou de fluor, ou par un radical alcoyle (méthyle), alcoxy (méthoxy), dialcoylamino (diméthylamino), acylamino (acétylamino) ou alcoxycarbonylamino (tert-butoxycarbonylamino) ou carboxy ou carbamoyle, ou thiényle-2 ou -3 ou furyle-2 ou -3, thiazolyle-2 ou -4 ou -5 et R₃ représente un radical phényle substitué par un ou plusieurs atomes d'halogène ou par un radical alcoyle (éthyle, tert-butyle), alcoxy (méthoxy), nitro ou trifluorométhyle.

D'un intérêt encore plus particulier sont les produits de formule générale (I) dans laquelle Ar représente un radical phényle et R₁ représente un radical benzoyle ou tert.butoxycarbonyle et R₃ représente un radical fluoro-2 phényle, fluoro-3 phényle, fluoro-4 phényle, pentafluorophényle, éthyl-4 phényle, tert-butyl-4 phényle, nitro-3 phényle, nitro-4 phényle ou trifluorométhyle.

Les produits de formule générale (I) sont connus en particulier par la demande internationale PCT WO 94/17052.

Dans le brevet européen EP 0 253 738 sont décrits des produits de formule générale (I) dans laquelle R₃ représente un radical phényle non substitué.

Par ailleurs, il est connu, d'après la demande internationale PCT WO 94/08984 que la réduction électrolytique d'un dérivé du taxane conduit à produit hydroxylé en position 10.

Selon l'invention les taxoïdes de formule générale (I) peuvent être obtenus par estérification d'un produit de formule générale : 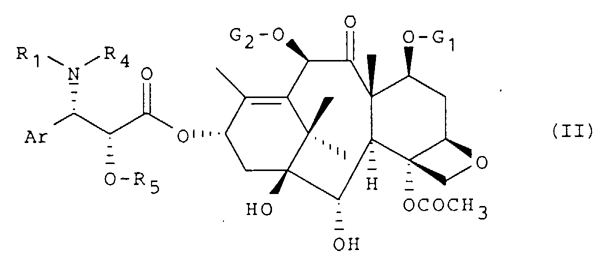 dans laquelle Ar et R₁ sont définis comme précédemment, et, ou bien R₄ représente un atome d'hydrogène et R₅ représente un groupement protecteur de la fonction hydroxy, ou bien R₄ et R₅ forment ensemble un hétérocycle saturé à 5 ou 6 chaînons, G₁ représente un groupement protecteur de la fonction hydroxy et G₂ représente un radical acétyle ou un groupement protecteur de la fonction hydroxy, au moyen d'un acide de formule générale :

R₃-CO-OH (III)

dans laquelle R₃ est défini comme précédemment, ou d'un dérivé activé de cet acide, pour obtenir un produit de formule générale : 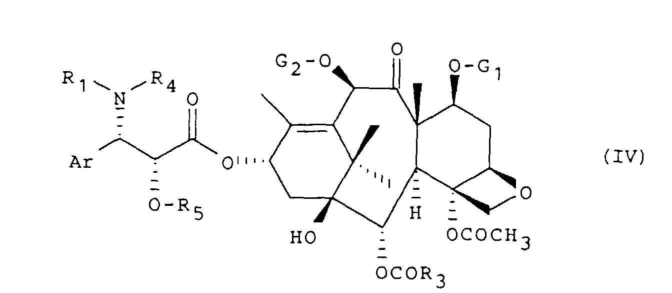 dans laquelle Ar, R₁, R₃, R₄, R₅, G₁ et G₂ sont définis comme précédemment, dont le remplacement des groupements protecteurs R₅, lorsque R₄ représente un atome d'hydrogène, ou bien R₄ et R₅, lorsque R₄ et R₅ forment ensemble un hétérocycle saturé à 5 ou 6 chaînons, G₁ et éventuellement G₂ par des atomes d'hydrogène conduit au produit de formule générale (I) en passant éventuellement, selon les significations de R₁, R₄ et R₅, par un produit de formule générale : 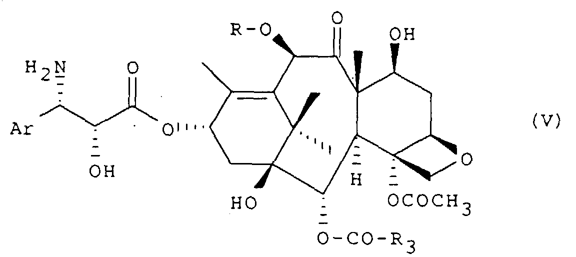 dans laquelle R est défini comme précédemment, qui est acylé au moyen de chlorure de benzoyle ou d'un produit de formule générale :

R₂-O-CO-X (VI)

dans laquelle R₂ est défini comme précédemment et X représente un atome d'halogène (fluor, chlore) ou un reste -O-R₂ ou -O-CO-O-R₂.

Lorsque R₄ représente un atome d'hydrogène, R₅ représente de préférence un radical méthoxyméthyle, éthoxy-1 éthyle, benzyloxyméthyle, triméthylsilyle, triéthylsilyle, (β-trimétylsilyléthoxy) méthyle ou tétrahydropyranyle. Lorsque R₄ et R₅ forment ensemble un hétérocycle, celui-ci est de préférence un cycle oxazolidine éventuellement mono-substitué ou gem-disubstitué en position -2.

Lorsque G₁ et G₂ représentent un groupement protecteur de la fonction hydroxy, ces groupements sont généralement différents. De préférence G₁ représente un radical trialcoylsilyle, dialcoylarylsilyle, alcoyldiarylsilyle ou triarylsilyle dans lesquels les parties alcoyles contiennent 1 à 4 atomes de carbone et les parties aryles sont de préférence des radicaux phényles et G₂ représente un radical alcoxyacétyle tel que méthoxyacétyle.

L'estérification du produit de formule générale (II) peut être effectuée en faisant réagir l'acide de formule générale (III) de préférence sous forme d'halogénure, tel que le chlorure, sur le produit de formule générale (II) préalablement métallé. La métallation est généralement effectuée au moyen d'un alcoylure de métal alcalin tel que le butyllithium en opérant dans un solvant organique inerte tel qu'un éther comme le tétrahydrofuranne à une température inférieure à -50°C et, de préférence au voisinage de -78°C. L'estérification est généralement effectuée en opérant à la même température dans le même solvant.

Selon la nature des groupements protecteurs du produit de formule générale (IV), leur remplacement par des atomes d'hydrogène peut être effectué de la manière suivante :
1) lorsque R₄ représente un atome d'hydrogène, R₅ est défini comme précédemment et G₁ représente un radical silylé et G₂ représente un radical acétyle, le remplacement des groupements protecteurs par des atomes d'hydrogène peut être effectué en traitant le produit de formule générale (IV) par un acide minéral (acide chlorhydrique, acide sulfurique, acide fluorhydrique) ou organique (acide formique, acide acétique, acide méthanesulfonique, acide trifluorométhanesulfonique, acide p.toluènesulfonique) utilisé seul ou en mélange en opérant dans un solvant organique choisi parmi les alcools, les éthers, les esters, les hydrocarbures aliphatiques, les hydrocarbures aliphatiques halogénés, les hydrocarbures aromatiques ou les nitriles à une température comprise entre -10 et 60°C.
2) lorsque R₄ et R₅ forment ensemble un hétérocycle saturé à 5 ou 6 chaînons et plus particulièrement un cycle oxazolidine de formule générale : 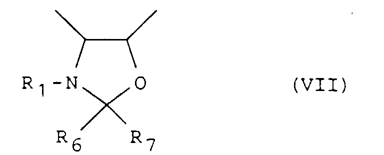 dans laquelle R₁ est défini comme précédemment, R₆ et R₇, identiques ou différents, représentent un atome d'hydrogène ou un radical alcoyle contenant 1 à 4 atomes de carbone, ou un radical aralcoyle dont la partie alcoyle contient 1 à 4 atomes de carbone et la partie aryle représente, de préférence, un radical phényle éventuellement substitué par un ou plusieurs radicaux alcoxy contenant 1 à 4 atomes de carbone, ou un radical aryle représentant, de préférence un radical phényle éventuellement substitué par un ou plusieurs radicaux alcoxy contenant 1 à 4 atomes de carbone, ou bien R₆ représente un radical alcoxy contenant 1 à 4 atomes de carbone ou un radical trihalométhyle tel que trichlorométhyle ou un radical phényle substitué par un radical trihalométhyle tel que trichlorométhyle et R₇ représente un atome d'hydrogène, ou bien R₆ et R₇ forment ensemble avec l'atome de carbone auquel ils sont liés un cycle ayant 4 à 7 chaînons, G₁ représente un radical silylé et G₂ représente un radical acétyle, le remplacement des groupements protecteurs par des atomes d'hydrogène peut être effectué, selon les significations de R₁, R₆ et R₇, de la manière suivante :
   a) lorsque R₁ représente un radical t.butoxycarbonyle, R₆ et R₇, identiques ou différents, représentent un radical alcoyle ou un radical aralcoyle (benzyle) ou aryle (phényle), ou bien R₆ représente un radical trihalométhyle ou un radical phényle substitué par un radical trihalométhyle et R₇ représente un atome d'hydrogène, ou bien R₆ et R₇ forment ensemble un cycle ayant de 4 à 7 chaînons, le traitement d'un produit de formule générale (IV) par un acide minéral ou organique éventuellement dans un solvant organique tel qu'un alcool conduit à un produit de formule générale (V) qui est acylé au moyen d'un produit de formule générale (VI). De préférence, le produit de formule générale (IV) est traité par l'acide formique à une température voisine de 20°C. De préférence, l'acylation du produit de formule générale (V) au moyen d'un produit de formule générale (VI) est effectuée dans un solvant organique inerte choisi parmi les esters tels que l'acétate d'éthyle, l'acétate d'isopropyle ou l'acétate de n.butyle et les hydrocarbures aliphatiques halogénés tels que le dichlorométhane ou le dichloro-1,2 éthane en présence d'une base minérale telle que le bicarbonate de sodium ou organique telle que la triéthylamine. La réaction est effectuée à une température comprise entre 0 et 50°C, de préférence voisine de 20°C.
   b) lorsque R₁ représente un radical benzoyle ou un radical R₂-O-CO- dans lequel R₂ est défini comme précédemment, R₆ représente un atome d'hydrogène ou un radical alcoxy contenant 1 à 4 atomes de carbone ou un radical phényle substitué par un ou plusieurs radicaux alcoxy contenant 1 à 4 atomes de carbone et R₇ représente un atome d'hydrogène, le remplacement des groupements protecteurs par des atomes d'hydrogène s'effectue en présence d'un acide minéral (acide chlorhydrique, acide sulfurique) ou organique (acide acétique, acide méthane-sulfonique, acide trifluorométhanesulfonique, acide p.toluènesulfonique) utilisé seul ou en mélange, en opérant dans un solvant organique choisi parmi les alcools, les éthers, les esters, les hydrocarbures aliphatiques, les hydrocarbures aliphatiques halogénés et les hydrocarbures aromatiques à une température comprise entre -10 et 60°C, de préférence entre 15 et 30°C. L'acide peut être utilisé en quantité catalytique ou stoechiométrique.
3) lorsque G₁ représente un radical silylé, G₂ représente un radical alcoxyacétyle et R₄ et R₅ sont définis comme au point 1) ci-dessus, on effectue d'abord le remplacement des groupements protecteurs G₁ et R₅ par des atomes d'hydrogène en opérant dans les conditions acides décrites au point 1) ci-dessus, puis remplace éventuellement le groupement protecteur G₂ par un atome d'hydrogène par traitement en milieu alcalin dans des conditions qui ne touchent pas au reste de la molécule. Généralement, le traitement alcalin est effectué par action de l'ammoniac en milieu hydro-alcoolique à une température voisine de 20°C ou par un halogénure de zinc tel que le bromure ou l'iodure de zinc dans le méthanol à une température voisine de 20°C.
4) lorsque G₁ représente un radical silylé, G₂ représente un radical alcoxyacétyle et R₄ et R₅ sont définis comme au point 2-a) ci-dessus, on effectue d'abord le remplacement du groupement protecteur G₁ par un traitement en milieu acide dans des conditions qui ne touchent pas au reste de la molécule, par exemple au moyen d'acide chlorhydrique dilué dans un alcool comme l'éthanol à une température voisine de 0°C, puis remplace éventuellement le groupement protecteur G₂ par un atome d'hydrogène dans les conditions décrites au point 3) ci-dessus, puis traite le produit de formule générale (V) obtenu dans les conditions de déprotection et d'acylation décrites au point 2-a) ci-dessus.
5) lorsque G₁ représente un radical silylé, G₂ représente un radical alcoxyacétyle et R₄ et R₅ sont définis comme au point 2-b) ci-dessus, on effectue d'abord le remplacement du groupement protecteur G₁ par un traitement en milieu acide dans des conditions qui ne touchent pas au reste de la molécule, par exemple au moyen d'acide chlorhydrique dilué dans un alcool comme l'éthanol à une température voisine de 0°C, puis remplace éventuellement le groupement protecteur G₂ par un atome d'hydrogène dans les conditions décrites au point 3) ci-dessus, puis traite le produit obtenu dans les conditions décrites au point 2-b) ci-dessus.

Selon l'invention, les produits de formule générale (II) peuvent être obtenus par réduction électrolytique d'un produit de formule générale : 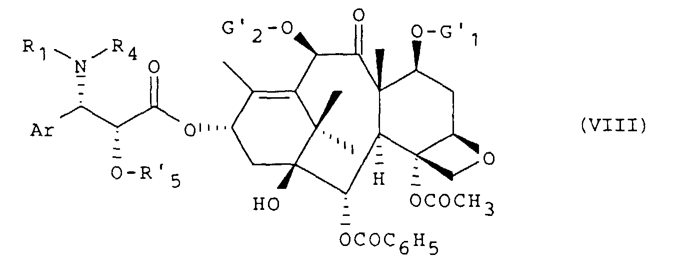 dans laquelle Ar, R₁ et R₄ sont définis comme précédemment, R'₅ représente un atome d'hydrogène ou un groupement protecteur de la fonction hydroxy, R₄ représentant un atome d'hydrogène, G'₁ représente un atome d'hydrogène ou un groupement protecteur de la fonction hydroxy et G'₂ représente un atome d'hydrogène, un radical acétyle ou un groupement protecteur de la fonction hydroxy, selon le schéma suivant : 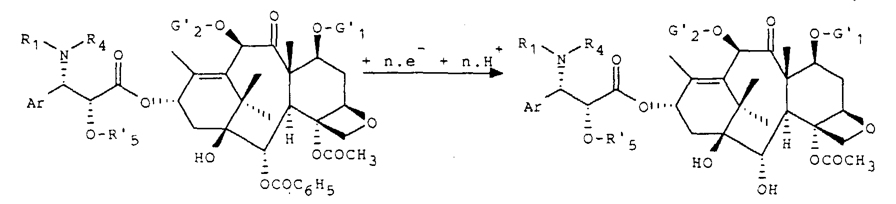 étant entendu que, lorsque n est égal à 2, le produit de coupure est le benzaldéhyde et, lorsque n est égal à 4, le produit de coupure est l'alcool benzylique, suivie éventuellement de la protection des fonctions hydroxy définies par -OR'₅, -O-G'₁ et -O-G'₂ pour obtenir le produit de formule générale (II).

La réduction électrolytique à partir du produit de formule générale (VIII) est réalisée dans un électrolyseur contenant un catholyte support dans lequel est dissous le produit de formule générale (VIII) à une concentration comprise entre 0,1 g/l et la saturation de la solution en produit de formule générale (VIII).

Préférentiellement, la réduction s'effectue dans un électrolyseur à diaphragme.

Selon un mode de mise en oeuvre du procédé selon l'invention, la réduction électrolytique est effectuée dans un électrolyseur comportant une cathode, un compartiment cathodique, un diaphragme séparateur, un compartiment anodique et une anode dont les caractéristiques sont les suivantes :
a) la cathode est constituée d'une nappe de mercure,
b) le compartiment cathodique contient le catholyte qui est constitué d'une solution du produit de formule générale (VIII) dans un milieu organique,
c) le diaphragme séparateur est constitué d'un matériau poreux tel qu'une plaque, un manchon ou une bougie de verre fritté ou de porcelaine ou par une membrane échangeuse d'ions, de préférence par une membrane échangeuse de cations,
d) le compartiment anodique contient l'anolyte constitué de préférence par le même solvant ou mélange de solvants et le même électrolyte support que celui qui est utilisé dans le compartiment cathodique,
e) l'anode est constituée par un matériau conducteur de l'électricité dont la nature n'est pas essentielle à la mise en oeuvre du procédé.

Généralement, l'anode est constituée par un matériau conducteur de l'électricité inattaquable dans les conditions de l'électrolyse tel que par exemple le platine poli, massif ou sur support conducteur, le graphite ou le carbone vitreux.

L'électrolyte support est constitué d'un sel d'ammonium quaternaire tel que l'acétate de tétraméthylammonium, l'acétate de tétraéthylammonium ou le tétrafluoroborate de tétraéthylammonium, ou leurs mélanges solubles dans le solvant ou le mélange de solvants.

Généralement, on utilise des solvants qui solubilisent facilement les produits de formule générale (II) et (VIII) et qui sont peu résistants tels que les alcools comme le méthanol, les nitriles comme l'acétonitrile ou les amides comme le diméthylformamide.

Le pH doit être compatible avec la stabilité du substrat. Le milieu doit être tamponné en ajoutant un acide faible tel que l'acide acétique à la solution du sel d'ammonium quaternaire.

Selon un mode préféré de mise en oeuvre du procédé, l'anode, la cathode et le diaphragme séparateur sont dans des plans parallèles horizontaux, la cathode étant constituée d'une nappe de mercure.

La température du bain d'électrolyse est généralement comprise entre 0 et 30°C. De préférence, elle est maintenue au dessous de 20°C.

L'électrolyse est effectuée à potentiel contrôlé qui peut être compris entre -1,85 et -2,10 volt par rapport à une électrode de référence saturée au calomel.

Il est nécessaire de désaérer la solution par barbotage d'un gaz inerte tel que l'argon pendant une dizaine de minutes avant le début de l'électrolyse, l'atmosphère inerte étant maintenue pendant toute la durée de l'électrolyse.

Pour obtenir un produit de formule générale (II) dans laquelle G₁, G₂ et R₅ représentent chacun un groupement protecteur de la fonction hydroxy, on effectue d'abord la protection des fonction hydroxy du produit de formule générale (VIII) définies par -O-G'₁ et -O-R'₅ avant de protéger la fonction hydroxy définie par -O-G'₂.

De préférence, la protection des fonctions hydroxy du produit de formule générale (VIII) définies par -O-G'₁ et -O-R'₅ sous forme d'éther silylé s'effectue généralement par action d'un halogénosilane de formule générale :

X-Si(R₈)₃ (IX)

dans laquelle X représente un atome d'halogène et les radicaux R₈, identiques ou différents, représentent un radical trialcoylsilyle, dialcoylarylsilyle, alcoyldiarylsilyle ou triarylsilyle dans lesquels les parties alcoyles contiennent 1 à 4 atomes de carbone et les parties aryles sont de préférence des radicaux phényles.

Généralement, l'action du produit de formule générale (IX) sur le produit de formule générale (VIII) dans laquelle G'₁, G'₂ et R'₅ représentent chacun un atome d'hydrogène est effectuée dans un solvant organique basique tel que la pyridine ou dans un solvant organique inerte tel que le dichlorométhane ou le chloroforme en présence d'une base organique telle que la triéthylamine ou la base de Hünig ou la pyridine à une température voisine de 20°C.

De préférence, la protection de la fonction hydroxy du produit de formule générale (VIII) défini par -O-G'₂ sous forme d'un radical alcoxyacétyle s'effectue par action d'un halogénure d'acide de formule générale :

R₉-O-CH₂-CO-Y (X)

dans laquelle R₉ représente un radical alcoyle contenant 1 à 4 atomes de carbone et Y représente un atome d'halogène en opérant dans un solvant organique basique tel que la pyridine à une température voisine de 20°C.

Selon l'invention, les produits de formule générale (II) peuvent aussi être obtenus par estérification d'un produit de formule générale : 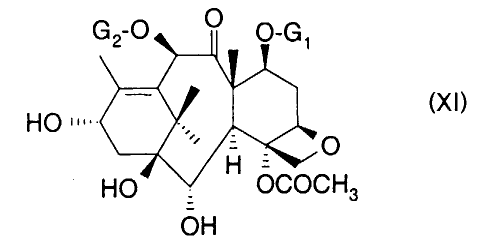 dans laquelle G₁ et G₂ sont définis comme précédemment, au moyen d'un acide de formule générale : 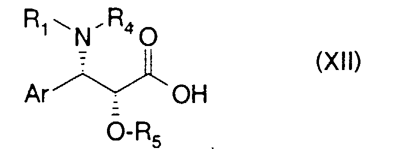 dans laquelle Ar, R₁, R₄ et R₅ sont définis comme précédemment, ou d'un dérivé de cet acide tel qu'un halogénure ou l'anhydride ou un anhydride mixte en opérant dans un solvant organique tel que le toluène en présence d'un agent d'activation tel qu'une aminopyridine comme la diméthylamino-4 pyridine ou la pyrrolidino-4 pyridine et éventuellement d'un agent de condensation tel qu'un imide comme le dicyclohexylcarbodiimide à une température comprise entre 0 et 90°C.

Le produit de formule générale (XI) peut être obtenu par réduction électrolytique d'un produit de formule générale : 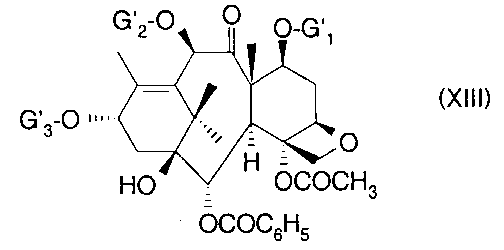 dans laquelle G'₁ et G'₂ sont définis comme précédemment et G'₃ représente un atome d'hydrogène ou un groupement protecteur de la fonction hydroxy identique à G'₁ en opérant dans des conditions identiques à celles utilisées pour la réduction électrolytique d'un produit de formule générale (VIII).

Selon les cas, il est nécessaire de remplacer sélectivement le groupement protecteur G'₃ par un atome d'hydrogène ou de protéger sélectivement les fonctions hydroxy en position -7 et -10 avant de procéder à l'estérification.

Les exemples suivants illustrent la présente invention.

### EXEMPLE 1

A une solution de 1,5 g de tert-butoxycarbonyl-3 diméthyl-2,2 phényl-4 oxazolidinecarboxylate-5-(4S,5R) de diacétoxy-4,10β dihydroxy-1,2α époxy-5β,20 oxo-9 triéthylsilyloxy-7β taxène-11 yle-13α dans 16 cm3 de tétrahydrofuranne anhydre, maintenue sous atmosphère d'argon, on ajoute goutte à goutte, à une température de -78°C, 1,lcm3 d'une solution 1,6M de n.butyllithium dans l'hexane. L'addition terminée, le mélange réactionnel est agité pendant 20 minutes à une température voisine de -78°C puis on ajoute goutte à goutte, à la même température, 0,3 cm3 de chlorure de fluoro-3 benzoyle. Le mélange réactionnel est maintenu sous agitation à -78°C pendant 3 heures puis on laisse la température remonter au voisinage de 0°C puis on ajoute 5 cm3 d'une solution aqueuse saturée de chlorure d'ammonium. La phase organique est séparée par décantation, lavée par 2 fois 10 cm3 d'eau distillée, séchée sur sulfate de magnésium, filtrée puis concentrée à sec sous pression réduite (2,7 kPa) à 40°C. On obtient 1,7 g d'une meringue blanche que l'on purifie par chromatographie sur 50 g de silice (0,063-0,2 mm) contenus dans une colonne de 2,5 cm de diamètre [éluant : dichlorométhane-méthanol (99-1 en volumes)] en recueillant des fractions de 3 cm3. Les fractions ne contenant que le produit cherché sont réunies et concentrées à sec sous pression réduite (2,7 kPa) à une température voisine de 40°C. On obtient ainsi 0,48 g de tert-butoxycarbonyl-3 diméthyl-2,2 phényl-4 oxazolidinecarboxylate-5-(4S,5R) de diacétoxy-4,10β époxy-5β,20 (fluoro-3 benzoyloxy)-2α hydroxy-1 oxo-9 triéthylsilyloxy-7β taxène-11 yle-13α sous forme d'une meringue blanche.

Une solution de 0,48 g de tert-butoxycarbonyl-3 diméthyl-2,2 phényl-4 oxazolidinecarboxylate-5-(4S,5R) de diacétoxy-4,10β époxy-5β,20 (fluoro-3 benzoyloxy)-2α hydroxy-1 oxo-9 triéthylsilyloxy-7β taxène-11 yle-13α dans 4,8 cm3 d'acide formique est agitée pendant 2,5 heures à une température voisine de 20°C puis concentrée à sec sous pression réduite (0,7 kPa puis 0,07 kPa) à 30°C. Le résidu est additionné de 20 cm3 de dichlorométhane puis de 20 cm3 d'une solution aqueuse saturée d'hydrogénocarbonate de sodium. La phase aqueuse est séparée par décantation et réextraite par 20 cm3 de dichlorométhane. Les phases organiques sont réunies, séchées sur sulfate de magnésium, filtrées puis concentrées à sec sous pression réduite (2,7 kPa) à 40°C. On obtient 0,33 g d'une meringue blanche que l'on purifie par chromatographie sur 20 g de silice (0,063-0,2 mm) contenus dans une colonne de 2 cm de diamètre [éluant : dichlorométhane-méthanol (98-2 en volumes)] en recueillant des fractions de 10 cm3. Les fractions ne contenant que le produit cherché sont réunies et concentrées à sec sous pression réduite (2,7 kPa) à une température voisine de 40°C. On obtient ainsi 0,091 g d'amino-3 hydroxy-2 phényl-3 propionate-(2R,3S) de diacétoxy-4,10β dihydroxy-1,7β époxy-5β,20 (fluoro-3 benzoyloxy)-2α oxo-9 taxène-11 yle-13α sous forme d'une meringue blanche.

A une solution de 87 mg d'amino-3 hydroxy-2 phényl-3 propionate-(2R,3S) de diacétoxy-4,10β dihydroxy-1,7β époxy-5β,20 (fluoro-3 benzoyloxy)-2α oxo-9 taxène-11 yle-13α dans 2,5 cm3 d'acétate d'éthyle, maintenue sous atmosphère d'argon, on ajoute 2,5 cm3 d'une solution saturée d'hydrogénocarbonate de sodium et 2,5 cm3 d'eau distillée puis en une seule fois 0,014 cm3 (14 µl) de chlorure de benzoyle. Le mélange réactionnel est agité pendant 15 minutes à une température voisine de 20°C. La phase aqueuse est séparée par décantation puis réextraite par 3 fois 3 cm3 d'acétate d'éthyle. Les phases organiques sont réunies, séchées sur sulfate de magnésium, filtrées puis concentrées à sec sous pression réduite (2,7 kPa) à 40°C. On obtient 88 mg d'une meringue blanche que l'on purifie par chromatographie sur 3 g de silice (0,063-0,2 mm) contenus dans une colonne de 1 cm de diamètre [éluant : dichlorométhane-méthanol (98-2 en volumes)] en recueillant des fractions de 1,5 cm3. Les fractions 14 à 21 sont réunies et concentrées à sec sous pression réduite (0,27 kPa) à 40°C. On obtient ainsi 80 mg de benzoylamino-3 hydroxy-2 phényl-3 propionate-(2R,3S) de diacétoxy-4,10β dihydroxy-1,7β époxy-5β,20 (fluoro-3 benzoyloxy)-2α oxo-9 taxène-11 yle-13α sous forme d'une meringue blanche dont les caractéristiques sont les suivantes :
- pouvoir rotatoire : [α]²⁰_{D} = -45° (c = 1,0 ; méthanol)
- spectre de R.M.N.: (400 MHz ; CDCl₃ ; δ en ppm).
1,15 (s, 3H : -CH₃ 16 ou 17) ; 1,25 (s, 3H : -CH₃ 16 ou 17) ; 1,69 (s, 3H : -CH₃ 19) ; 1,80 (s, 3H : -CH₃ 18) ; 1,89 [mt, 1H: -(CH)-H6] ; 1,94 (s, 1H : -OH 1) ; 2,26 (s, 3H : -COCH₃ en 10) ; 2,32 (d, J = 9 Hz, 2H : -CH₂- 14) ; 2,37 (s, 3H : -COCH₃ en 4) ; 2,53 (d, J = 4 Hz, 1H : -OH 7) ; 2,55 [mt, 1H : -(CH)-H 6) ; 3,69 (d, J = 5 Hz, 1H : -OH 2') ; 3,80 (d, J = 7 Hz, 1H : -H 3) ; 4,18 et 4,30 (2d, J = 8 Hz, 1H chacun : -(CH₂)- 20) ; 4,40 (mt, 1H : -H 7) ; 4,78 (dd, J = 5 et 3 Hz, 1H : -H 2') ; 4,96 (dd, J = 10 et 2 Hz, 1H : -H 5) ; 5,65 (d, J = 7 Hz, 1H : -H- 2) ; 5,77 (dd, J = 9,5 et 3 Hz, 1H : -H 3') ; 6,21 (t, J = 9 Hz, 1H : -H 13) ; 6,28 (s, 1H : -H 10) ; 7,00 (d, J = 9,5 Hz, 1H : -NHCO-) ; 7,30 à 7,50 [mt, 7H : -C₆H₅ en 3', -NHCOC₆H₅ (-H 4), -OCOC₆H₄F (-H 4)] ; 7,50 [t, J = 7,5 Hz, 2H : -NHCOC₆H₅(-H 3 et -H 5)]; 7,50 [mt, 1H : -OCOC₆H₄F(-H 5)] ; 7,63 [d, J = 7,5 Hz, 2H : -NHCOC₆H₅(-H 2 et -H 6)]; 7,81 (d large, J = 9 Hz, 1H: -OCOC₆H₄F(-H 2)] ; 7,94 (d, J = 7,5 Hz, 1H : -OCOC₆H₄F(-H 6)].

Le tert-butoxycarbonyl-3 diméthyl-2,2 phényl-4 oxazolidinecarboxylate-5-(4S,5R) de diacétoxy-4,10β dihydroxy-1,2α époxy-5β,20 oxo-9 triéthylsilyloxy-7β taxène-11 yle-13α peut être préparé de la manière suivante :

On procède à la réduction électrolytique du tert-butoxycarbonyl-3 diméthyl-2,2 phényl-4 oxazolidinecarboxylate-5-(4S,5R) de diacétoxy-4,10β hydroxy-1 benzoyloxy-2α époxy-5β,20 oxo-9 triéthylsilyloxy-7β taxène-11 yle-13α dans une cellule d'électrolyse possédant les caractéristiques suivantes :
- la cellule est un vase en verre de 50 cm3 divisé en 2 compartiments par une membrane échangeuse de cations,
- la cathode est une nappe de mercure dont la surface utile est de 12 cm2 environ,
- l'anode est une grille de platine,
- l'électrode de référence est une électrode au calomel saturée.

Dans le compartiment cathodique, on introduit 50 cm3 d'une solution contenant:
- tert-butoxycarbonyl-3 diméthyl-2,2 phényl-4 oxazolidinecarboxylate-5-(4S,5R) de diacétoxy-4,10β hydroxy-1 benzoyloxy-2α époxy-5β,20 oxo-9 triéthylsilyloxy-7β taxène-11 yle-13α 2,0 g
- méthanol 50 cm3
- acétate de tétraéthylammonium q.s.p. 0,2M
- acide acétique 0,28 cm3

Dans le compartiment anodique, on introduit environ 10 cm3 d'une solution de même composition ne contenant pas le substrat.

La cellule est plongée dans un bain de glace fondante. La température intérieure reste voisine de 10°C. Après désaération de la solution pendant 10 minutes par barbotage d'un courant d'argon, le potentiel de la cathode est fixé à -2,05 volt.

La consommation des ions H⁺ est compensée par addition de 0,114 cm3 d'acide acétique lorsque 1 faraday par mole de substrat est passé. On ajoute également 0,26 g d'acétate de tétraéthylammonium dans le compartiment anodique à chaque augmentation de la résistance électrique.

Après passage de 960 coulombs, l'électrolyse est arrêtée. Le solvant est évaporé sous pression réduite à une température inférieure à 35°C. Le résidu est repris par 50 cm3 d'eau. Le mélange réactionnel est extrait par 50 puis 2 fois 25 cm3 d'acétate d'éthyle. La phase organique est rincée par 50 cm3 d'une solution aqueuse d'hydrogénocarbonate de sodium puis séchée sur sulfate de magnésium. Après filtration et évaporation du solvant sous pression réduite à une température inférieure à 35°C, on isole 1,86 g d'un solide blanc qui est chromatographié sur 100 g de silice (0,063-0,2 mm) contenus dans une colonne de 24 mm de diamètre. On élue avec un mélange dichlorométhane-méthanol (99-1 en volumes) en recueillant des fractions de 6 cm3. Les fractions 140 à 220 contenant le produit attendu purifié sont réunies et concentrées à sec sous pression réduite (0,27 kPa). On obtient ainsi 0,77 g de tert-butoxycarbonyl-3 diméthyl-2,2 phényl-4 oxazolidinecarboxylate-5-(4S,5R) de diacétoxy-4,10β dihydroxy-1,2α époxy-5β,20 oxo-9 triéthylsilyloxy-7β taxène-11 yle-13α sous forme d'une meringue blanche.

Le tert-butoxycarbonyl-3 diméthyl-2,2 phényl-4 oxazolidinecarboxylate-5-(4S,5R) de diacétoxy-4,10β hydroxy-1 benzoyloxy-2α époxy-5β,20 oxo-9 triéthylsilyloxy-7β taxène-11 yle-13α peut être préparé dans les conditions décrites dans la demande internationale PCT WO 9209589.

### EXEMPLE 2

A une solution de 0,8 g de tert-butoxycarbonyl-3 diméthyl-2,2 phényl-4 oxazolidinecarboxylate-5- (4S,5R) d'acétoxy-4 dihydroxy-1,2α époxy-5β,20 méthoxyacétyloxy-10β oxo-9 triéthylsilyloxy-7β taxène-11 yle-13α dans 10 cm3 de tétrahydrofuranne anhydre, maintenue sous atmosphère d'argon, on ajoute goutte à goutte à une température voisine de -78°C, 0,65 cm3 d'une solution 1,6M de n.butyllithium dans l'hexane. L'addition terminée le milieu réactionnel est agité pendant 30 minutes à une température voisine de -78°C puis on ajoute goutte à goutte, en se maintenant à -78°C, 0,157 cm3 de chlorure de fluoro-3 benzoyle. L'addition terminée le milieu réactionnel est maintenu sous agitation à une température voisine de -78°C pendant 3 heures puis réchauffé jusqu'à une température voisine de 0°C et additionné de 2 cm3 d'une solution aqueuse saturée de chlorure d'ammonium. La phase aqueuse est séparée par décantation et réextraite par 3 fois 5 cm3 d'acétate d'éthyle. Les phases organiques sont réunies, séchées sur sulfate de magnésium, filtrées puis concentrées à sec sous pression réduite (2,7 kPa puis 0,7 kPa) à 40°C. On obtient 0,91 g d'une meringue blanche que l'on purifie par chromatographie sur 25 g de silice (0,063-0,2 mm) contenus dans une colonne de 1,5 cm de diamètre [éluant : dichlorométhane-méthanol (99-1 en volumes)] en recueillant des fractions de 3 cm3. Les fractions 35 à 42 sont réunies et concentrées à sec sous pression réduite (2,7 kPa) à une température voisine de 40°C. On obtient ainsi 0,51 g de tert-butoxycarbonyl-3 diméthyl-2,2 phényl-4 oxazolidinecarboxylate-5-(4S,5R) d'acétoxy-4 époxy-5β,20 (fluoro-3 benzoyloxy)-2α hydroxy-1 méthoxyacétyloxy-10β oxo-9 triéthylsilyloxy-7β taxène-11 yle-13α sous forme d'une meringue blanche.

On ajoute 0,1 g de tert-butoxycarbonyl-3 diméthyl-2,2 phényl-4 oxazolidinecarboxylate-5-(4S,5R) d'acétoxy-4 époxy-5β,20 (fluoro-3 benzoyloxy)-2α hydroxy-1 méthoxyacétyloxy-10β oxo-9 triéthylsilyloxy-7β taxène-11 yle-13α à 2 cm3 d'une solution éthanolique 0,1N d'acide chlorhydrique. La solution obtenue est agitée pendant 17 heures à une température voisine de 0°C puis additionnée de 10 cm3 de dichlorométhane et de 5 cm3 d'une solution aqueuse saturée d'hydrogénocarbonate de sodium. Après agitation la phase organique est séparée par décantation, séchée sur sulfate de magnésium, filtrée puis concentrée à sec sous pression réduite (2,7 kPa) à 40°C. On obtient 120 mg d'une meringue blanche que l'on purifie par chromatographie sur 3 g de silice (0,04-0,063 mm) contenus dans une colonne de 1 cm de diamètre [éluant : dichlorométhane-méthanol (99-1 en volumes)] en recueillant des fractions de 1 cm3. Les fractions 31 à 38 sont réunies et concentrées à sec sous pression réduite (2,7 kPa puis 0,07 kPa) à une température voisine de 40°C. On obtient ainsi 41 mg de tert-butoxycarbonyl-3 diméthyl-2,2 phényl-4 oxazolidinecarboxylate-5- (4S,5R) d'acétoxy-4 dihydroxy-1,7β époxy-5βb,20 (fluoro-3 benzoyloxy)-2α méthoxyacétyloxy-10β oxo-9 taxène-11 yle-13α sous forme d'une meringue blanche.

A une solution de 40 mg de tert-butoxycarbonyl-3 diméthyl-2,2 phényl-4 oxazolidinecarboxylate-5- (4S,5R) d'acétoxy-4 dihydroxy-1,7β époxy-5β,20 (fluoro-3 benzoyloxy)-2α méthoxyacétyloxy-10β oxo-9 taxène-11 yle-13α dans 1,5 cm3 de méthanol on ajoute, à une température voisine de 20°C, 0,5 cm3 d'une solution aqueuse 1M d'ammoniac. Le milieu réactionnel est agité pendant 1 heure à une température voisine de 20°C puis concentré à sec sous pression réduite (2,7 kPa puis 0,7 kPa) à 20°C. Le solide résiduel est purifié par chromatographie sur 1,5 g de silice (0,04-0,063 mm) contenus dans une colonne de 1 cm de diamètre [éluant : dichlorométhane-méthanol (98-2 en volumes)] en recueillant des fractions de 1 cm3. Les fractions 62 à 76 sont réunies et concentrées à sec sous pression réduite (2,7 kPa) à 40°C. On obtient ainsi 12 mg de tert-butoxycarbonyl-3 diméthyl-2,2 phényl-4 oxazolidinecarboxylate-5-(4S,5R) d'acétoxy-4 époxy-5β,20 (fluoro-3 benzoyloxy)-2α oxo-9 trihydroxy-1,7β,10β taxène-11 yle-13α sous forme d'une meringue blanche.

Une solution de 12 mg de tert-butoxycarbonyl-3 diméthyl-2,2 phényl-4 oxazolidinecarboxylate-5-(4S,5R) d'acétoxy-4 époxy-5β,20 (fluoro-3 benzoyloxy)-2α oxo-9 trihydroxy-1,7β,10β taxène-11 yle-13α dans 0,12 cm3 d'acide formique est agitée pendant 2,5 heures à une température voisine de 20°C puis additionnée de 10 cm3 de dichlorométhane et de 0,5 cm3 d'une solution aqueuse saturée d'hydrogénocarbonate de sodium. La phase aqueuse est séparée par décantation et réextraite par 1 cm3 de dichlorométhane. Les phases organiques sont réunies, séchées sur sulfate de magnésium, filtrées puis concentrées à sec sous pression réduite (2,7 kPa puis 0,7 kPa) à 40°C. On obtient ainsi 10 mg d'amino-3 hydroxy-2 phényl-3 propionate-(2R,3S) d'acétoxy-4 époxy-5β,20 (fluoro-3 benzoyloxy)-2α oxo-9 trihydroxy-1,7β,10β taxène-11 yle-13α sous forme d'une meringue blanche.

A une solution de 10 mg d'amino-3 hydroxy-2 phényl-3 propionate-(2R,3S) d'acétoxy-4 époxy-5β,20 (fluoro-3 benzoyloxy)-2α oxo-9 trihydroxy-1,7β,10β taxène-11 yle-13α dans 2 cm3 de tétrahydrofuranne, maintenue sous atmosphère d'argon, on ajoute à une température voisine de 20°C, 1,4 mg d'hydrogénocarbonate de sodium et 3,6 mg de dicarbonate de di.tert-butyle. Le mélange réactionnel est agité pendant 8 heures à une température voisine de 20°C puis concentré à sec sous pression réduite (2,7 kPa) à une tempérture voisine de 30°C. Le solide résiduel est additionné de 2 cm3 de dichlorométhane et de 1 cm3 d'eau distillée. Après agitation, la phase organique est séparée par décantation, séchée sur sulfate de magnésium, filtrée puis concentrée à sec sous pression réduite (2,7 kPa) à 40°C. On obtient 21 mg d'une meringue blanche que l'on purifie par chromatographie sur gel de silice déposé sur plaque (gel de 1 mm d'épaisseur ; plaque de 20 x 20 cm) par fraction de 10 mg. Après localisation aux rayons U.V. de la zone correspondant au produit cherché adsorbé, cette zone est grattée et la silice recueillie est lavée sur verre fritté par 10 fois 2 cm3 de dichlorométhane et par 5 fois 1 cm3 de méthanol. Les filtrats sont réunis et concentrés à sec sous pression réduite (0,27 kPa) à 40°C. On obtient ainsi 4 mg de tert-butoxycarbonylamino-3 hydroxy-2 phényl-3 propionate-(2R,3S) d'acétoxy-4 époxy-5β,20 (fluoro-3 benzoyloxy)-2α oxo-9 trihydroxy-1,7β,10β taxène-11 yle-13α sous forme d'une meringue blanche dont les caractéristiques sont les suivantes :
- spectre de R.M.N. : (300 MHz ; CDCl₃ ; δ en ppm)
   1,15 (s, 3H : -CH₃ 16 ou 17) ; 1,25 (s, 3H : -CH₃ 16 ou 17) ; 1,37 [s, 9H : -C(CH₃)₃] ; 1,78 (s, 3H : -CH₃ 19) ; 1,82 (s, 1H : -OH en 1) ; 1,87 (s, 3H : -CH₃ 18) ; 1,87 [mt, 1H : -(CH)-H 6] ; 2,27 (d, J = 9 Hz, 2H : -CH₂- 14) ; 2,37 (s, 3H : -COCH₃) ; 2,60 [mt, 1H : -(CH)-H 6] ; 3,93 (d, J = 7 Hz, 1H : -H 3) ; 4,18 (d, J = 8 Hz, 1H : -(CH)-H 20) ; 4,23 (mt, 1H : -H 7) ; 4,32 [d, J = 8 Hz, 1H : -(CH)-H 20] ; 4,61 (s large, 1H : -H 2') ; 4,96 (d large, J = 10 Hz, 1H : -H 5) ; 5,21 (s, 1H : -H 10) ; 5,33 (d large, J = 10 Hz, 1H: -H 3') ; 5,41 (d, J = 10 Hz, 1H: -CONH-) ; 5,67 (d, J = 7 Hz, 1H : -H 2) ; 6,21 (t, J = 9 Hz, 1H : -H 13) ; 7,30 à 7,50 [mt, 6H : -C₆H₅ en 3', -OCOC₆H₄F(-H 4)] ; 7,51 [mt, 1H: -OCOC₆H₄F(-H 5)] ; 7,81 [d large, J = 9 Hz, 1H : -OCOC₆H₄F(-H 2)] ; 7,92 [d, J = 7,5 Hz, 1H : -OCOC₆H₄F(-H 6)].

Le tert-butoxycarbonyl-3 diméthyl-2,2 phényl-4 oxazolidinecarboxylate-5-(4S,5R) d'acétoxy-4 dihydroxy-1,2α époxy-5β,20 méthoxyacétyloxy-10β oxo-9 triéthylsilyloxy-7β taxène-11 yle-13α peut être préparé de la manière suivante :

On procède à la réduction électrolytique du tert-butoxycarbonyl-3-diméthyl-2,2 phényl-4 oxazolidinecarboxylate-5-(4S,5R) d'acétoxy-4 hydroxy-1 benzoyloxy-2α époxy-5β,20 méthoxyacétyloxy-10β oxo-9 triéthylsilyloxy-7β taxène-11 yle-13α dans une cellule d'électrolyse possédant les caractéristiques suivantes :
- la cellule est un vase de verre de 10 cm3 divisé en 2 compartiments par une membrane échangeuse de cations,
- la cathode est une nappe de mercure dont la surface utile est de 4 cm2 environ,
- l'anode est une grille de platine,
- l'électrode de référence est une électrode au calomel saturée.

Dans le compartiment cathodique, on introduit 50 cm3 d'une solution contenant :
- tert-butoxycarbonyl-3 diméthyl-2,2 phényl-4 oxazolidinecarboxylate-5-(4S,5R) d' acétoxy-4 méthoxyacétyloxy-10β hydroxy-1 benzoyloxy-2α époxy-5β,20 oxo-9 triéthylsilyloxy-7β taxène-11 yle-13α 0,401 g
- méthanol 27 cm3
- tétrafluoroborate de tétraéthylammonium 0,1M
- acétate de tétrabutylammonium q.s.p. 0,02M
- acide acétique 0,02M

Dans le compartiment anodique, on introduit environ 10 cm3 d'une solution d'acide chlorhydrique environ 0,2M dans le méthanol.

La cellule est plongée dans un bain de glace fondante. La température intérieure reste voisine de 7°C. Après désaération de la solution pendant 10 minutes par barbotage d'un courant d'argon, le potentiel de la cathode est fixé à -1,9 volt puis à -2,0 volt après le passage de 150 coulombs.

Lorsque la résistance électrique augmente, on ajoute du méthanol chlorhydrique 3,8M dans le compartiment anodique.

Après passage de 450 coulombs, l'électrolyse est arrêtée. Le solvant est évaporé sous pression réduite à une température inférieure à 35°C. Le résidu est repris par 25 cm3 d'acétate d'éthyle puis par 25 cm3 d'eau permutée. La phase aqueuse est séparée par décantation et est extraite par 2 fois 12,5 cm3 d'acétate d'éthyle. La phase organique est rincée par 25 cm3 de tampon phosphate de sodium à pH 7,4 puis séchée sur sulfate de magnésium. Après filtration et évaporation du solvant sous pression réduite à une température inférieure à 35°C, on isole 0,355 g de meringue blanchâtre qui est chromatographiée sur 10 g de silice (0,04-0,063 mm) contenus dans une colonne de 15 mm de diamètre. On élue avec un mélange dichlorométhane-méthanol (98-2 en volumes) en recueillant des fractions de 15 cm3. Les fractions 21 à 25 contenant le produit attendu purifié sont réunies et concentrées à sec sous pression réduite (0,27 kPa) à une température inférieure à 35°C. On obtient ainsi, avec un rendement de 55 %, 195 mg de tert-butoxycarbonyl-3 diméthyl-2,2 phényl-4 oxazolidinecarboxylate-5-(4S,5R) d'acétoxy-4 dihydroxy-1,2α époxy-5β,20 méthoxyacétyloxy-10β oxo-9 triéthylsilyloxy-7β taxène-11 yle-13α sous forme d'une meringue blanche.

Le tert-butoxycarbonyl-3 diméthyl-2,2 phényl-4 oxazolidinecarboxylate-5-(4S,5R) d'acétoxy-4 hydroxy-1 benzoyloxy-2α époxy-5β,20 méthoxyacétyloxy-10β oxo-9 triéthylsilyloxy-7β taxène-11 yle-13α peut être préparé de la manière suivante :

A une solution de 1,46 g d'acétoxy-4 benzoyloxy-2α dihydroxy-1,13α époxy-5β,20 méthoxyacétyloxy-10β oxo-9 triéthylsilyloxy-7β taxène-11 dans 15 cm3 de toluène on ajoute 0,96 g d'acide tert-butoxycarbonyl-3 diméthyl-2,2 phényl-4 oxazolidinecarboxylique-5-(4S;5R), 0,66 g de N,N'-dicyclohexylcarbodiimide et 0,12 g de diméthylamino-4 pyridine. Le milieu réactionnel est chauffé sous agitation pendant 3 heures à une température voisine de 80°C, puis refroidi jusqu'à une température de 20°C et versé dans un mélange de 50 cm3 de dichlorométhane et de 150 cm3 d'une solution aqueuse saturée d'hydrogénocarbonate de sodium. La phase organique est séparée par décantation, lavée par 2 fois 20 cm3 d'eau distillée, séchée sur du sulfate de magnésium, filtrée puis concentrée à sec sous pression réduite (2,7 kPa) à 40°C. On obtient 2,5 g d'une meringue blanche que l'on purifie par chromatographie sur 75 g de silice (0,063-0,2 mm) contenus dans une colonne de 3 cm de diamètre [éluant : dichlorométhane-méthanol (99-1 en volumes)] en recueillant des fractions de 3 cm3. Les fractions 42 à 59 sont réunies et concentrées à sec sous pression réduite (2,7 kPa puis 0,07 kPa) à 40°C. On obtient ainsi 1,9 g tert-butoxycarbonyl-3 diméthyl-2,2 phényl-4 oxazolidinecarboxylate-5-(4S,5R) d'acétoxy-4 hydroxy-1 benzoyloxy-2α époxy-5β,20 méthoxyacétyloxy-10β oxo-9 triéthylsilyloxy-7β taxène-11 yle-13α.

L'acide tert-butoxycarbonyl-3 diméthyl-2,2 phényl-4 oxazolidinecarboxylique-5-(4S,5R) peut être préparé selon la méthode décrite dans la demande PCT WO 9209589.

L'acétoxy-4 benzoyloxy-2α dihydroxy-1,13α époxy-5β,20 méthoxyacétyloxy-10β oxo-9 triéthylsilyloxy-7β taxène-11 peut être préparé de la manière suivante :

A une solution de 3,29 g d'acétoxy-4 benzoyloxy-2α trihydroxy-1,10β,13α époxy-5β,20 oxo-9 triéthylsilyloxy-7β taxène-11 dans 125 cm3 de pyridine anhydre, maintenu sous atmosphère d'argon, on ajoute goutte à goutte en 5 minutes et à une température voisine de 5°C, 2,71 g de chlorure de méthoxyacétyle. L'addition terminée le milieu réactionnel est agité pendant 14 heures à une température voisine de 5°C puis pendant 24 heures à une température voisine de 20°C et versé dans un mélange de 250 cm3 de dichlorométhane et de 1000 cm3 d'une solution aqueuse saturée d'hydrogénocarbonate de sodium. La phase organique est séparée par décantation, lavée par 2 fois 100 cm3 d'eau distillée, séchée sur du sulfate de magnésium, filtrée puis concentrée à sec sous pression réduite (2,7 kPa) à 40°C. On obtient 4,1 g d'une meringue beige que l'on purifie par chromatographie sur 80 g de silice (0,063-0,2 mm) contenus dans une colonne de 3 cm de diamètre [éluant : dichlorométhane-méthanol (99-1 en volumes)]. Les fractions ne contenant que le produit cherché sont réunies et concentrées à sec sous pression réduite (2,7 kPa) à 40°C. On obtient ainsi 2,9 g d'acétoxy-4 benzoyloxy-2α dihydroxy-1,13α époxy-5β,20 méthoxyacétyloxy-10β oxo-9 triéthylsilyloxy-7β taxène-11 sous forme d'une meringue blanche.

L'acétoxy-4 benzoyloxy-2α trihydroxy-1,10β,13α époxy-5β,20 oxo-9 triéthylsilyloxy-7β taxène-11 peut être préparé selon la méthode décrite par J-N. Denis et al., J. Am. Chem. Soc., **110**, 5917, (1988).

### EXEMPLE 3

On procède à la réduction électrolytique du tert-butoxycarbonylamino-3 phényl-3 hydroxy-2 propionate-(2R,3S) d'acétoxy-4 benzoyloxy-2α époxy-5β,20 trihydroxy-1,7β,10β oxo-9 taxène-11 yle-13α (ou docetaxel) dans une cellule d'électrolyse ayant les caractéristiques suivantes :
- la cellule est un vase de verre de 50 cm3 divisé en 2 compartiments par une membrane échangeuse de cations,
- la cathode est une nappe de mercure dont la surface utile est de 4 cm2 environ,
- l'anode est une grille de platine,
- l'électrode de référence est une électrode au calomel saturée.

Dans le compartiment cathodique, on introduit 40 cm3 d'une solution contenant:
- tert-butoxycarbonylamino-3 phényl-3 hydroxy-2 propionate-(2R,3S) d'acétoxy-4 benzoyloxy-2α époxy-5β,20 trihydroxy-1,7β,10β oxo-9 taxène-11 yle-13α 402 mg (titrant 73 % soit 293 mg de docetaxel pur)
- méthanol 40 cm3
- tétrafluoroborate de tétraéthylammonium 0,87 g
- acétate de tétraéthylammonium 2,61 g
- acide acétique 0,4 cm3

Dans le compartiment anodique, on introduit environ 10 cm3 d'une solution aqueuse d'acide sulfurique à 1 % en volume.

Après désaération de la solution pendant 10 minutes par barbotage d'un courant d'argon, le potentiel de la cathode est fixé à -1,85 volt au début de l'électrolyse. Il est progressivement diminué jusqu'à -2,02 volt à la fin de l'électrolyse.

Après passage de 480 coulombs, l'électrolyse est arrêtée. Le solvant est évaporé sous pression réduite à une température inférieure à 35°C. Le résidu est repris par 20 cm3 d'eau extrait par 3 fois 20 cm3 de dichlorométhane. La phase organique est rincée par 20 cm3 de solution aqueuse saturée de chlorure de sodium puis séchée sur sulfate de magnésium. Après filtration et évaporation du solvant sous pression réduite à une température inférieure à 35°C, le produit brut obtenu est chromatographié sur 50 g de silice (0,063-0,2 mm) contenus dans une colonne de 24 mm de diamètre. On élue avec un mélange dichlorométhane-méthanol contenant 1, 2 puis 3 % de méthanol en volumes. Les fractions contenant le produit attendu purifié sont réunies et concentrées à sec sous pression réduite (0,27 kPa) à une température inférieure à 35°C. On obtient ainsi, avec un rendement de 53 %, 136 mg de tert-butoxycarbonylamino-3 phényl-3 hydroxy-2 propionate-(2R,3S) d'acétoxy-4 épéxy-5β,20 tétrahydroxy-1,2α,7β,10β oxo-9 taxène-11 yle-13α sous forme d'une meringue blanche.

Le produit ainsi obtenu peut être acylé en position 2 après protection des fonctions hydroxy en 7β, 10β et en position 2 de la chaîne latérale.

### EXEMPLE 4

On procède à la réduction électrolytique du tert-butoxycarbonylamino-3 phényl-3 triéthylsilyloxy-2 propionate-(2R,3S) d'acétoxy-4 benzoyloxy-2α époxy-5β,20 dihydroxy-1,10β triéthylsilyloxy-7α oxo-9 taxène-11 yle-13α dans une cellule d'électrolyse ayant les caractéristiques suivantes :
- la cellule est un vase de verre de 50 cm3 divisé en 2 compartiments par une membrane échangeuse de cations,
- la cathode est une nappe de mercure dont la surface utile est de 12 cm2 environ,
- l'anode est une grille de platine,
- l'électrode de référence est une électrode au calomel saturée.

Dans le compartiment cathodique, on introduit 25 cm3 d'une solution contenant:
- tert-butoxycarbonylamino-3 phényl-3 triéthylsilyloxy-2 propionate-(2R,3S) d'acétoxy-4 benzoyloxy-2α époxy-5β,20 dihydroxy-1,10β triéthylsilyloxy-7α oxo-9 taxène-11 yle-13α 500 mg
- méthanol 25 cm3
- acétate de tétraéthylammonium q.s.p 0,1M
- acide acétique 0,05 cm3

Dans le compartiment anodique, on introduit environ 25 cm3 d'une solution identique ne contenant pas le substrat.

La cellule est plongée dans un bain réfrigérant de glace fondante. La température du mélange réactionnel est voisine de 4°C. Après désaération de la solution pendant 10 minutes par barbotage d'un courant d'argon, le potentiel de la cathode est fixé à -1,95 volt au début de l'électrolyse.

Après passage de 340 coulombs, l'électrolyse est arrêtée. On ajoute à la solution obtenue 6 volumes d'une solution aqueuse saturée d'hydrogénocarbonate de sodium diluée à 50 %. On extrait par 3 fois 25 cm3 d'acétate d'éthyle. La phase organique est rincée par une solution aqueuse saturée de chlorure de sodium puis séchée sur sulfate de magnésium. Après filtration et évaporation du solvant sous pression réduite à une température inférieure à 35°C, le produit brut obtenu est chromatographié sur 50 g de silice contenus dans une colonne de 20 mm de diamètre. On élue avec un mélange dichlorométhane-méthanol sous une pression de 4 bars. Les fractions 12 à 59 contenant le produit attendu purifié sont réunies et concentrées à sec sous pression réduite (0,27 kPa) à une température inférieure à 35°C. On obtient ainsi, avec un rendement de 55 %, 248mg de tert-butoxycarbonylamino-3 phényl-3 triéthylsilyloxy-2 propionate-(2R,3S) d'acétoxy-4 époxy-5β,20 trihydroxy-1,2α,10β triéthylsilyloxy-7α oxo-9 taxène-11 yle-13α sous forme d'une meringue blanche.

Le produit ainsi obtenu peut être acylé en position 2 après protection de la fonction hydroxy en 10β.

### EXEMPLE 5

On procède à la réduction électrolytique de la désacétyl-10 baccatine III dans une cellule d'électrolyse présentant les caractéristiques suivantes :
- la cellule est un vase de verre de 100 cm3 divisé en deux compartiments par une membrane échangeuse de cations,
- la cathode est une nappe de mercure dont la surface utile est d'environ 12 cm2,
- l'anode est une grille de platine,
- l'électrode de référence est une électrode au calomel saturée.

Dans le compartiment cathodique, on introduit 50 cm3 d'une solution contenant :
- désacétyl-10 baccatine III 500 mg
- acétate de tétraméthylammonium 0,1 M
- acide acétique 0,1 M
- méthanol 50 cm3

Dans le compartiment anodique, on introduit 10 cm3 d'une solution aqueuse d'acide sulfurique 0,15 M.

Après désaération de la solution pendant 10 minutes par barbotage d'un courant d'argon, qui est maintenu pendant toute la durée de l'électrolyse, le potentiel de la cathode est fixé à -1,9 volt par rapport à l'électrode de référence.

On électrolyse la solution pendant 160 minutes c'est-à-dire pendant le temps nécessaire au passage de 700 coulombs (soit 7,6 faradays par mole). Après avoir éliminé le solvant sous pression réduite à 35°C, on reprend le résidu par 20 cm3 d'acétate d'éthyle et 20 cm3 d'eau saturée en chlorure de sodium et tamponnée à pH = 7 par une solution aqueuse de phosphate de sodium 0,2M. Après décantation, on extrait la phase aqueuse par de l'acétate d'éthyle (10 cm3 à chaque fois) jusqu'à épuisement. Après séchage de la phase organique sur sulfate de sodium, filtration et concentration à sec sous pression réduite à une température voisine de 35°C, on obtient 370 mg d'un produit brut que l'on purifie par chromatographie sur colonne de silice (Kieselgel 60 F 254, Merck) de 2 cm de diamètre et de 3 cm de hauteur. On élue successivement par 500 cm3 au total d'un mélange méthanol-chlorure de méthylène-acétate d'éthyle (5-47,5-47,5 en volumes), 250 cm3 au total d'un mélange méthanol-chlorure de méthylène-acétate d'éthyle (10-45-45 en volumes) et 250 cm3 au total d'un mélange méthanol-acétate d'éthyle (10-90 en volumes). Après évaporation des fractions éluées entre le 50ème cm3 du premier éluant, la totalité du second éluant et les 90 premiers cm3 du dernier éluant et séchage du produit obtenu sous pression réduite (0,27 kPa), on obtient avec un rendement de 83 %, 337,4 mg de diacétoxy-4,10β tétrahydroxy-1,2α,7β,13α époxy-5β,20 oxo-9 taxène-11.

### EXEMPLE 6

A une solution de 1,5 g de tert-butoxycarbonyl-3 (méthoxy-4 phényl)-2 phényl-4 oxazolidine-1,3 carboxylate-5-(2R,4S,5R) d'acétoxy-4α dihydroxy-1β,2α époxy-5β,20 méthoxyacétoxy-10β oxo-9 triéthylsilyloxy-7β taxène-11 yle-13α dans 20 cm3 de tétrahydrofuranne maintenue sous agitation et sous atmosphère d'argon, on ajoute successivement, à une température voisine de -78°C, 2,30 cm3 d'une solution 1,4M de n-butyllithium dans l'hexane et 510 mg de chlorure de 4-méthoxybenzoyle. La solution est ainsi maintenue agitée pendant 30 minutes puis on ajoute 3 cm3 d'une solution aqueuse saturée de chlorure d'ammonium. Le milieu réactionnel est ramené à une température voisine de 20°C en 1 heure. La solution obtenue est versée dans un mélange de 100 cm3 d'acétate d'éthyle et 100 cm3 d'une solution aqueuse saturée de chlorure d'ammonium. La phase aqueuse est séparée par décantation puis extraite par 2 fois 50 cm3 d'acétate d'éthyle. Les phases organiques sont réunies, lavées par 50 cm3 d'eau distillée puis séchées sur sulfate de magnésium, filtrées et concentrées à sec sous pression réduite (2,7 kPa) à 40°C. On obtient 2,15 g d'une meringue blanche que l'on purifie par chromatographie sur 1500 g de silice (0,063-0,2 mm) contenus dans une colonne de 4 cm de diamètre [éluant : acétate d'éthyle-cyclohexane (30-70 en volumes)] en recueillant des fractions de 10 cm3. Les fractions ne contenant que le produit cherché sont réunies et concentrées à sec sous pression réduite (2,7 kPa) à 40°C. On obtient 0,760 g de tert-butoxycarbonyl-3 (méthoxy-4 phényl)-2 phényl-4 oxazolidine-1,3 carboxylate-5-(2R,4S,5R) d'acétoxy-4α époxy-5β,20 hydroxy-1β méthoxyacétoxy-10β (méthoxy-4 benzoyloxy)-2α oxo-9 triéthylsilyloxy-7β taxène-11 yle-13α sous forme d'une meringue blanche dont les caractéristiques sont les suivantes :
- spectre de R.M.N. : (400 MHz ; CDCl₃ ; δ en ppm) : 0,59 (q, J=7,5 Hz, 6H: -CH₂-éthyl) ; 0,93 (t, J=7,5 Hz, 9H: -CH₃ éthyl) ; 1,10 [s, 9H: -C(CH₃)₃] ; 1,19 (s, 3H: -CH₃ 16 ou 17) ; 1,20 (s, 3H: -CH₃ 16 ou 17) : 1,67 (s, 3H: -CH₃ 19) ; 1,70 (s, 3H: -CH₃ 18) ; 1,73 (s, 1H: -OH en 1) ; 1,86 [mt, 1H: >(CH)-H 6] ; 1,87 (s, 3H: -COCH₃) ; 2,12 et 2,20 (2 dd, J=16 et 9 Hz, 1H chacun: -CH₂-14) ; 2,49 [mt, 1H: >(CH)-H 6] ; 3,52 (s, 3H: -OCH₃) ; 3,71 (d, J=7 Hz, 1H: -H 3) ; 3,83 (s, 3H: Ar-OCH₃ en 5') ; 3,89 (s, 3H: Ar-OCH₃ en 2) ; 4,10 [d, J=8 Hz, 1H: >(CH)-H 20] ; 4,18 (ab limite, J=16 Hz, 2H: -OCOCH₂O-) ; 4,24 [d, J=8 Hz, 1H: >(CH)-H 20] ; 4,43 (dd, J=11 et 7 Hz, 1H: -H 7) ; 4,58 (d, J=5,5 Hz, 1H: -H 2') ; 4,86 (d large, J=10 Hz, 1H: -H 5) ; 5,43 (d, J=5,5 Hz, 1H: -H 3') ; 5,62 (d, J=7 Hz, 1H: -H 2) ; 6,07 (t, J=9 Hz, 1H: -H 13) ; 6,39 (s large, 1H: -H 5') ; 6,44 (s, 1H: -H 10) ; 6,93 [d, J=7,5 Hz, 2H: -C₆H₅ en 5'(-H 3 et -H 5)] ; 6,98 (d, J=8,5 Hz, 2H: -H aromatiques en ortho du -OCH₃) ; de 7,30 à 7,45 [mt, 7H: -C₆H₅ en 3' et -C₆H₅ en 5'(-H 2 et -H 6)] ; 7,99 (d, J=8,5 Hz, 2H: -H aromatiques en méta du -OCH₃).

Une solution de 0,750 g de tert-butoxycarbonyl-3 (méthoxy-4 phényl)-2 phényl-4 oxazolidine-1,3 carboxylate-5-(2R,4S,5R) d'acétoxy-4α époxy-5β,20 hydroxy-1β méthoxyacétoxy-10β (méthoxy-4 benzoyloxy)-2α oxo-9 triéthylsilyloxy-7β taxène-11 yle-13α dans 13 cm3 d'une solution 0,1N d'éthanol chlorhydrique est maintenue sous agitation à une température voisine de 20°C pendant 16 heures. Le milieu réactionnel est concentré à sec sous pression réduite (2,7kPa) à 40°C. Le brut réactionnel est dissous dans 100 cm3 de dichlorométhane et 100 cm3 d'une solution aqueuse saturée de bicarbonate de sodium. La phase organique est séparée par décantation puis extraite par 2 fois 50 cm3 de dichlorométhane. Les phases organiques sont réunies, lavées par 50 cm3 d'eau distillée puis séchées sur sulfate de magnésium, filtrées et concentrées à sec sous pression réduite (2,7 kPa) à 40°C. On obtient 1,0 g d'une meringue blanche que l'on purifie par chromatographie sur 1000 g de silice (0,063-0,2 mm) contenus dans une colonne de 4 cm de diamètre (éluant : acétate d'éthyle-cyclohexane (30-70 en volumes) en recueillant des fractions de 10 cm3. Les fractions ne contenant que le produit cherché sont réunies et concentrées à sec sous pression réduite (2,7 kPa) à 40°C. On obtient 0,250 g de tert-butoxycarbonylamino-3 hydroxy-2 phényl-3 propionate-(2R,3S) d'acétoxy-4α dihydroxy-1β,7β époxy-5β,20 méthoxyacétoxy-10β (méthoxy-4 benzoyloxy)-2α oxo-9 taxène-11 yle-13α sous forme d'une meringue blanche dont les caractéristiques sont les suivantes :
- spectre de R.M.N. : (400 MHz ; CDCl₃ ; δ en ppm) : 1,15 (s, 3H: -CH₃ 16 ou 17); 1,27 (s, 3H: -CH₃ 16 ou 17) ; 1,36 [s, 9H: -C(CH₃)₃],; 1,69 (s, 3H: -CH₃ 19) ; de 1,80 à 1,95 [mt, 1H: >(CH)-H 6] ; 1,87 (s, 3H: -CH₃ 18) ; 2,30 (mt, 2H: -CH₂- 14) ; 2,40 (s, 3H: -COCH₃) ; 2,55 [mt, 1H: >(CH)-H 6] ; 3,49 (s, 3H: -OCH₃) ; 3,78 (d, J=7 Hz, 1H: -H 3) ; 3,89 (s, 3H: Ar-OCH₃) ; 4,17 [d, J=8 Hz, 1H: >(CH)-H 20] ; 4,24 (ab limite, J=16 Hz, 2H: -OCOCH₂O-) ; 4,31 [d, J=8 Hz, 1H: >(CH)-H 20] ; 4,43 (dd, J=11 et 7 Hz, 1H: -H 7) ; 4,64 (s large, 1H: -H 2') ; 4,95 (d large, J=10 Hz, 1H: -H 5) ; 5,25 (d large, J=10 Hz, 1H: -H 3') ; 5,52 (d, J=10 Hz, 1H: -CONH-) ; 5,64 (d, J=7 Hz, 1H: -H 2) ; 6,22 (t, J=9 Hz, 1H: -H 13) ; 6,40 (s, 1H: -H 10) ; 6,98 (d, J=8,5 Hz, 2H: -H aromatiques en ortho du -OCH₃) ; de 7,25 à 7,45 (mt, 5H: -C₆H₅ en 3') ; 8,05 (d, J=8,5 Hz, 2H: -H aromatiques en méta du -OCH₃).

### EXEMPLE 7

A une solution de 0.200 g de tert-butoxycarbonylamino-3 hydroxy-2 phényl-3 propionate (2R,3S) d'acétoxy-4α dihydroxy-1β,7β époxy-5β,20 méthoxyacétoxy-10β (méthoxy-4 benzoyloxy)-2α oxo-9 taxène-11 yle-13α dans 15 cm3 de méthanol maintenue sous agitation à une température voisine de 25°C, sont ajoutés successivement 1 g de tamis moléculaire 4Å en poudre et 0,700 g de iodure de zinc. La solution est ainsi maintenue agitée pendant 5 heures puis 10 cm3 d'eau distillée sont ajoutés. Le milieu réactionnel est filtré et le précipité est rincé avec 5 cm3 d'acétate d'éthyle et 5 cm3 d'eau distillée. La phase organique est séparée par décantation. La phase aqueuse est concentrée sous pression réduite (2,7 kPa), reprise dans 20 cm3 d'acétate d'éthyle et 20 cm3 d'eau distillée. Les phases organiques sont réunies et lavées avec 20 cm3 d'une solution saturée de thiosulfate de sodium et 20 cm3 d'eau distillée. La phase organique est séchée sur sulfate de magnésium, filtrée et concentrée à sec sous pression réduite (2,7 kPa). On obtient 0,100 g d'une huile jaune que l'on purifie par chromatographie préparative sur plaque de silice de 2 mm d'épaisseur [éluant : dichlorométhane-méthanol (95-5en volumes)]. On obtient ainsi 0,048 g de tert-butoxycarbonylamino-3 hydroxy-2 phényl-3 propionate-(2R,3S) d'acétoxy-4α époxy-5β,20 (méthoxy-4 benzoyloxy)-2α oxo-9 trihydroxy-1β,7β,10β taxène-11 yle-13α sous forme d'une meringue blanche dont les caractéristiques sont les suivantes :
- spectre de R.M.N. : (400 MHz ; CDCl₃ ; δ en ppm) : 1,14 (s, 3H: -CH₃ 16 ou 17) ; 1,24 (s, 3H: -CH₃ 16 ou 17) ; 1,37 [s, 9H: -C(CH₃)₃] ; 1,72 (s, 1H: -OH en 1) ; 1,78 (s, 3H: -CH₃ 19) ; de 1,75 à 1,90 [mt, 1H: >(CH)-H 6] ; 1,87 (s, 3H: -CH₃ 18) ; 2,28 (d, J=9 Hz, 2H: -CH₂- 14) ; 2,40 (s, 3H: -COCH₃) ; 2,61 [mt, 1H: >(CH)-H6] ; 3,32 (d, J=4.5 Hz, 1H: -OH en 2') ; 3,89 (s, 3H: -OCH₃) ; 3,92 (d, J=7 Hz, 1H: -H 3) ; 4,20 [d, J=8 Hz, 1H: >(CH)-H 20] ; 4,21 (s large, 1H: -OH en 10) ; 4,23 (mt, 1H: -H 7) ; 4,33 [d, J=8 Hz, 1H: >(CH)-H20] ; 4,63 (mt, 1H: -H 2') ; 4,95 (d large, J=10 Hz, 1H: -H 5) ; 5,21 (s large, 1H: -H 10) ; 5,27 (d large, J=10 Hz, 1H: -H 3') ; 5,43 (d, J=10 Hz, 1H: -CONH-) ; 5,67 (d, J=7 Hz, 1H: -H 2) ; 6,23 (t, J=9 Hz, 1H: -H 13) ; 6,98 (d, J=8,5 Hz, 2H: -H aromatiques en ortho du -OCH₃) ; de 7,25 à 7,45 (mt, 5H: -C₆H₅ en 3') ; 8,08 (d, J=8,5 Hz, 2H: -H aromatiques en méta du -OCH₃).

### EXEMPLE 8

En opérant comme dans l'exemple 6 à partir de matières premières convenables, on obtient le tert-butoxycarbonyl-3 (méthoxy-4 phényl)-2 phényl-4 oxazolidine-1,3 carboxylate-5 (2R,4S,5R) d'acétoxy-4α époxy-5β,20 (fluoro-4 benzoyloxy)-2α hydroxy-1β méthoxyacétoxy-10β oxo-9 triéthylsilyloxy-7β taxène-11 yle-13α dont les caractéristiques sont les suivantes :
- spectre de R.M.N.: (400 MHz ; CDCl₃ ; δ en ppm) : 0,60 (q, J=7,5 Hz, 6H: -CH₂-éthyl) ; 0,94 (t, J=7,5 Hz, 9H: -CH₃ éthyl) ; 1,08 [s, 9H: -C(CH₃)₃] ; 1,18 (s, 3H: -CH₃ 16 ou 17) ; 1,19 (s, 3H: -CH₃ 16 ou 17) ; 1,65 (s, 3H: -CH₃ 19) ; 1,68 (s, 3H: -CH₃ 18) ; 1,71 (s, 1H: -OH en 1) ; 1,86 [mt, 1H: >(CH)-H 6] ; 1,87 (s, 3H: -COCH₃) ; 2,09 et 2,18 (2 dd, J=16 et 9 Hz, 1H chacun: -CH₂-14) ; 2,48 [mt, 1H: >(CH)-H 6] ; 3,52 (s, 3H: -OCH₃) ; 3,72 (d, J=7 Hz, 1H: -H 3) ; 3,81 (s, 3H: Ar-OCH₃) ; 4,09 [d, J=8 Hz, 1H: >(CH)-H20] ; 4,16 (ab limite, J=16 Hz, 2H: -OCOCH₂O-) ; 4,19 [d, J=8 Hz, 1H: >(CH)-H 20] ; 4,43 (dd, J=11 et 7 Hz, 1H: -H 7) ; 4,57 (d, J=5,5 Hz, 1H: -H 2') ; 4,85 (d large, J=10 Hz, 1H: -H 5) ; 5,43 (d, J=5,5 Hz, 1H: -H 3') ; 5,60 (d, J=7 Hz, 1H: -H 2) ; 6,06 (t, J=9 Hz, 1H: -H 13) ; 6,39 (s large, 1H: -H 5') ; 6,43 (s, 1H: -H 10) ; 6,92 [d, J=7,5 Hz, 2H: -C₆H₅ en 5'(-H 3 et -H 5)] ; 7,13 (t, J=8,5 Hz, 2H: -H aromatiques en ortho du -F) ; de 7,30 à 7,45 [mt, 7H: -C₆H₅ en 3' et -C₆H₅ en 5'(-H 2 et -H 6)] ; 8,03 (d, J=8,5 Hz, 2H: -H aromatiques en méta du -F).

En opérant comme dans l'exemple 6, on obtient le tert-butoxycarbonylamino-3 hydroxy-2 phényl-3 propionate (2R,3S) d'acétoxy-4α dihydroxy-1β,7β époxy-5β,20 (fluoro-4 benzoyloxy)-2α méthoxyacétoxy-10β oxo-9 taxène-11 yle-13α dont les caractéristiques sont les suivantes :
- spectre de R.M.N. : (400 MHz ; CDCl₃ ; δ en ppm) : 1,15 (s, 3H: -CH₃ 16 ou 17) ; 1,27 (s, 3H: -CH₃ 16 ou 17) ; 1,35 [s, 9H: -C(CH₃)₃] ; 1,70 (s, 3H: -CH₃ 19) ; 1,87 (s, 3H: -CH₃ 18) ; 1,89 [mt, 1H: >(CH)-H 6] ; 2,27 et 2,33 (2dd, J=17 et 9 Hz, 1H chacun: -CH₂- 14) ; 2,40 (s, 3H: -COCH₃) ; 2,58 [mt, 1H: >(CH)-H 6] ; 3,33 (mf étalé, 1H: -OH en 2') ; 3,53 (s, 3H: -OCH₃) ; 3,81 (d, J=7 Hz, 1H: -H 3) ; 4,17 [d, J=8 Hz, 1H: >(CH)-H 20] ; 4,26 (ab limite, J=16 Hz, 2H: -OCOCH₂O-) ; 4,29 [d, J=8 Hz, 1H: >(CH)-H 20] ; 4,42 (dd, J=11 et 7 Hz, 1H: -H 7) ; 4,66 (s large, 1H: -H 2') ; 4,96 (dd, J=10 et 1,5 Hz, 1H: -H 5) ; 5,27 (d large, J=10 Hz, 1H: -H 3') ; 5,37 (d, J=10 Hz, 1H: -CONH-) ; 5,66 (d, J=7 Hz, 1H: -H 2) ; 6,25 (t large, J=9 Hz, 1H: -H 13) ; 6,39 (s, 1H: -H 10) ; 7,19 (t, J=8,5 Hz, 2H: -H aromatiques en ortho du -F) ; de 7,30 à 7,45 (mt, 5H: -C₆H₅ en 3') ; 8,13 (dd, J=8,5 et 6 Hz, 2H: -H aromatiques en méta du -F).

### EXEMPLE 9

En opérant comme dans l'exemple 7, on obtient le tert-butoxycarbonylamino-3 hydroxy-2 phényl-4 propionate-(2R,4S) d'acétoxy-4α époxy-5β,20 (fluoro-4 benzoyloxy)-2α oxo-9 trihydroxy-1β,7β,10β taxène-11 yle-13α dont les caractéristiques sont les suivantes :
- spectre de R.M.N. : (400 MHz ; CDCl₃ ; δ en ppm) : 1,14 (s, 3H: -CH₃ 16 ou 17) ; 1,26 (s, 3H: -CH₃ 16 ou 17) ; 1,37 [s, 9H: -C(CH₃)₃] ; 1,63 (s, 1H: -OH en 1) ; 1,77 (s, 3H: -CH₃ 19) ; 1,86 [mt, 1H: >(CH)-H 6] ; 1,87 (s, 3H: -CH₃ 18) ; 2.28 (d, J=9 Hz, 2H: -CH₂- 14) ; 2,39 (s, 3H: -COCH₃) ; 2,60 [mt, 1H: >(CH)-H 6] ; 3,33 (mf, 1H: -OH en 2') ; 3,92 (d, J=7 Hz, 1H: -H 3) ; 4,19 [d, J=8 Hz, 1H: >(CH)-H 20] ; 4,21 (s large, 1H: -OH en 10) ; 4,24 (mt, 1H: -H7) ; 4,31 [d, J=8 Hz, 1H: >(CH)-H 20] ; 4,64 (s large, 1H: -H 2') ; 4,96 (dd, J=10 et 1,5 Hz, 1H: -H 5) ; 5,21 (s, 1H: -H 10) ; 5,27 (d large, J=10 Hz, 1H: -H 3') ; 5,43 (d, J=10 Hz, 1H: -CONH-) ; 5,67 (d, J=7 Hz, 1H: -H 2) ; 6,24 (t, J=9 Hz, 1H: -H 13) ; 7,19 (t, J=8,5 Hz, 2H: -H aromatiques en ortho du -F) ; de 7,25 à 7,45 (mt, 5H: -C₆H₅ en 3') ; 8,12 (dd, J=8,5 et 6 Hz, 2H: -H aromatiques en méta du -F).

### EXEMPLE 10

En opérant comme dans l'exemple 6 à partir de matières premières convenables, on obtient le tert-butoxycarbonyl-3 (méthoxy-4 phényl)-2 phényl-4 oxazolidine-1,3 carboxylate-5-(2R,4S,5R) d'acétoxy-4α époxy-5β,20 (trifluorométhyl-4 benzoyloxy)-2α hydroxy-1β méthoxyacétoxy-10β oxo-9 triéthylsilyloxy-7β taxène-11 yle-13α dont les craractéristiques sont les suivantes :
- spectre de R.M.N. : (400 MHz ; CDCl₃ ; δ en ppm) : 0,60 (q, J=7,5 Hz, 6H: -CH₂-éthyl) ; 0,96 (t, J=7,5 Hz, 9H: -CH₃ éthyl) ; 1,09 [s, 9H: -C(CH₃)₃] ; 1,18 (s, 3H: -CH₃ 16 ou 17) ; 1,20 (s, 3H: -CH₃ 16 ou 17) ; 1,64 (s, 1H: -OH en 1) ; 1,66 (s, 3H: -CH₃ 19) ; 1,68 (s, 3H: -CH₃ 18) ; 1,86 [mt, 1H: >(CH)-H 6] ; 1,90 (s, 3H: -COCH₃) ; 2,11 et 2,19 (2 dd, J=15 et 9 Hz, 1H chacun: -CH₂- 14) ; 2,50 [mt, 1H: >(CH)-H 6] ; 3,52 (s, 3H: -OCH₃) ; 3,73 (d, J=7 Hz, 1H: -H 3) ; 3,82 (s, 3H: Ar-OCH₃) ; 4,10 [d, J=8 Hz, 1H: >(CH)-H 20] ; 4,17 (ab limite, J=16 Hz, 2H: -OCOCH₂O-) ; 4,20 [d, J=8 Hz, 1H: >(CH)-H 20] ; 4,43 (dd, J=11 et 7 Hz, 1H: -H 7) ; 4,58 (d, J=5 Hz, 1H: -H 2') ; 4,86 (d large, J=10 Hz, 1H: -H 5) ; 5,45 (d, J=5 Hz, 1H: -H 3') ; 5,63 (d, J=7 Hz, 1H: -H 2) ; 6,05 (t, J=9 Hz, 1H: -H 13) ; 6,40 (s large, 1H: -H 5') ; 6,45 (s, 1H: -H 10) ; 6,92 [d, J=7,5 Hz, 2H: -C₆H₅ en 5'(-H 3 et -H 5)] ; de 7,35 à 7,45 [mt, 7H: -C₆H₅ en 3' et -C₆H₅ en 5'(-H 2 et -H 6)] ; 7,76 (d, J=8,5 Hz, 2H: -H aromatiques en ortho du -CF₃) ; 8,14 (d, J=8,5 Hz, 2H: -H aromatiques en méta du -CF₃).

En opérant comme dans l'exemple 6, on obtient le tert-butoxycarbonylamino-3 hydroxy-2 phényl-4 propionate-(2R,4S) d'acétoxy-4α dihydroxy-1β,7β époxy-5β,20 (trifluorométhyl-4 benzoyloxy)-2α méthoxyacétoxy-10β oxo-9 taxène-11 yle-13α dont les caractéristiques sont les suivantes :
- spectre de R.M.N. : (400 MHz ; CDCl₃ ; δ en ppm) : 1,16 (s, 3H: -CH₃ 16 ou 17) ; 1,28 (s, 3H: -CH₃ 16 ou 17) ; 1,32 [s, 9H: -C(CH₃)₃] ; 1,70 (s, 3H: -CH₃ 19) ; de 1,85 à 1,95 [mt, 1H: >(CH)-H 6] ; 1,90 (s, 3H: -CH₃ 18); 2,31 (ab limite dédoublé, J=18 et 9 Hz, 2H: -CH₂- 14) ; 2,42 (s, 3H: -COCH₃) ; 2,58 (mt, 1H: >(CH)- H 6) ; de 3,20 à 3,60 (mt étalé, 1H: -OH en 2') ; 3,52 (s, 3H: -OCH₃) ; 3,84 (d, J=7 Hz, 1H: -H 3) ; 4,18 (d, J=8 Hz, 1H: >(CH)-H 20) ; 4,26 (ab limite, J=16 Hz, 2H: -OCOCH₂O-) ; 4,28 (d, J=8 Hz, 1H: >(CH)-H 20) ; 4,43 (dd, J=11 et 7 Hz, 1H: H 7); 4,68 (s large, 1H: -H 2') ; 4,96 (d large, J=10 Hz, 1H: -H 5) ; 5,30 (d large, J=10 Hz, 1H: -H 3') ; 5,42 (d, J=10 Hz, 1H: -CONH-) ; 5,67 (d, J=7 Hz, 1H: -H 2) ; 6,28 (t, J=9 Hz, 1H: -H 13) ; 6,40 (s, 1H: -H 10) ; de 7,30 à 7,45 (mt, 5H: -C₆H₅ en 3') ; 7,79 (d, J=8,5 Hz, 2H: -H aromatiques en ortho du -CF₃) ; 8,24 (d, J=8,5 Hz, 2H: -H aromatiques en méta du -CF₃).

### EXEMPLE 11

En opérant comme dans l'exemple 7, on obtient le tert-butoxycarbonylamino-3 hydroxy-2 phényl-4 propionate (2R,4S) d'acétoxy-4α époxy-5β,20 (trifluorométhyl-4 benzoyloxy)-2α oxo-9 trihydroxy-1β,7β,10β taxène-11 yle-13α dont les caractéristiques sont les suivantes :
- spectre de R.M.N.: (400 MHz; CDCl₃ ; δ en ppm) : 1,14 (s, 3H: -CH₃ 16 ou 17) ; 1,26 (s, 3H: -CH₃ 16 ou 17) ; 1,34 [s, 9H: -C(CH₃)₃] ; 1,79 (s, 3H: -CH₃ 19) ; de 1,80 à 1,95 [mt, 1H: >(CH)-H 6] ; 1,89 (s, 3H: -CH₃ 18) ; 2,28 (mt, 2H: -CH₂- 14) ; 2,42 (s, 3H: -COCH₃) ; 2,61 [mt, 1H: >(CH)-H 6] ; 3,32 (mf, 1H: -OH en 2') ; 3,95 (d, J=7 Hz, 1H: -H 3) ; de 4,10 à 4,35 (mf étalé, 1H: -OH en 10) ; 4,19 [d, J=8 Hz, 1H: >(CH)-H 20] ; 4,25 (mt, 1H: -H 7) ; 4,29 (d, J=8 Hz, 1H: >(CH)-H 20] ; 4,66 (s large, 1H: -H 2') ; 4,96 (d large, J=10 Hz, 1H: -H 5) ; 5,21 (s, 1H: -H 10) ; 5,30 (d large, J=10 Hz, 1H: -H 3') ; 5,41 (d, J=10 Hz, 1H: -CONH-) ; 5,68 (d, J=7 Hz, 1H: -H 2) ; 6,27 (mt, 1H: -H 13) ; de 7,30 à 7,45 (mt, 5H: -C₆H₅ en 3') ; 7,80 (d, J=8,5 Hz, 2H: -H aromatiques en ortho du -CF₃) ; 8,20 (d, J=8,5 Hz, 2H: -H aromatiques en méta du -CF₃).

### EXEMPLE 12

En opérant comme dans l'exemple 6 à partir de matières premières convenables, on obtient le tert-butoxycarbonyl-3 (méthoxy-4 phényl)-2 phényl-4 oxazolidine-1,3 carboxylate-5-(2R,4S,5R) d'acétoxy-4α époxy-5β,20 (fluoro-2 benzoyloxy)-2α hydroxy-1β méthoxyacétoxy-10β oxo-9 triéthylsilyloxy-7β taxène-11 yle-13α dont les caractéristiques sont les suivantes :
- spectre de R.M.N. : (400 MHz; CDCl₃ ; δ en ppm) : 0,60 (q, J=7,5 Hz, 6H: -CH₂-éthyl) ; 0,94 (t, J=7,5 Hz, 9H: -CH₃ éthyl) ; 1,10 [s, 9H: -C(CH₃)₃] ; 1,19 (s, 3H: -CH₃ 16 et -CH₃17) ; 1,68 (s, 3H: -CH₃ 19) ; 1,69 (s, 3H: -CH₃ 18) ; 1,74 (s, 1H: -OH en 1) ; 1,81 (s, 3H: -COCH₃) ; 1,86 [mt, 1H: >(CH)-H 6] ; 2,17 et 2,22 (2 dd, J=15 et 9 Hz, 1H chacun: -CH₂- 14) ; 2,48 [mt, 1H: >(CH)-H6] ; 3,52 (s, 3H: -OCH₃) ; 3,71 (d, J=7 Hz, 1H: -H 3) ; 3,82 (s, 3H: Ar-OCH₃) ; de 4,10 à 4,25 (mt, 4H: -OCOCH₂O- et -CH₂- 20) ; 4,42 (dd, J=11 et 7 Hz, 1H: -H 7) ; 4,58 (d, J=5 Hz, 1H: -H 2') ; 4,86 (d large, J=10 Hz, 1H: -H 5) ; 5,45 (s très large, 1H: -H 3') ; 5,67 (d, J=7 Hz, 1H: -H 2) ; 6,08 (t, J=9 Hz, 1H: -H 13) ; 6,40 (s très large, 1H: -H 5') ; 6,45 (s, 1H: -H 10) ; 6,92 [d, J=7,5 Hz, 2H: -C₆H₅ en 5'(-H 3 et -H 5)] ; 7,16 [mt, 1H: -C₆H₅ en 2 (-H 3)] ; 7,28 [t, J=8,5 Hz, 1H: -C₆H₅ en 2 (-H 5)] ; de 7,35 à 7,45 [mt, 7H: -C₆H₅ en 3' et -C₆H₅ en 5'(-H 2 et -H 6)]; 7,58 [mt, 1H: -C₆H₅ en 2 (-H 4)] ; 7,96 [dd, J=8,5 et 8 Hz, 1H: -C₆H₅ en 2 (-H 6)]

En opérant comme dans l'exemple 6, on obtient le tert-butoxycarbonylamino-3 hydroxy-2 phényl-4 propionate-(2R,4S) d'acétoxy-4α dihydroxy-1β,7β époxy-5β,20 (fluoro-2 benzoyloxy)-2α méthoxyacétoxy-10β oxo-9 taxène-11 yle-13α dont les caractéristiques sont les suivantes :
- spectre de R.M.N. : (400 MHz ; CDCl₃ ; δ en ppm) : 1,16 (s, 3H: -CH₃ 16 ou 17) ; 1,26 (s, 3H: -CH₃ 16 ou 17) ; 1,40 [s, 9H: -C(CH₃)₃] ; 1,72 (s, 3H: -CH₃ 19) ; 1,88 (s, 3H: -CH₃ 18) ; 1,92 [mt, 1H: >(CH)-H 6] ; 2,32 (s, 3H: -COCH₃) ; 2,33 (mt, 2H: -CH₂-14) ; 2,56 [mt, 1H: >(CH)-H 6] ; 3,52 (s, 3H: -OCH₃) ; 3,79 (d, J=7 Hz, 1H: -H 3) ; 4,26 (ab limite, J=17 Hz, 2H: -OCOCH₂O-) ; 4,28 [ab limite, J=8 Hz, 2H: -CH₂- 20] ; 4,40 (dd, J=11 et 7 Hz, 1H: -H 7) ; 4,61 (s large, 1H: -H 2') ; 4,95 (d large, J=10 Hz, 1H: -H 5) ; 5,24 (d large, J=10 Hz, 1H: -H 3') ; 5,47 (d, J=10 Hz, 1H: -CONH-) ; 5,70 (d, J=7 Hz, 1H: -H 2) ; 6,19 (t large, J=9 Hz, 1H: -H 13) ; 6,38 (s, 1H: -H 10); 7,20 [dd, J=10,5 et 8,5 Hz, 1H: -C₆H₅ en 2 (-H 3)] ; 7,28 [t, J=8,5 Hz, 1H: -C₆H₅ en 2 (-H 5)] ; de 7,30 à 7,45 (mt, 5H: -C₆H₅ en 3') ; 7,60 [mt, 1H: -C₆H₅ en 2 (-H 4)] ; 8,03 [dd, J=8,5 et 8 Hz, 1H: -C₆H₅ en 2 (-H 6)].

### EXEMPLE 13

En opérant comme dans l'exemple 7, on obtient le tert-butoxycarbonylamino-3 hydroxy-2 phényl-4 propionate-(2R,4S) d'acétoxy-4α époxy-5β,20 (fluoro-2 benzoyloxy)-2α oxo-9 trihydroxy-1β,7β,10β taxène-11 yle-13α dont les caractéristiques sont les suivantes :
- spectre de R.M.N. : (400 MHz ; CDCl₃ ; δ en ppm) : 1,13 (s, 3H: -CH₃ 16 ou 17) ; 1,24 (s, 3H: -CH₃ 16 ou 17) ; 1,38 [s, 9H: -C(CH₃)₃] ; 1,73 (s, 1H: -OH en 1); 1,78 (s, 3H: -CH₃ 19) ; 1,84 (s, 3H: -CH₃ 18) ; 1,85 [mt, 1H: >(CH)-H 6] ; 2,30 (mt, 2H: -CH₂- 14); 2,30 (s, 3H: -COCH₃) ; 2,58 [mt, 1H: >(CH)-H 6] ; 3,43 (d, J=5,5 Hz, 1H: -OH en 2') ; 3,90 (d, J=7 Hz, 1H: -H 3) ; 4,23 (s large, 1H: -OH en 10) ; 4,23 (mt, 1H: -H 7) ; 4,28 (ab limite, J=9 Hz, 2H: -CH₂- 20) ; 4,60 (s large, 1H: -H 2') ; 4,94 (dd, J=10 et 1,5 Hz, 1H: -H 5) ; 5,21 (s, 1H: -H 10); 5,25 (d large, J=10 Hz, 1H: -H 3') ; 5,49 (d, J=10 Hz, 1H: -CONH-) ; 5,70 (d, J=7 Hz, 1H: -H 2) ; 6,19 (t, J=9 Hz, 1H: -H 13) ; 7,20 [dd, J=10.5 et 8,5 Hz, 1H: -C₆H₅ en 2 (-H 3)] ; 7,29 [t, J=8,5 Hz, 1H: -C₆H₅ en 2 (-H 5)] ; de 7,30 à 7,45 (mt, 5H: -C₆H₅ en 3') ; 7,59 [mt, 1H: -C₆H₅ en 2 (-H 4)] ; 8,03 [dd, J=8,5 et 8 Hz, 1H: -C₆H₅ en 2 (-H 6)].

### EXEMPLE 14

En opérant comme dans l'exemple 6 à partir de matières premières convenables, on obtient le tert-butoxycarbonyl-3 (méthoxy-4 phényl)-2 phényl-4 oxazolidine-1,3 carboxylate-5 (2R,4S,5R) d'acétoxy-4α époxy-5β,20 (tert-butyl-4 benzoyloxy)-2α hydroxy-1β méthoxyacétoxy-10β oxo-9 triéthylsilyloxy-7β taxène-11 yle-13α dont les caractéristiques sont les suivantes :
- spectre de R.M.N.: (400 MHz; CDCl₃ ; δ en ppm) : 0,60 (q, J=7,5 Hz, 6H: -CH₂-éthyl) ; 0,93 (t, J= 7,5 Hz, 9H: -CH₃ éthyl) ; 1,10 [s, 9H: -C(CH₃)₃] ; 1,18 (s, 3H: -CH₃ 16 ou 17) ; 1,21 (s, 3H: -CH₃ 16 ou 17) ; 1,38 [s, 9H: Ar-C(CH₃)₃] ; 1,66 (s, 3H: -CH₃ 19) ; 1,68 (s, 1H: -OH en 1) ; 1,70 (s, 3H: -CH₃ 18) ; 1,87 [mt, 1H: >(CH)-H 6] ; 1,90 (s, 3H: -COCH₃) ; 2,13 et 2,20 (2 dd, J=16 et 9 Hz, 1H chacun: -CH₂- 14) ; 2,49 [mt, 1H: >(CH)-H 6] ; 3,52 (s, 3H: -OCH₃) ; 3,73 (d, J=7 Hz, 1H: -H 3) ; 3,82 (s, 3H: Ar-OCH₃) ; 4,12 [d, J=8,5 Hz, 1H: >CH)-H 20] ; 4,15 (ab limite, J= 16 Hz, 2H: -OCOCH₂O-) ; 4,27 [d, J=8,5 Hz, 1H: >(CH)-H 20] ; 4,44 (dd, J=11 et 6.5 Hz, 1H: -H 7) ; 4,58 (d, J=5 Hz, 1H: -H 2') ; 4,87 (d large, J=10 Hz, 1H: -H 5) ; 5,44 (d, J=5 Hz, 1H: -H 3') ; 5,63 (d, J=7 Hz, 1H: -H 2) ; 6,06 (t, J=9 Hz, 1H: -H 13) ; 6,38 (s, 1H: -H 5') ; 6,44 (s, 1H: -H 10) ; 6,92 [d, J=8,5 Hz, 2H: -C₆H₅ en 5'(-H 3 et -H 5)] ; de 7,35 à 7,45 [mt, 7H: -C₆H₅ en 3' et -C₆H₅ en 5'(-H 2 et -H 6)]; 7,49 [d, J=8,5 Hz, 2H: -H aromatiques en ortho du -C(CH₃)₃] ; 7,97 [d, J= 8,5 Hz, 2H: -H aromatiques en méta du -C(CH₃)₃].

En opérant comme dans l'exemple 6, on obtient le tert-butoxycarbonylamino-3 hydroxy-2 phényl-3 propionate-(2R,3S) d'acétoxy-4α dihydroxy-1β,7β époxy-5β,20 (tert-butyl-4 benzoyloxy)-2α méthoxyacétoxy-10β oxo-9 taxène-11 yle-13α dont les caractéristiques sont les suivantes :
- spectre de R.M.N. : (400 MHz ; CDCl₃ ; δ en ppm) : 1,15 (s, 3H: -CH₃ 16 ou 17) ; 1,28 (s, 3H: -CH₃ 16 ou 17) ; 1,35 [s, 18H: -C(CH₃)₃] ; 1,70 (s, 3H: -CH₃ 19) ; 1,75 (s, 1H: -OH en 1) ; 1,88 (s, 3H: -CH₃ 18) ; 1,90 [mt, 1H: >(CH)-H 6] ; 2,28 et 2,37 (2dd, J=16 et 9 Hz, 1H chacun: -CH₂- 14) ; 2,36 (d, J=3,5 Hz, 1H: -OH en 7) ; 2,41 (s, 3H: -COCH₃) ; 2,58 [mt, 1H: >(CH)-H 6] ; 3,34 (d, J=5 Hz, 1H: -OH en 2'); 3,54 (s, 3H: -OCH₃) ; 3,82 (d, J= 7 Hz, 1H: -H3) ; 4,19 [d, J=8,5 Hz, 1H: >(CH)-H 20] ; 4,28 (ab limite, J=16 Hz, 2H: -OCOCH₂O-) ; 4,36 [d, J= 8,5 Hz, 1H: >(CH)-H 20] ; 4,43 (mt, 1H: -H 7) ; 4,63 (d large, J=5 Hz, 1H: -H 2') ; 4,97 (d large, J=10 Hz, 1H: -H 5) ; 5,28 (d large, J=10 Hz, 1H: -H 3') ; 5,38 (d, J= 10 Hz, 1H: -CONH-) ; 5,69 (d, J= 7 Hz, 1H: -H 2) ; 6,25 (t, J= 9 Hz, 1H: -H 13) ; 6,40 (s, 1H: -H 10) ; de 7,30 à 7,45 (mt, 5H: -C₆H₅ en 3') ; 7,52 [d, J=8,5 Hz, 2H: -H aromatiques en ortho du -C(CH₃)₃] ; 8,05 [d, J=8,5 Hz, 2H: -H aromatiques en méta du -C(CH₃)₃].

### EXEMPLE 15

En opérant comme dans l'exemple 7, on obtient le tert-butoxycarbonylamino-3 hydroxy-2 phényl-3 propionate-(2R,3S) d'acétoxy-4α époxy-5β,20 (tert-butyl-4 benzoyloxy)-2α oxo-9 trihydroxy-1β,7β,10β taxène-11 yle-13α dont les caractéristiques sont les suivantes :
- spectre de R.M.N. : (400 MHz ; CDCl₃ ; δ en ppm) : 1,14 (s, 3H: -CH₃ 16 ou 17) ; 1,25 (s, 3H: -CH₃ 16 ou 17) ; 1,37 [s, 18 H: -C(CH₃)₃] ; 1,72 (s, 1H: -OH en 1) ; 1,77 (s, 3H: -CH₃ 19) ; 1,85 [mt, 1H: >(CH)-H 6] ; 1,86 (s, 3H: -CH₃ 18) ; 2,28 (ab limite, J= 16 et 9 Hz, 2H: -CH₂- 14) ; 2,42 (s, 3H: -COCH₃) ; 2,60 [mt, 1H: >(CH)-H 6] ; 3,38 (d, J= 5,5 Hz, 1H: -OH en 2') ; 3,92 (d, J=7 Hz, 1H: -H 3) ; 4,20 [d, J=8 Hz, 1H: >(CH)-H 20] ; 4,23 (s large, 1H: -OH en 10) ; 4,25 (mt, 1H: -H 7) ; 4,36 [d, J=8 Hz, 1H: >(CH)-H 20] ; 4,64 (mt, 1H: -H 2') ; 4,96 (d large, J= 10 Hz, 1H: -H 5) ; 5,22 (s large, 1H: -H 10) ; 5,28 (d large, J=10 Hz, 1H: -H 3') ; 5,46 (d, J=10 Hz, 1H: -CONH-) ; 5,68 (d, J=7 Hz, 1H: -H 2) ; 6,23 (t, J=9 Hz, 1H: -H 13) ; de 7,30 à 7,45 (mt, 5H: -C₆H₅ en 3') ; 7,52 [d, J=8 Hz, 2H: -H aromatiques en ortho du -C(CH₃)₃] ; 8,04 [d, J=8 Hz, 2H: -H aromatiques en méta du -C(CH₃)₃].

### EXEMPLE 16

En opérant comme dans l'exemple 6 à partir de matières premières convenables, on obtient le tert-butoxycarbonyl-3 (méthoxy-4 phényl)-2 phényl-4 oxazolidine-1,3 carboxylate-5- (2R,4S,5R) d'acétoxy-4α époxy-5β,20 (nitro-4 benzoyloxy)-2α hydroxy-1β méthoxyacétoxy-10β oxo-9 triéthylsilyloxy-7β taxène-11 yle-13α dont les caractéristiques sont les suivantes :
- spectre de R.M.N.: (400 MHz ; CDCl₃ ; δ en ppm) : 0,59 (q, J=7,5 Hz, 6H: -CH₂-éthyl) ; 0,93 (t, J=7,5 Hz, 9H: -CH₃ éthyl) ; 1,07 [s, 9H: -C(CH₃)₃] ; 1,17 (s, 3H: -CH₃ 16 ou 17) ; 1,19 (s, 3H: -CH₃ 16 ou 17) ; 1,64 (s, 6H: -CH₃ 19 et -CH₃ 18) ; 1,83 [mt, 1H: >(CH)-H 6] ; 1,90 (s, 3H: -COCH₃) ; 2,07 et 2.18 (2 dd, J= 16 et 9 Hz, 1H chacun: -CH₂- 14) ; 2,49 [mt, 1H: >(CH)-H 6] ; 3,52 (s, 3H: -OCH₃) ; 3,72 (d, J= 7 Hz, 1H: -H 3) ; 3,82 (s, 3H: Ar-OCH₃) ; 4,06 [d, J=8,5 Hz, 1H: >(CH)-H 20] ; 4,16 (ab limite, J=16 Hz, 2H: -OCOCH₂O-) ; 4,16 [d, J=8,5 Hz, 1H: >(CH)-H 20] ; 4,41 (dd, J=11 et 7 Hz, 1H: -H 7) ; 4,58 (d, J=6 Hz, 1H: -H 2') ; 4,86 (d large, J=10 Hz, 1H: -H 5) ; 5,43 (mf, 1H: -H 3') ; 5,61 (d, J=7 Hz, 1H: -H 2) ; 6,03 (t large, J=9 Hz, 1H: -H 13) ; 6,40 (mf, 1H: -H 5') ; 6,43 (s, 1H: -H 10) ; 6,92 [d, J=8,5 Hz, 2H: -C₆H₅ en 5'(-H 3 et -H 5)] ; 7,41 [d, J=8,5 Hz, 2H: -C₆H₅ en 5' (-H 2 et -H 6)] ; 7,42 (mt, 5H: -C₆H₅ en 3') ; 8,18 (d, J=8,5 Hz, 2H: -H aromatiques en méta du -NO₂) ; 8,32 (d, J=8,5 Hz, 2H: -H aromatiques en ortho du -NO₂).

En opérant comme dans l'exemple 6, on obtient le tert-butoxycarbonylamino-3 hydroxy-2 phényl-3 propionate (2R,3S) d'acétoxy-4α dihydroxy-1β,7β époxy-5β,20 (nitro-4 benzoyloxy)-2α méthoxyacétoxy-10β oxo-9 taxène-11 yle-13α dont les caractéristiques sont les suivantes :
- spectre de R.M.N. : (400 MHz ; CDCl₃ ; δ en ppm) : 1,15 (s, 3H: -CH₃ 16 ou 17) ; 1,25 (s, 3H: -CH₃ 16 ou 17) ; 1,35 [s, 9 H: -C(CH₃)₃] ; 1,67 (s, 1H: -OH en 1) ; 1,78 (s, 3H: -CH₃ 19) ; 1,87 [mt, 1H: >(CH)-H 6] ; 1,90 (s, 3H: -CH₃ 18) ; 2,28 et 2,34 (2 dd, J=16 et 9 Hz, 1H chacun: -CH₂- 14) ; 2,30 (d, J=2 Hz, 1H: -OH en 7) ; 2,43 (s, 3H: -COCH₃) ; 2,62 [mt, 1H: >(CH)-H 6] ; 3,32 (mf, 1H: -OH en 2') ; 3,96 (d, J=7 Hz, 1H: -H 3) ; 4,20 [d, J= 8,5 Hz, 1H: >(CH)-H 20] ; 4,23 (s large, 1H: -OH en 10) ; 4,25 (mt, 1H: -H 7) ; 4,26 [d, J=8,5 Hz, 1H: >(CH)-H 20] ; 4,68 (mt, 1H: -H 2') ; 4,96 (d large, J=10 Hz, 1H: -H 5) ; 5,22 (s large, 1H: -H 10) ; 5,31 (d large, J=10 Hz, 1H: -H 3') ; 5,41 (d, J=10 Hz, 1H: -CONH-) ; 5,69 (d, J=7 Hz, 1H: -H 2) ; 6,27 (t, J=9 Hz, 1H: -H 13) ; de 7,30 à 7,50 (mt, 5H: -C₆H₅ en 3') ; 8,30 (d, J=8,5 Hz, 2H: -H aromatiques en méta du -NO₂); 8,38 (d, J= 8,5 Hz, 2H: -H aromatiques en ortho du -NO₂).

### EXEMPLE 17

En opérant comme dans l'exemple 7, on obtient le tert-butoxycarbonylamino-3 hydroxy-2 phényl-3 propionate-(2R,3S) d'acétoxy-4α époxy-5β,20 (nitro-4 benzoyloxy)-2α oxo-9 trihydroxy-1β,7β,10β taxène-11 yle-13α dont les caractéristiques sont les suivantes :
- spectre de R.M.N. : (400 MHz ; CDCl₃ ; δ en ppm) : 1,15 (s, 3H: -CH₃ 16 ou 17) ; 1,25 (s, 3H: -CH₃ 16 ou 17) ; 1,35 [s, 9 H: -C(CH₃)₃] ; 1,67 (s, 1H: -OH en 1) ; 1,78 (s, 3H: -CH₃ 19) ; 1,87 [mt, 1H: >(CH)-H 6] ; 1,90 (s, 3H: -CH₃ 18) ; 2,28 et 2,34 (2 dd, J=16 et 9 Hz, 1H chacun: -CH₂- 14) ; 2,30 (d, J=2 Hz, 1H: -OH en 7) ; 2,43 (s, 3H: -COCH₃) ; 2,62 [mt, 1H: >(CH)-H 6] ; 3,32 (mf, 1H: -OH en 2') ; 3,96 (d, J=7 Hz, 1H: -H 3) ; 4,20 [d, J=8,5 Hz, 1H: >(CH)-H 20] ; 4,23 (s large, 1H: -OH en 10) ; 4,25 (mt, 1H: -H 7) ; 4,26 [d, J=8,5 Hz, 1H: >CH)-H 20] ; 4,68 (mt, 1H: -H 2') ; 4,96 (d large, J=10 Hz, 1H: -H 5) ; 5,22 (s large, 1H: -H 10) ; 5,31 (d large, J=10 Hz, 1H: -H 3') ; 5,41 (d, J=10 Hz, 1H: -CONH-) ; 5,69 (d, J=7 Hz, 1H: -H 2) ; 6,27 (t, J=9 Hz, 1H: -H 13); de 7,30 à 7,50 (mt, 5H: -C₆H₅ en 3') ; 8,30 (d, J=8,5 Hz, 2H: -H aromatiques en méta du -NO₂) ; 8,38 (d, J=8,5 Hz, 2H: -H aromatiques en ortho du -NO₂).

### EXEMPLE 18

En opérant comme dans l'exemple 6 à partir de matières premières convenables, on obtient le tert-butoxycarbonyl-3 (méthoxy-4 phényl)-2 phényl-4 oxazolidine-1,3 carboxylate-5-(2R,4S,5R) d'acétoxy-4α époxy-5β,20 (éthyl-4 benzoyloxy)-2α hydroxy-1β méthoxyacétoxy-10β oxo-9 tfiéthylsilyloxy-7β taxène-11 yle-13α dont les caractéristiques sont les suivantes :
- spectre de R.M.N.: (400 MHz ; CDCl₃ ; δ en ppm) : 0,58 (q, J=7,5 Hz, 6H: -CH₂-éthyl) ; 0,93 (t, J=7,5 Hz, 9H: -CH₃ éthyl) ; 1,07 [s, 9H: -C(CH₃)₃] ; 1,17 (s, 3H: -CH₃ 16 ou 17) ; 1,19 (s, 3H: -CH₃ 16 ou 17) ; 1,29 (t, J=7,5 Hz, 3H: Ar-CH₂-CH₃) ; 1,65 (s, 3H: -CH₃ 19) ; 1,67 (s, 3H: -CH₃ 18) ; 1,70 (s, 1H: -OH en 1) ; 1,83 [mt, 1H: >(CH)-H 6] ; 1,84 (s, 3H: -COCH₃) ; 2,09 et 2,18 (2 dd, J=16 et 9 Hz, 1H chacun: -CH₂- 14) ; 2,48 [mt, 1H: >(CH)-H 6] ; 2,74 (t, J=7,5 Hz, 2H: Ar-CH₂- éthyl) ; 3,51 (s, 3H: -OCH₃) ; 3,70 (d, J=7 Hz, 1H: -H 3) ; 3,82 (s, 3H: Ar-OCH₃) ; 4,09 [d, J=8 Hz, 1H: >(CH)-H 20] ; 4,17 (ab limite, J=16 Hz, 2H: -OCOCH₂O-) ; 4,25 [d, J=8 Hz, 1H: >(CH)-H 20] ; 4,42 (dd, J=11 et 7 Hz, 1H: -H 7) ; 4,57 (d, J=5 Hz, 1H: -H 2') ; 4,86 (d large, J=10 Hz, 1H: -H 5) ; 5,41 (mf, 1H: -H 3') ; 5,61 (d, J=7 Hz, 1H: -H 2) ; 6,06 (t large, J=9 Hz, 1H: -H 13) ; 6,39 (mf, 1H: -H 5') ; 6,44 (s, 1H: -H 10) ; 6,92 [d, J=8,5 Hz, 2H: -C₆H₅ en 5'(-H 3 et -H 5)] ; 7,30 (d, J= 8 Hz, 2H: -H aromatiques en ortho de l'éthyl) ; 7,40 [d, J=8,5 Hz, 2H: -C₆H₅ en 5' (-H 2 et -H 6)] ; 7,41 (mt, 5H: -C₆H₅ en 3') ; 7,95 (d, J=8 Hz, 2H: -H aromatiques en méta de l'éthyl)

En opérant comme dans l'exemple 6, on obtient le tert-butoxycarbonylamino-3 hydroxy-2 phényl-3 propionate-(2R,3S) d'acétoxy-4α dihydroxy-1β,7β époxy-5β,20 (éthyl-4 benzoyloxy)-2α méthoxyacétoxy-10β oxo-9 taxène-11 yle-13α dont les caractéristiques sont les suivantes :
- spectre de R.M.N. : (400 MHz ; CDCl₃ ; δ en ppm) : 1,16 (s, 3H: -CH₃ 16 ou 17) ; 1,26 (s, 3H: -CH₃ 16 ou 17) ; 1,28 (t, J=7,5 Hz, 3H: -CH₃ éthyl) ; 1,35 [s, 9H: -C(CH₃)₃] ; 1,70 (s, 3H: -CH₃ 19) ; 1,76 (s, 1H: -OH en 1) ; 1,88 (s, 3H: -CH₃ 18) ; 1,90 [mt, 1H: >(CH)-H 6] ; 2,30 (ab limite, J=16 et 9 Hz, 2H: -CH₂- 14) ; 2,38 (d, J=4,5 Hz, 1H: -OH en 7) ; 2,41 (s, 3H: -COCH₃) ; 2,58 [mt, 1H: >(CH)-H 6] ; 2,74 (q, J=7,5 Hz, 2H: ArCH₂- éthyl) ; 3,35 (d, J=4 Hz, 1H: -OH en 2') ; 3,53 (s, 3H: -OCH₃) ; 3,80 (d, J=7 Hz, 1H: -H3) 4,18 [d, J=8,5 Hz, 1H: >(CH)-H 20] ; 4,26 (ab limite, J=16 Hz, 2H: -OCOCH₂O-) ; 4,34 [d, J=8,5 Hz, 1H: >(CH)-H 20]; 4,43(mt, 1H: -H 7) ; 4,64 (mt, 1H: -H 2') ; 4,96 (d large, J=10 Hz, 1H: -H 5) ; 5,27 (d large, J=10 Hz, 1H: -H 3') ; 5,39 (d, J=10 Hz, 1H: -CONH-) ; 5,67 (d, J=7 Hz, 1H: -H 2) ; 6,24 (t, J=9 Hz, 1H: -H 13) ; 6,40 (s, 1H: -H 10) ; 7,34 (d, J= 8Hz, 2H: -H aromatiques en ortho de l'éthyl) ; de 7,30 à 7,45 (mt, 5H: -C₆H₅ en 3') ; 8,03 (d, J=8 Hz, 2H: -H aromatiques en méta de l'éthyl).

### EXEMPLE 19

En opérant comme dans l'exemple 7, on obtient le tert-butoxycarbonylamino-3 hydroxy-2 phényl-3 propionate-(2R,3S) d'acétoxy-4α époxy-5β,20 (éthyl-4 benzoyloxy)-2α oxo-9 trihydroxy-1β,7β,10β taxène-11 yle-13α dont les caractéristiques sont les suivantes :
- spectre de R.M.N.: (400 MHz ; CDCl₃ ; δ en ppm) : 1,14 (s, 3H: -CH₃ 16 ou 17) ; 1,26 (s, 3H: -CH₃ 16 ou 17) ; 1,28 (t, J=7,5 Hz, 3H: -CH₃ éthyl) ; 1,38 [s, 9 H: -C(CH₃)₃] ; 1,70 (s, 1H: -OH en 1) ; 1,78 (s, 3H: -CH₃ 19) ; 1,85 [mt, 1H: >(CH)-H 6] ; 1,86 (s, 3H: -CH₃ 18) ; 2,28 (ab limite, J=16 et 9 Hz, 2H: -CH₂-14) ; 2,38 (s, 3H: -COCH₃) ; 2,60 [mt, 1H: >(CH)-H 6] ; 2,73 (q, J=7,5 Hz, 2H: ArCH₂- éthyl) ; 3,35 (mf, 1H: -OH en 2') ; 3,91 (d, J=7 Hz, 1H: -H 3) ; 4,20 [d, J=8,5 Hz, 1H: >(CH)-H 20] ; 4,23 (s large, 1H: -OH en 10) ; 4,25 (mt, 1H: -H 7) ; 4,34 [d, J=8,5 Hz, 1H: >(CH)-H 20] ; 4,64 (mt, 1H: -H 2') ; 4,96 (d large, J=10 Hz, 1H: -H5) ; 5,21 (s large, 1H: -H 10) ; 5,27 (d large, J=10 Hz, 1H: -H 3') ; 5,45 (d, J=10 Hz, 1H: -CONH-) ; 5,67 (d, J=7 Hz, 1H: -H 2) ; 6,22 (t, J=9 Hz, 1H: -H 13) ; 7,33 (d, J= 8,5 Hz, 2H: -H aromatiques en ortho de l'éthyl) ; de 7,30 à 7,45 (mt, 5H: -C₆H₅ en 3') ; 8,03 (d, J=8,5 Hz, 2H: -H aromatiques en méta de l'éthyl).

### EXEMPLE 20

En opérant comme dans l'exemple 6 à partir de matières premières convenables, on obtient le tert-butoxycarbonyl-3 (méthoxy-4 phényl)-2 phényl-4 oxazolidine-1,3 carboxylate-5-(2R,4S,5R) d'acétoxy-4α époxy-5β,20 pentafluorobenzoyloxy-2α hydroxy-1β méthoxyacétoxy-10β oxo-9 triéthylsilyloxy-7β taxène-11 yle-13α dont les caractéristiques sont les suivantes :
- spectre de R.M.N. : (400 MHz ; CDCl₃ ; δ en ppm) : 0,57 (q, J=8 Hz, 6H: -CH₂-éthyl) ; 0,92 (t, J=8 Hz, 9H: -CH₃éthyl) ; 1,07 [s, 9H: -C(CH₃)₃] ; 1,16 (s, 3H: -CH₃ 16 ou 17) ; 1,19 (s, 3H: -CH₃ 16 ou 17) ; 1,54 (s, 1H: -OH en 1) ; 1,67 (s, 9H: -CH₃ 19, -CH₃ 18 et -COCH₃) ; 1,86 [mt, 1H: >(CH)-H 6] ; 2,05 et 2,19 (2 dd, J=16 et 9 Hz, 1H chacun: -CH₂- 14) ; 2,48 [mt, 1H: >CH)-H 6] ; 3,52 (s, 3H: -OCH₃) ; 3,68 (d, J=7 Hz, 1H: -H 3) ; 3,82 (s, 3H: Ar-OCH₃) ; 4,16 (ab limite, J= 16 Hz, 2H: -OCOCH₂O-) ; 4,18 (ab limite, J=8 Hz, 2H: -CH₂- 20) ; 4,38 (dd, J=11 et 6,5 Hz, 1H: -H7) ; 4,56 (d, J=5,5 Hz, 1H: -H 2') ; 4,82 (d large, J=10 Hz, 1H: -H5); 5,40 (mf, 1H: -H 3') ; 5,62 (d, J=7 Hz, 1H: -H 2) ; 6,07 (t large, J=9 Hz, 1H: -H 13) ; 6,37 (mf, 1H: -H 5') ; 6,43 (s, 1H: -H10) ; 6,92 [d, J=8,5 Hz, 2H: -C₆H₅ en 5'(-H 3 et -H 5)] ; de 7,35 à 7,45 [mt, 7H: -C₆H₅ en 3' et -C₆H₅ en 5'(-H 2 et -H 6)].

En opérant comme dans l'exemple 6, on obtient le tert-butoxycarbonylamino-3 hydroxy-2 phényl-3 propionate-(2R,3S) d'acétoxy-4α dihydroxy-1β,7β époxy-5β,20 pentafluorobenzoyloxy-2α méthoxyacétoxy-10β oxo-9 taxène-11 yle-13α dont les caractéristiques sont les suivantes :
- spectre de R.M.N.: (400 MHz ; CDCl₃ ; δ en ppm) : 1,14 (s, 3H: -CH₃ 16 ou 17) ; 1,27 (s, 3H: -CH₃ 16 ou 17) ; 1,42 [s, 9H: -C(CH₃)₃] ; 1,68 (s, 1H: -OH en 1) ; 1,72 (s, 3H: -CH₃ 19) ; 1,85 (s, 3H: -CH₃ 18) ; 1,91 [mt, 1H: >(CH)-H 6] ; 2,20 (s, 3H: -COCH₃) ; 2,23 et 2,35 (2dd, J= 16 et 9 Hz, 1H chacun: -CH₂- 14) ; 2,38 (d, J=4 Hz, 1H: -OH en 7) ; 2,57 [mt, 1H: >(CH)-H 6] ; 3,39 (d, J=4 Hz, 1H: -OH en 2') ; 3,53 (s, 3H: -OCH₃) ; 3,78 (d, J=7 Hz, 1H: -H 3) ; 4,27 (ab limite, J=16 Hz, 2H: -OCOCH₂O-) ; 4,30 (ab limite, J=8,5 Hz, 2H: -CH₂- 20) ; 4,39 (mt, 1H: -H 7) ; 4,57 (mt, 1H: -H 2') ; 4,94 (d large, J=10 Hz, 1H: -H 5) ; 5,19 (d large, J=10 Hz, 1H: -H3') ; 5,35 (d, J=10 Hz, 1H: -CONH-) ; 5,68 (d, J=7 Hz, 1H: -H 2) ; 6,16 (t, J=9 Hz, 1H: -H 13) ; 6,38 (s, 1H: -H 10) ; de 7,30 à 7,45 (mt, 5H: -C₆H₅ en 3').

### EXEMPLE 21

En opérant comme dans l'exemple 7, on obtient le tert-butoxycarbonylamino-3 hydroxy-2 phényl-3 propionate-(2R,3S) d'acétoxy-4α époxy-5β,20 pentafluorobenzoyloxy-2α oxo-9 trihydroxy-1β,7β,10β taxène-11 yle-13α dont les caractéristiques sont les suivantes :
- spectre de R.M.N. : (400 MHz ; CDCl₃ ; δ en ppm) : 1,12 (s, 3H: -CH₃ 16 ou 17) ; 1,26 (s, 3H: -CH₃ 16 ou 17) ; 1,43 [s, 9H: -C(CH₃)₃] ; 1,68 (d, J=12,5 Hz, 1H: -OH en 7) ; 1,78 (s, 3H: -CH₃ 19) ; de 1,75 à 1,90 [mt, 1H: >(CH)-H 6] ; 1,83 (s, 3H: -CH₃18) ; 2,19 (s, 3H: -COCH₃) ; 2,20 et 2,29 (2 dd, J=16 et 9 Hz, 1H chacun: -CH₂- 14) ; 2,58 [mt, 1H: >(CH)-H 6] ; 3,88 (d, J=7 Hz, 1H: -H 3) ; de 4,15 à 4,30 (mf, 1H: -OH en 10) ; 4,22 (mt, 1H: -H 7) ; 4,30 (ab limite, J=8,5 Hz, 2H: -CH₂- 20) ; 4,55 (mt, 1H: -H 2') ; 4,93 (d large, J=10 Hz, 1H: -H 5) ; 5,19 (d large, J=10 Hz, 1H: -H 3') ; 5,20 (s large, 1H: -H 10) ; 5,45 (d, J=10 Hz, 1H: -CONH-) ; 5,68 (d, J=7 Hz, 1H: -H 2) ; 6,15 (t, J=9 Hz, 1H: -H 13) ; de 7,30 à 7,45 (mt, 5H: -C₆H₅ en 3').

### EXEMPLE 22

En opérant comme dans l'exemple 6 à partir de matières premières convenables, on obtient le tert-butoxycarbonyl-3 (méthoxy-4 phényl)-2 phényl-4 oxazolidine-1,3 carboxylate-5-(2R,4S,5R) de diacétoxy-2α,4α époxy-5β,20 hydroxy-1β méthoxyacétoxy-10β oxo-9 triéthylsilyloxy-7β taxène-11 yle-13α dont les caractéristiques sont les suivantes :
- spectre de R.M.N.: (400 MHz ; CDCl₃ ; δ en ppm) : 0,59 (q, J=7,5 Hz, 6H: -CH₂-éthyl) ; 0,92 (t, J=7,5 Hz, 9H: -CH₃ éthyl) ; 1,09 [s, 9H: -C(CH₃)₃] ; 1,14 (s, 3H: -CH₃ 16 ou 17) ; 1,16 (s, 3H: -CH₃ 16 ou 17) ; 1,58 (s, 1H: -OH en 1) ; 1,60 (s, 3H: -CH₃ 19) ; 1,66 (s, 3H: -CH₃ 18) ; 1,82 (s, 3H: -COCH₃ en 4) ; 1,85 [mt, 1H: >(CH)-H 6] ; 1,95 [dd, J=16 et 9 Hz, 1H:- >(CH)-H 14] ; 2,03 (s, 3H: -COCH₃ en 2); 2,18 [dd, J=16 et 9 Hz, 1H: >(CH)-H14] ; 2,47 [mt, 1H: >(CH)-H 6] ; 3,51 (s, 3H: -OCH₃) ; 3,60 (d, J=7 Hz, 1H: -H 3) ; 3,81 (s, 3H: Ar-OCH₃) ; 4,13 (ab limite, J=16 Hz, 2H: -OCOCH₂O-) ; 4,15 [d, J=8 Hz, 1H: >(CH)-H 20] ; 4,40 [d, J= 8Hz, 1H: >(CH)-H 20] ; 4,40 (mt, 1H: -H 7) ; 4,53 (d, J=5 Hz, 1H: -H 2') ; 4,86 (d large, J=10 Hz, 1H: -H 5) ; 5,35 (d, J=7 Hz, 1H: -H 2) ; 5,46 (d, J=5 Hz, 1H: -H 3') ; 5,99 (t, J=9 Hz, 1H: -H 13) ; 6,37 (s, 1H: -H 5') ; 6,40 (s, 1H: -H 10) ; 6,92 [d, J=8,5 Hz, 2H: -C₆H₅ en 5'(-H 3 et -H 5)] ; de 7,30 à 7,50 (mt, 5H: -C₆H₅ en 3') ; 7,40 [d, J=8,5 Hz, 2H: -C₆H₅ en 5' (-H 2 et -H 6)].

En opérant comme dans l'exemple 6, on obtient le tert-butoxycarbonylamino-3 hydroxy-2 phényl-3 propionate-(2R,3S) de diacétoxy-2α,4α dihydroxy-1β,7β époxy-5β,20 méthoxyacétoxy-10β oxo-9 taxène-11 yle-13α dont les caractéristiques sont les suivantes :
- spectre de R.M.N. : (400 MHz ; CDCl₃ ; δ en ppm) : 1,10 (s, 3H: -CH₃ 16 ou 17) ; 1,24 (s, 3H: -CH₃ 16 ou 17) ; 1,43 [s, 9H: -C(CH₃)₃] ; 1,65 (s, 3H: -CH₃ 19) ; 1,85 (s, 3H: -CH₃ 18) ; 1,90 [mt, 1H: >(CH)-H 6] ; 2,13 (s, 3H: -COCH₃ en 2) ; 2,23 [mt, 1H: >(CH)-H 14] ; 2,26 (s, 3H: -COCH₃) ; 2,36 [mt, 2H: -OH en 7 et >(CH)-H 14] ; 2,58 [mt, 1H: >(CH)-H 6] ; 3,32 (d, J=5,5 Hz, 1H: -OH en 2') ; 3,53 (s, 3H: -OCH₃) ; 3,90 (d, J=7 Hz, 1H: -H 3) ; 4,22 [d, J= 8,5 Hz, 1H: >(CH)-H 20] ; 4,25 (ab limite, J=16 Hz, 2H: -OCOCH₂O-) ; 4,39 (mt, 1H: -H 7) ; 4,50 [d, J= 8,5 Hz, 1H: >(CH)-H20] ; 4,57 (dd, J= 5,5 et 2 Hz, 1H: -H 2') ; 4,96 (d large, J=10 Hz, 1H: -H 5) ; 5,20 (d large, J=10 Hz, 1H: -H 3') ; 5,37 (d, J=10 Hz, 1H: -CONH-) ; 5,41 (d, J=7 Hz, 1H: -H 2) ; 6,15 (t, J=9 Hz, 1H: -H13) ; 6,37 (s, 1H: -H 10) ; de 7,30 à 7,45 (mt, 5H: -C₆H₅ en 3').

### EXEMPLE 23

En opérant comme dans l'exemple 7, on obtient le tert-butoxycarbonylamino-3 hydroxy-2 phényl-3 propionate-(2R,3S) de diacétoxy-2α,4α trihydroxy-1β,7β,10β époxy-5β,20 oxo-9 taxène-11 yle-13α dont les caractéristiques sont les suivantes :
- spectre de R.M.N.: (400 MHz ; CDCl₃ ; δ en ppm) : 0,59 (q, J=7,5 Hz, 6H: -CH₂-éthyl) ; 0,92 (t, J=7,5 Hz, 9H: -CH₃ éthyl) ; 1,09 [s, 9H: -C(CH₃)₃] ; 1,14 (s, 3H: -CH₃ 16 ou 17) ; 1,16 (s, 3H: -CH₃ 16 ou 17) ; 1,58 (s, 1H: -OH en 1) ; 1,60 (s, 3H: -CH₃ 19); 1,66 (s, 3H: -CH₃ 18) ; 1,82 (s, 3H: -COCH₃ en 4) ; 1,85 [mt, 1H: >(CH)-H 6] ; 1,95 [dd, J=16 et 9 Hz, 1H: >(CH)-H 14]; 2,03 (s, 3H: -COCH₃ en 2); 2,18 [dd, J=16 et 9 Hz, 1H: >(CH)-H14] ; 2,47 [mt, 1H: >(CH)-H 6] ; 3,51 (s, 3H: -OCH₃) ; 3,60 (d, J=7 Hz, 1H: -H 3) ; 3,81 (s, 3H: Ar-OCH₃) ; 4,13 (ab limite, J=16 Hz, 2H: -OCOCH₂O-) ; 4,15 [d, J=8 Hz, 1H: >(CH)-H 20] ; 4,40 [d, J=8 Hz, 1H: >(CH)-H 20] ; 4,40 (mt, 1H: -H 7) ; 4,53 (d, J=5 Hz, 1H: -H 2') ; 4,86 (d large, J=10 Hz, 1H: -H 5) ; 5,35 (d, J=7 Hz, 1H: -H 2) ; 5,46 (d, J=5 Hz, 1H: -H 3') ; 5,99 (t, J= 9 Hz, 1H: -H 13) ; 6,37 (s, 1H: -H 5') ; 6,40 (s, 1H: -H 10) ; 6,92 [d, J=8,5 Hz, 2H: -C₆H₅ en 5'(-H 3 et -H 5)] ; de 7,30 à 7,50 (mt, 5H: -C₆H₅ en 3') ; 7,40 [d, J=8,5 Hz, 2H: -C₆H₅ en 5' (-H 2 et -H 6)].

## Revendications

1. Procédé de préparation de taxoïdes de formule générale : 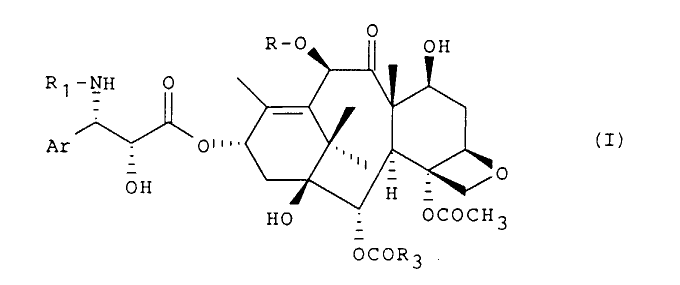 dans laquelle
Ar représente un radical aryle ou un radical hétérocyclique aromatique éventuellement substitué,
R représente un atome d'hydrogène ou un radical acétyle ou alcoxyacétyle,
R₁ représente un radical benzoyle ou un radical R₂-O-CO- dans lequel R₂ représente :
- un radical alcoyle droit ou ramifié contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone ou bicycloalcoyle contenant 7 à 11 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle, cyano, carboxy ou alcoyloxycarbonyle dont la partie alcoyle contient 1 à 4 atomes de carbone,
- ou un radical phényle éventuellement substitué par un ou plusieurs atomes ou radicaux choisis parmi les atomes d'halogène et les radicaux alcoyles contenant 1 à 4 atomes de carbone, alcoyloxy contenant 1 à 4 atomes de carbone,
- ou un radical hétérocyclyle saturé ou non saturé contenant 4 à 6 chaînons et éventuellement substitué par un ou plusieurs radicaux alcoyles contenant 1 à 4 atomes de carbone, et
R₃ représente
- un radical alcoyle droit ou ramifié contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone ou bicycloalcoyle contenant 7 à 11 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle éventuellement substitué par un ou plusieurs substituants choisis parmi les radicaux cyano, carboxy ou alcoyloxycarbonyle dont la partie alcoyle contient 1 à 4 atomes de carbone,
- ou un radical aryle éventuellement substitué par un ou plusieurs atomes ou radicaux, étant entendu que R₃ ne peut pas représenter un radical phényle non substitué,
- ou un radical hétérocyclyle saturé ou non saturé contenant 4 à 6 chaînons éventuellement substitué par un ou plusieurs atomes ou radicaux, étant entendu que les radicaux cycloalcoyles, cycloalcényles ou bicycloalcoyles peuvent être éventuellement substitués par un ou plusieurs radicaux alcoyles contenant 1 à 4 atomes de carbone,
caractérisé en ce que l'on estérifie un produit de formule générale : 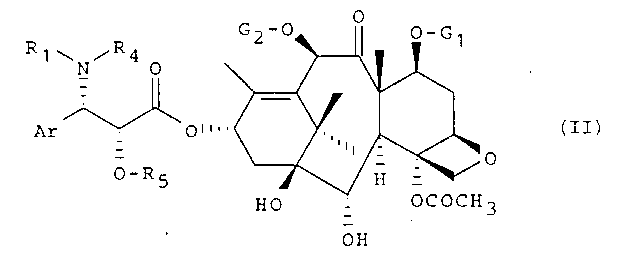 dans laquelle Ar et R₁ sont définis comme précédemment ou bien R₄ représente un atome d'hydrogène et R₅ représente un groupement protecteur de la fonction hydroxy, ou bien R₄ et R₅ forment ensemble un hétérocycle saturé à 5 ou 6 chaînons, G₁ représente un groupement protecteur de la fonction hydroxy et G₂ représente un radical acétyle ou un groupement protecteur de la fonction hydroxy, au moyen d'un acide de formule générale :
R₃-CO-OH (III)
dans laquelle R₃ est défini comme précédemment, ou d'un dérivé activé de cet acide, pour obtenir un produit de formule générale : 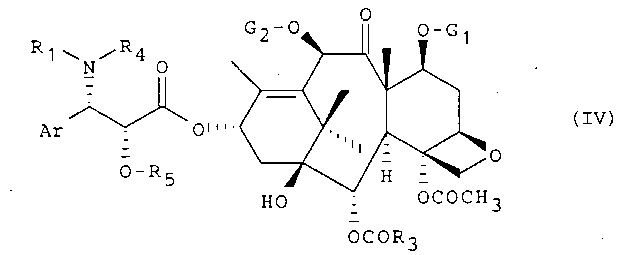 dans laquelle Ar, R₁, R₃, R₄, R₅, G₁ et G₂ sont définis comme précédemment, dont le remplacement des groupements protecteurs R₅, lorsque R₄ représente un atome d'hydrogène, ou bien R₄ et R₅, lorsque R₄ et R₅ forment ensemble un hétérocycle saturé à 5 ou 6 chainons, G₁ et éventuellement G₂ par des atomes d'hydrogène conduit au produit de formule générale (I) en passant éventuellement, selon les significations de R₁, R₄ et R₅, par un produit de formule générale : 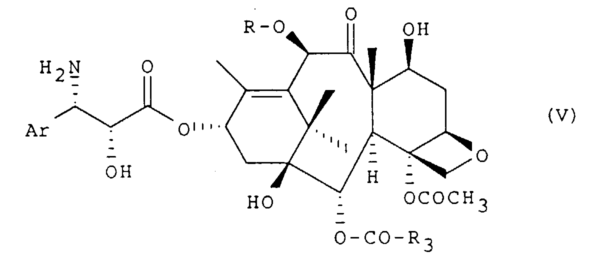 dans laquelle R est défini comme précédemment, qui est acylé au moyen de chlorure de benzoyle ou d'un produit de formule générale :
R₂-O-CO-X (VI)
dans laquelle R₂ est défini comme précédemment et X représente un atome d'halogène ou un reste -O-R₂ ou -O-CO-O-R₂.

2. Procédé selon la revendication 1 caractérisé en ce que l'estérification du produit de formule générale (II) est effectuée en faisant réagir l'acide de formule générale (III), de préférence sous forme d'halogénure, sur le produit de formule générale (II) préalablement métallé au moyen d'un alcoylure de métal alcalin en opérant dans un solvant organique inerte tel qu'un éther à une tempétature inférieure à -50°C.

3. Procédé selon la revendication 1 caractérisé en ce que le remplacement des groupements protecteurs de fonctions hydroxy par des atomes d'hydrogène est effectué :
1) lorsque R₄ représente un atome d'hydrogène, R₅ est défini comme précédemment et G₁ représente un radical silylé et G₂ représente un radical acétyle, en traitant le produit de formule générale (IV) par un acide minéral choisi parmi les acides chlorhydrique, sulfurique et fluorhydrique ou organique choisi parmi les acides formique, acétique, méthanesulfonique, trifluorométhanesulfonique, p.toluène-sulfonique utilisés seuls ou en mélange en opérant dans un solvant organique choisi parmi les alcools, les éthers, les esters, les hydrocarbures aliphatiques, les hydrocarbures aliphatiques halogénés, les hydrocarbures aromatiques ou les nitriles à une température comprise entre -10 et 60°C,
2) lorsque R₄ et R₅ forment ensemble un hétérocycle saturé de formule générale : 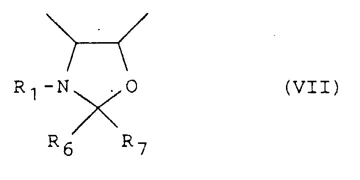 dans laquelle R₁ est défini comme dans l'une des revendications 1, 2 ou 3, R₆ et R₇, identiques ou différents, représentent un atome d'hydrogène ou un radical alcoyle contenant 1 à 4 atomes de carbone, ou un radical aralcoyle dont la partie alcoyle contient 1 à 4 atomes de carbone et la partie aryle représente, de préférence, un radical phényle éventuellement substitué par un ou plusieurs radicaux alcoxy, contenant 1 à 4 atomes de carbone, ou un radical aryle représentant, de préférence un radical phényle éventuellement substitué par un ou plusieurs radicaux alcoxy contenant 1 à 4 atomes de carbone, ou bien R₆ représente un radical alcoxy contenant 1 à 4 atomes de carbone ou un radical trihalométhyle ou un radical phényle substitué par un radical trihalométhyle et R₇ représente un atome d'hydrogène, ou bien R₆ et R₇ forment ensemble avec l'atome de carbone auquel ils sont liés un cycle ayant 4 à 7 chaînons, G₁ représente un radical silylé et G₂ représente un radical acétyle, est effectué, selon les significations de R_{1,} R₆ et R₇, de la manière suivante :
a) lorsque R₁ représente un radical t.butoxycarbonyle, R₆ et R₇, identiques ou différents, représentent un radical alcoyle ou un radical aralcoyle ou aryle, ou bien R₆ représente un radical trihalométhyle ou un radical phényle substitué par un radical trihalométhyle et R₇ représente un atome d'hydrogène, ou bien R₆ et R₇ forment ensemble un cycle ayant de 4 à 7 chaînons, en traitant un produit de formule générale (IV) par un acide minéral ou organique éventuellement dans un solvant organique tel qu'un alcool pour obtenir un produit de formule générale (V) qui est acylé au moyen d'un produit de formule générale (VI).
b) lorsque R₁ représente un radical benzoyle ou un radical R₂-O-CO- dans lequel R₂ est défini comme précédemment, R₆ représente un atome d'hydrogène ou un radical alcoxy contenant 1 à 4 atomes de carbone ou un radical phényle substitué par un ou plusieurs radicaux alcoxy contenant 1 à 4 atomes de carbone et R₇ représente un atome d'hydrogène, en traitant un produit de formule générale (IV) en présence d'une quantité catalytique ou stoechiométrique d'un acide minéral choisi parmi les acides chlorhydrique et sulfurique ou organique choisi parmi les acides acétique, méthanesulfonique, trifluorométhanesulfonique et p.toluènesulfonique utilisés seuls ou en mélange, dans un solvant organique choisi parmi les alcools, les éthers, les esters, les hydrocarbures aliphatiques, les hydrocarbures aliphatiques halogénés et les hydrocarbures aromatiques à une température comprise entre -10 et 60°C.
3) lorsque G₁ représente un radical silylé, G₂ représente un radical alcoxyacétyle et R₄ et R₅ sont définis comme au point 1) ci-dessus, en remplaçant d'abord les groupements protecteurs G₁ et R₅ par des atomes d'hydrogène en opérant dans les conditions acides décrites au point 1 ci-dessus, puis en remplaçant le groupement protecteur G₂ par un atome d'hydrogène par traitement en milieu alcalin au moyen d'ammoniac dans des conditions qui ne touchent pas au reste de la molécule ou au moyen d'un halogénure de zinc dans le méthanol,
4) lorsque G₁ représente un radical silylé, G₂ représente un radical alcoxyacétyle et R₄ et R₅ sont définis comme au point 2-a) ci-dessus, en remplaçant d'abord le groupement protecteur G₁ par traitement en milieu acide dans des conditions qui ne touchent pas au reste de la molécule puis en remplaçant éventuellement le groupement protecteur G₂ par un atome d'hydrogène dans les conditions décrites au point 3) ci-dessus, puis en traitant le produit de formule générale (V) obtenu dans les conditions de déprotection et d'acylation décrites au point 2-a) ci-dessus.
5) lorsque G₁ représente un radical silylé, G₂ représente un radical alcoxyacétyle et R₄ et R₅ sont définis comme au point 2-b) ci-dessus, en remplaçant d'abord le groupement protecteur G₁ par un traitement en milieu acide dans des conditions qui ne touchent pas au reste de la molécule, puis en remplaçant éventuellement le groupement protecteur G₂ par un atome d'hydrogène dans les conditions décrites au point 3) ci-dessus, puis traite le produit obtenu dans les conditions décrites au point 2-b) ci-dessus.

4. Procédé de préparation selon la revendication 1 pour la préparation d'un taxoïde de formule générale (I) dans laquelle R₁ et R₂ étant définis comme dans la revendication 1, les radicaux aryles représentés par Ar et R₃ sont des radicaux phényles ou α- ou β-naphtyles éventuellement substitués par un ou plusieurs atomes ou radicaux choisis parmi les atomes d'halogène (fluor, chlore, brome, iode) et les radicaux alcoyles, alcényles, alcynyles, aryles, arylalcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoyl-amino, carboxy, alcoxycarbonyle, carbamoyle, dialcoylcarbamoyle, cyano, nitro et trifluorométhyle, étant entendu que les radicaux alcoyles et les portions alcoyles des autres radicaux contiennent 1 à 4 atomes de carbone, que les radicaux alcényles et alcynyles contiennent 2 à 8 atomes de carbone et que les radicaux aryles sont des radicaux phényles ou α- ou β-naphtyles, et que le radical R₃ ne peut pas représenter un radical phényle non substitué, et les radicaux hétérocycliques représentés par Ar et R₃ sont des radicaux hétérocycliques aromatiques ayant 5 chaînons et contenant un ou plusieurs atomes, identiques ou différents, choisis parmi les atomes d'azote, d'oxygène ou de soufre, éventuellement substitués par un ou plusieurs substituants, identiques ou différents, choisis parmi les atomes d'halogène (fluor, chlore, brome, iode) et les radicaux alcoyles contenant 1 à 4 atomes de carbone, aryles contenant 6 à 10 atomes de carbone, alcoxy contenant 1 à 4 atomes de carbone, aryloxy contenant 6 à 10 atomes de carbone, amino, alcoylamino contenant 1 à 4 atomes de carbone, dialcoylamino dont chaque partie alcoyle contient 1 à 4 atomes de carbone, acylamino dont la partie acyle contient 1 à 4 atomes de carbone, alcoxycarbonyl-amino contenant 1 à 4 atomes de carbone, acyle contenant 1 à 4 atomes de carbone, arylcarbonyle dont la partie aryle contient 6 à 10 atomes de carbone, cyano, nitro, trifluorométhyle, carboxy, carbamoyle, alcoylcarbamoyle dont la partie alcoyle contient 1 à 4 atomes de carbone, dialcoylcarbamoyle dont chaque partie alcoyle contient 1 à 4 atomes de carbone ou alcoxycarbonyle dont la partie alcoxy contient 1 à 4 atomes de carbone.

5. Procédé de préparation selon la revendication 1 pour la préparation d'un taxoïde de formule générale (I) dans laquelle R₁ et R₂ étant définis comme dans la revendication 1, Ar représente un radical phényle, thiényle-2 ou -3 ou furyle-2 ou -3 ou thiazolyle-2 ou -4 ou -5 éventuellement substitué par un ou plusieurs atomes ou radicaux, identiques ou différents, choisis parmi les atomes d'halogène et les radicaux alcoyles, alcoxy, amino, alcoylamino, dialcoylamino, acylamino, alcoxycarbonylamino et trifluorométhyle et R₃ représente un radical phényle substitué par un ou plusieurs atomes ou radicaux, identiques ou différents, choisis parmi les atomes d'halogène et les radicaux alcoyles, alcoxy, amino, alcoylamino, dialcoylamino, acylamino, alcoxycarbonylamino, nitro et trifluoro-méthyle ou un radical thiényle-2 ou -3 ou furyle-2 ou -3 ou thiazolyle-2 ou -4 ou -5 éventuellement substitué par un ou plusieurs atomes ou radicaux, identiques ou différents, choisis parmi les atomes d'halogène et les radicaux alcoyles, alcoxy, amino, alcoylamino, dialcoylamino, acylamino, alcoxycarbonylamino et trifluorométhyle.

6. Procédé de préparation selon la revendication 1 pour la préparation d'un taxoïde de formule générale (I) dans laquelle R représente un atome d'hydrogène ou un radical acétyle ou méthoxyacétyle, Ar représente un radical phényle, R₁ représente un radical benzoyle ou tert-butoxycarbonyle et R₃ représente un radical fluoro-2 phényle, fluoro-3 phényle, fluoro-4 phényle, pentafluorophényle, éthyl-4 phényle, tert-butyl-4 phényle, nitro-3 phényle, nitro-4 phényle ou trifluorométhyl-4 phényle.

7. Le produit de formule générale : 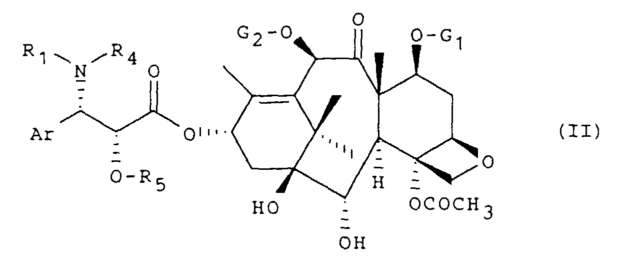 dans laquelle Ar et R₁ sont définis comme dans la revendication 1 et, ou bien R₄ représente un atome d'hydrogène et R₅ représente un groupement protecteur de la fonction hydroxy, ou bien R₄ et R₅ forment ensemble un hétérocycle saturé à 5 ou 6 chaînons, G₁ représente un groupement protecteur de la fonction hydroxy et G₂ représente un un radical acétyle ou un groupement protecteur de la fonction hydroxy.

8. Nouveau produit selon la revendication 7 caractérisé en ce que Ar et R₁ étant définis comme dans la revendication 1 et, lorsque R₄ représente un atome d'hydrogène, R₅ représente de préférence un radical méthoxy-méthyle, éthoxy-1 éthyle, benzyloxyméthyle, triméthylsilyle, triéthylsilyle, (β trimétylsilyléthoxy) méthyle ou tétrahydropyranyle et, lorsque R₄ et R₅ forment ensemble un hétérocycle, celui-ci est un cycle oxazolidine éventuellement mono-substitué ou gem-disubstitué en position -2 et G₁ représente un radical trialcoylsilyle, dialcoylarylsilyle, alcoyldiarylsilyle ou triarylsilyle dans lesquels les parties alcoyles contiennent 1 à 4 atomes de carbone et les parties aryles sont de préférence des radicaux phényles et G₂ représente un radical alcoxyacétyle.

9. Procédé de préparation d'un produit selon l'une des revendications 7 ou 8 caractérisé en ce que l'on effectue une réduction électrolytique d'un produit de formule générale : 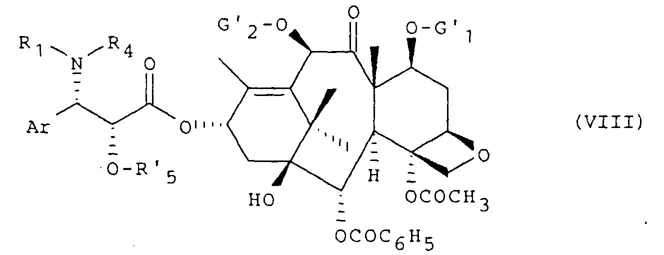 dans laquelle Ar et R₁ sont définis comme dans la revendication 1, R'₅ représente un atome d'hydrogène ou un groupement protecteur de la fonction hydroxy, R₄ représentant un atome d'hydrogène, G'₁ représente un atome d'hydrogène ou un groupement protecteur de la fonction hydroxy et G'₂ représente un atome d'hydrogène, un radical acétyle ou un groupement protecteur de la fonction hydroxy, en opérant dans un électrolyte constitué d'un sel d'ammonium quaternaire soluble dans le solvant organique ou dans un mélange hydro-organique à un potentiel contrôlé.

10. Procédé de préparation d'un produit selon l'une des revendications 7 ou 8 caractérisé en ce que l'on estérifie un produit de formule générale : 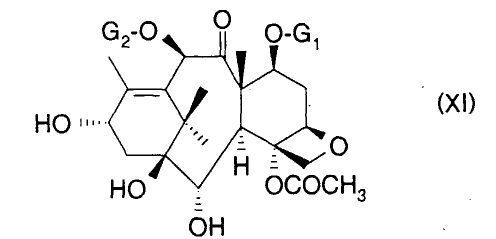 dans laquelle G₁ et G₂ sont définis comme dans l'une des revendications 7 ou 8 au moyen d'un acide de formule générale : 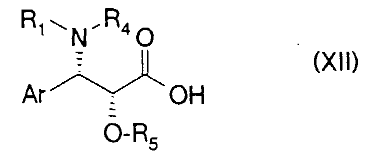 dans laquelle Ar, R₁, R₄ et R₅ sont définis comme dans l'une des revendications 7 ou 8, ou d'un dérivé de cet acide tel qu'un halogénure, l'anhydride ou un anhydride mixte en présence d'un agent d'activation tel qu'une aminopyridine et éventuellement d'un agent de condensation tel qu'un imide en opérant dans un solvant organique à une température comprise entre 0 et 90°C.

11. Procédé de préparation d'un produit de formule générale (XI) tel que défini dans la revendication 10 caractérisé en ce que l'on effectue la réduction électrolytique d'un produit de formule générale : 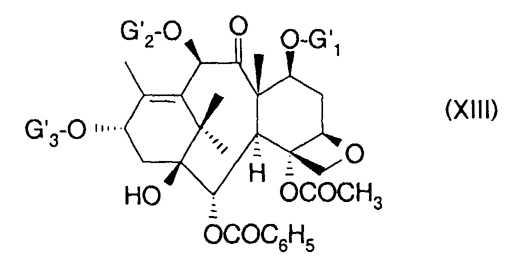 dans laquelle G'₁ et G'₂ sont définis comme précédemment et G'₃ représente un atome d'hydrogène ou un groupement protecteur de la fonction hydroxy identique à G'₁ en opérant dans un électrolyte constitué d'un sel d'ammonium quaternaire soluble dans le solvant organique ou dans un solvant hydro-organique à un potentiel contrôlé, puis selon le cas, remplace sélectivement le groupement protecteur G'₃ par un atome d'hydrogène ou protége sélectivement les fonctions hydroxy en positions -7 et -10.

## Patentansprüche

1. Verfahren zur Herstellung von Taxoiden der allgemeinen Formel (I) 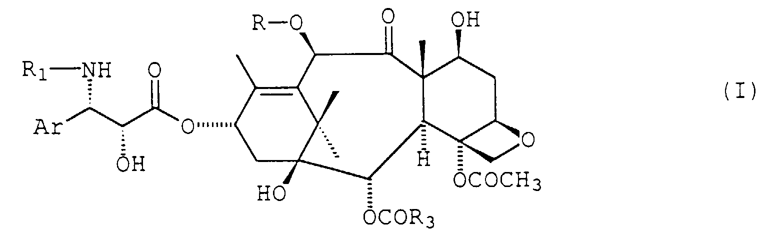 in der
Ar einen Rest Aryl oder einen aromatischen heterocyclischen Rest, gegebenenfalls substituiert, darstellt,
R ein Wasserstoffatom oder einen Rest Acetyl oder Alkoxyacetyl bedeutet,
R₁ einen Rest Benzoyl oder einen Rest R₂-O-CO- darstellt, worin R₂ ist:
- ein gerader oder verzweigter Rest Alkyl mit 1 bis 8 Kohlenstoffatomen, Alkenyl mit 2 bis 8 Kohlenstoffatomen, Alkinyl mit 3 bis 8 Kohlenstoffatomen, Cycloalkyl mit 3 bis 6 Kohlenstoffatomen, Cycloalkenyl mit 4 bis 6 Kohlenstoffatomen oder Bicycloalkyl mit 7 bis 11 Kohlenstoffatomen, wobei diese Reste gegebenenfalls substituiert sind durch einen oder mehrere Substituenten, ausgewählt unter den Halogenatomen und den Resten Hydroxy, Alkyloxy mit 1 bis 4 Kohlenstoffatomen, Dialkylamino, von dem jeder Alkylteil 1 bis 4 Kohlenstoffatome enthält, Piperidino, Morpholino, 1-Piperazinyl (gegebenenfalls substituiert in -4 durch einen Alkylrest mit 1 bis 4 Kohlenstoffatomen oder durch einen Phenylalkylrest, dessen Alkylteil 1 bis 4 Kohlenstoffatome enthält), Cycloalkyl mit 3 bis 6 Kohlenstoffatomen, Cycloalkenyl mit 4 bis 6 Kohlenstoffatomen, Phenyl, Cyano, Carboxy oder Alkyloxycarbonyl, dessen Alkylteil 1 bis 4 Kohlenstoffatome enthält,
- oder ein Rest Phenyl, gegebenenfalls substituiert durch ein oder mehrere Atome oder Reste, ausgewählt unter den Halogenatomen und den Resten Alkyl mit 1 bis 4 Kohlenstoffatomen oder Alkyloxy mit 1 bis 4 Kohlenstoffatomen,
- oder ein Rest eines gesättigten oder ungesättigten Heterocyclus mit 4 bis 6 Ringgliedern, gegebenenfalls substituiert durch einen oder mehrere Alkylreste mit 1 bis 4 Kohlenstoffatomen, und R₃ darstellt:
- einen geraden oder verzweigten Rest Alkyl mit 1 bis 8 Kohlenstoffatomen, Alkenyl mit 2 bis 8 Kohlenstoffatomen, Alkinyl mit 3 bis 8 Kohlenstoffatomen, Cycloalkyl mit 3 bis 6 Kohlenstoffatomen, Cycloalkenyl mit 4 bis 6 Kohlenstoffatomen oder Bicycloalkyl mit 7 bis 11 Kohlenstoffatomen, wobei diese Reste gegebenenfalls substituiert sind durch einen oder mehrere Substituenten, ausgewählt unter den Halogenatomen und den Resten Hydroxy, Alkyloxy mit 1 bis 4 Kohlenstoffatomen, Dialkylamino, von dem jeder Alkylteil 1 bis 4 Kohlenstoffatome enthält, Piperidino, Morpholino, 1-Piperazinyl (gegebenenfalls substituiert in -4 durch einen Alkylrest mit 1 bis 4 Kohlenstoffatomen oder durch einen Phenylalkylrest, dessen Alkylteil 1 bis 4 Kohlenstoffatome enthält), Cycloalkyl mit 3 bis 6 Kohlenstoffatomen, Cycloalkenyl mit 4 bis 6 Kohlenstoffatomen, Phenyl, gegebenenfalls substituiert durch einen oder mehrere Substituenten, ausgewählt unter den Resten Cyano, Carboxy oder Alkyloxycarbonyl, dessen Alkylteil 1 bis 4 Kohlenstoffatome enthält, oder
- einen Rest Aryl, gegebenenfalls substituiert durch ein oder mehrere Atome oder Reste, mit der Maßgabe, daß R₃ keinen unsubstituierten Phenylrest bedeuten kann,
- oder einen Rest eines gesättigten oder ungesättigten Heterocyclus mit 4 bis 6 Ringgliedern, gegebenenfalls substituiert durch einen oder mehrere Atome oder Reste,
mit der Maßgabe, daß die Reste Cycloalkyl, Cycloalkenyl oder Bicycloalkyl gegebenenfalls durch einen oder mehrere Alkylreste mit 1 bis 4 Kohlenstoffatomen substituiert sein können,
dadurch gekennzeichnet, daß man
ein Produkt der allgemeinen Formel (II) 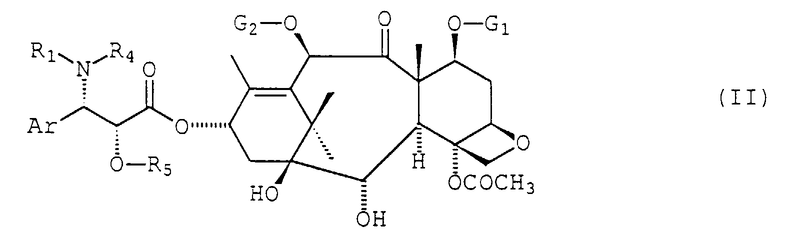 in der Ar und R₁ wie oben definiert sind, R₄ entweder ein Wasserstoffatom darstellt und R₅ eine Schutzgruppe für die Hydroxyfunktion bedeutet, oder R₄ und R₅ zusammen einen gesättigten Heterocyclus mit 5 oder 6 Ringgliedern bilden, G₁ eine Schutzgruppe für die Hydroxyfunktion darstellt und G₂ einen Acetylrest oder eine Schutzgruppe für die Hydroxyfunktion bedeutet, mit Hilfe einer Säure der allgemeinen Formel (III)
R₃-CO-OH (III)
oder einem aktivierten Derivat dieser Säure, worin R₃ wie oben definiert ist, verestert, um ein Produkt der allgemeinen Formel (IV) 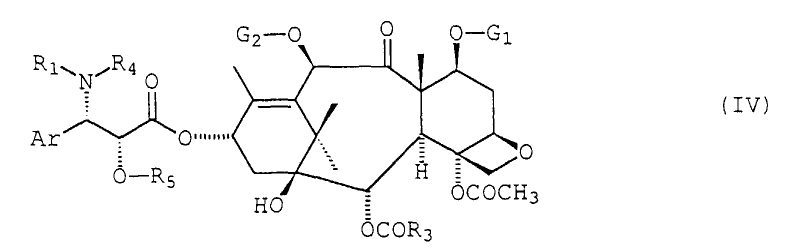 zu erhalten, in der Ar, R₁, R₃, R₄, R₅, G₁ und G₂ wie oben definiert sind, bei dem man die Schutzgruppen R₅, wenn R₄ ein Wasserstoffatom ist, oder R₄ und R₅, wenn R₄ und R₅ zusammen einen gesättigten Heterocyclus mit 5 oder 6 Ringgliedern bilden, G₁ und gegebenenfalls G₂ durch Wasserstoffatome austauscht, was zu einem Produkt der allgemeinen Formel (I) führt, gegebenenfalls je nach der Bedeutung von R₁, R₄ und R₅ über ein Produkt der allgemeinen Formel (V) 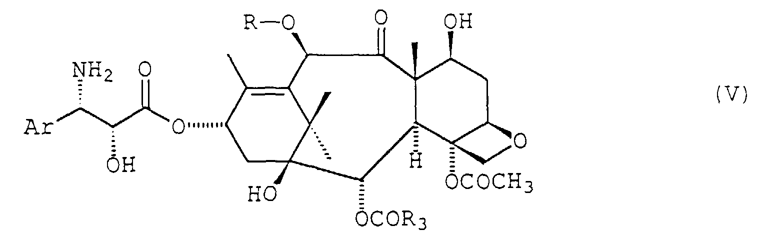 in der R wie oben definiert ist, das man anschließend mit Hilfe von Benzoylchlorid oder einem Produkt der allgemeinen Formel (VI)
R₂-O-CO-X (VI)
acyliert, in der R₂ wie oben definiert ist und X ein Halogenatom oder einen Rest -O-R₂ oder -O-CO-O-R₂ darstellt.

2. Verfahren nach Anspruch 1, dadurch gekennzeichnet, daß die Veresterung des Produktes der allgemeinen Formel (II) mittels Reaktion der Säure der allgemeinen Formel (III), vorzugsweise in Form des Halogenides, mit einem Produkt der allgemeinen Formel (II) durchgeführt wird, das zuvor mit Hilfe eines Alkalimetallalkyls metalliert wurde, wobei man in einem inerten organischen Lösungsmittel wie einem Ether und bei einer Temperatur von unter -50 °C arbeitet.

3. Verfahren nach Anspruch 1, dadurch gekennzeichnet, daß der Austausch der Schutzgruppen für die Hydroxyfunktionen durch Wasserstoffatome durchgeführt wird:
1) wenn R₄ ein Wasserstoffatom darstellt, R₅ wie oben definiert ist, G₁ einen Silylrest bedeutet und G₂ ein Acetylrest ist, unter Behandlung des Produktes der allgemeinen Formel (IV) mit einer Mineralsäure, ausgewählt unter Chlorwasserstoffsäure, Schwefelsäure und Fluorwasserstoffsäure, oder organischen Säure, ausgewählt unter Ameisensäure, Essigsäure, Methansulfonsäure, Trifluormethan-sulfonsäure und para-Toluolsulfonsäure, allein angewendet oder in Mischung, wobei man in einem organischen Lösungsmittel arbeitet, ausgewählt unter den Alkoholen, den Ethern, den Estern, den aliphatischen Kohlenwasserstoffen, den halogenierten aliphatischen Kohlenwasserstoffen, den aromatischen Kohlenwasserstoffen oder den Nitrilen, und bei einer Temperatur zwischen -10 °C und 60 °C,
2) wenn R₄ und R₅ zusammen einen gesättigten Heterocyclus der allgemeinen Formel (VII) 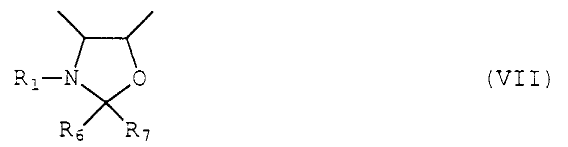 bilden, in der R₁ wie in einem der Ansprüche 1, 2 oder 3 definiert ist und R₆ und R₇, gleich oder verschieden, ein Wasserstoffatom oder einen Rest Alkyl mit 1 bis 4 Kohlenstoffatomen oder Aralkyl darstellen, dessen Alkylteil 1 bis 4 Kohlenstoffatome enthält und der Arylteil vorzugsweise einen Phenylrest bedeutet, gegebenenfalls substituiert durch einen oder mehrere Alkoxyreste mit 1 bis 4 Kohlenstoffatomen, oder einen Arylrest darstellen, der vorzugsweise ein Phenylrest ist, gegebenenfalls substituiert durch einen oder mehrere Alkoxyreste mit 1 bis 4 Kohlenstoffatomen, oder auch R₆ ein Alkoxyrest mit 1 bis 4 Kohlenstoffatomen oder ein Rest Trihalomethyl oder ein Phenylrest ist, substituiert durch einen Rest Trihalomethyl, und R₇ ein Wasserstoffatom bedeutet, oder auch R₆ und R₇ zusammen mit dem Kohlenstoffatom, an das sie gebunden sind, einen Zyklus mit 4 bis 7 Ringgliedern bilden, G₁ einen Silylrest darstellt und G₂ ein Acetylrest ist, je nach der Bedeutung von R₁, R₆ und R₇ in der folgenden Weise durchgeführt wird:
a) wenn R₁ einen Rest tert.-Butoxycarbonyl darstellt, R₆ und R₇, gleich oder verschieden, einen Rest Alkyl, Aralkyl oder Aryl bedeuten, oder auch R₆ ein Rest Trihalomethyl oder ein Phenylrest ist, substituiert durch einen Rest Trihalomethyl, und R₇ ein Wasserstoffatom bedeutet, oder auch R₆ und R₇ zusammen einen Zyklus mit 4 bis 7 Ringgliedern bilden, unter Behandlung des Produktes der allgemeinen Formel (IV) mit einer Mineralsäure oder organischen Säure, gegebenenfalls in einem organischen Lösungsmittel wie einem Alkohol, um ein Produkt der allgemeinen Formel (V) zu erhalten, das mit Hilfe eines Produktes der allgemeinen Formel (VI) acyliert wird,
b) wenn R₁ einen Rest Benzoyl oder ein Rest R₂-O-CO- darstellt, worin R₂ wie oben definiert ist, R₆ ein Wasserstoffatom oder einen Alkoxyrest mit 1 bis 4 Kohlenstoffatomen oder einen Phenylrest bedeutet, substituiert durch ein oder mehrere Alkoxyreste mit 1 bis 4 Kohlenstoffatomen, und R₇ ein Wasserstoffatom ist, unter Behandlung eines Produktes der allgemeinen Formel (IV) in Anwesenheit einer katalytischen oder stöchiometrischen Menge einer Mineralsäure, ausgewählt unter Chlorwasserstoffsäure und Schwefelsäure, oder organischen Säure, ausgewählt unter Essigsäure, Methansulfonsäure, Trifluormethan-sulfonsäure und para-Toluolsulfonsäure, allein angewendet oder in Mischung, wobei man in einem organischen Lösungsmittel arbeitet, ausgewählt unter den Alkoholen, den Ethern, den Estern, den aliphatischen Kohlenwasserstoffen, den halogenierten aliphatischen Kohlenwasserstoffen und den aromatischen Kohlenwasserstoffen, und bei einer Temperatur zwischen -10 °C und 60 °C,
3) wenn G₁ einen Silylrest darstellt, G₂ einen Rest Alkoxyacetyl bedeutet und R₄ und R₅ wie oben unter Punkt 1) definiert sind, zunächst unter Austausch der Schutzgruppen G₁ und R₅ durch Wasserstoffatome, wobei man unter den oben in Punkt 1) beschriebenen sauren Bedingungen arbeitet, und anschließend unter Austausch der Schutzgruppe G₂ durch ein Wasserstoffatom mittels Behandlung im alkalischen Medium mit Hilfe von Ammoniak unter Bedingungen, die den Rest des Moleküls nicht beeinträchtigen, oder auch mit Hilfe eines Zinkhalogenides in Methanol,
4) wenn G₁ einen Silylrest darstellt, G₂ einen Rest Alkoxyacetyl bedeutet und R₄ und R₅ wie oben unter Punkt 2-a) definiert sind, zunächst unter Austausch der Schutzgruppe G₁ durch Behandlung im sauren Medium unter Bedingungen, die den Rest des Moleküls nicht beeinträchtigen, und anschließend gegebenenfalls unter Austausch der Schutzgruppe G₂ durch ein Wasserstoffatom unter den oben in Punkt 3) beschriebenen Bedingungen, sowie mittels Behandlung des erhaltenen Produktes der allgemeinen Formel (V) unter den oben in Punkt 2-a) beschriebenen Bedingungen zur Abspaltung der Schutzgruppe und zur Acylierung,
5) wenn G₁ einen Silylrest darstellt, G₂ einen Rest Alkoxyacetyl bedeutet und R₄ und R₅ wie oben unter Punkt 2-b) definiert sind, zunächst unter Austausch der Schutzgruppe G₁ durch Behandlung im sauren Medium unter Bedingungen, die den Rest des Moleküls nicht beeinträchtigen, und anschließend gegebenenfalls unter Austausch der Schutzgruppe G₂ durch ein Wasserstoffatom unter den oben in Punkt 3) beschriebenen Bedingungen, sowie mittels Behandlung des erhaltenen Produktes unter den oben in Punkt 2-b) beschriebenen Bedingungen.

4. Verfahren zur Herstellung nach Anspruch 1 zur Herstellung eines Taxoides der allgemeinen Formel (I), in der R₁ und R₂ wie in Anspruch 1 definiert sind, die durch Ar und R₃ dargestellten Arylreste Phenylreste oder Reste α- oder β-Naphthyl sind, gegebenenfalls substituiert durch ein oder mehrere Atome oder Reste, ausgewählt unter den Halogenatomen (Fluor, Chlor, Brom, Iod) und den Resten Alkyl, Alkenyl, Alkinyl, Aryl, Arylalkyl, Alkoxy, Alkylthio, Aryloxy, Arylthio, Hydroxy, Hydroxyalkyl, Mercapto, Formyl, Acyl, Acylamino, Arylamino, Alkoxycarbonylamino, Amino, Alkylamino, Dialkylamino, Carboxy, Alkoxycarbonyl, Carbamoyl, Dialkylcarbamoyl, Cyano, Nitro und Trifluormethyl, mit der Maßgabe, daß die Alkylreste und Alkylteile der anderen Reste 1 bis 4 Kohlenstoffatome, die Reste Alkenyl und Alkinyl 2 bis 8 Kohlenstoffatome enthalten und die Arylreste Phenylreste oder Reste α- oder β-Naphthyl sind, und daß der Rest R₃ kein unsubstituierter Phenylrest sein kann, und die durch Ar und R₃ dargestellten heterocyclischen Reste aromatische heterocyclische Reste mit 5 Ringgliedern sind, die ein oder mehrere, gleiche oder verschiedene Atome enthalten, ausgewählt unter den Atomen von Stickstoff, Sauerstoff und Schwefel, gegebenenfalls substituiert durch einen oder mehrere, gleiche oder verschiedene Substituenten, ausgewählt unter den Halogenatomen (Fluor, Chlor, Brom, Iod) und den Resten Alkyl mit 1 bis 4 Kohlenstoffatomen, Aryl mit 6 bis 10 Kohlenstoffatomen, Alkoxy mit 1 bis 4 Kohlenstoffatomen, Aryloxy mit 6 bis 10 Kohlenstoffatomen, Amino, Alkylamino mit 1 bis 4 Kohlenstoffatomen, Dialkylamino, von dem jeder Alkylteil 1 bis 4 Kohlenstoffatome enthält, Acylamino, dessen Acylteil 1 bis 4 Kohlenstoffatome enthält, Alkoxycarbonylamino mit 1 bis 4 Kohlenstoffatomen, Acyl mit 1 bis 4 Kohlenstoffatomen, Arylcarbonyl, dessen Arylteil 6 bis 10 Kohlenstoffatome enthält, Cyano, Nitro, Trifluormethyl, Carboxy, Carbamoyl, Alkylcarbamoyl, dessen Alkylteil 1 bis 4 Kohlenstoffatome enthält, Dialkylcarbamoyl, von dem jeder Alkylteil 1 bis 4 Kohlenstoffatome enthält, oder Alkoxycarbonyl, dessen Alkoxyteil 1 bis 4 Kohlenstoffatome enthält.

5. Verfahren zur Herstellung nach Anspruch 1 zur Herstellung eines Taxoides der allgemeinen Formel (I), in der R₁ und R₂ wie in Anspruch 1 definiert sind, Ar einen Rest Phenyl, 2-Thienyl oder 3-Thienyl oder 2-Furyl oder 3-Furyl oder 2-Thiazolyl oder 4-Thiazolyl oder 5-Thiazolyl darstellt, gegebenenfalls substituiert durch ein oder mehrere, gleiche oder verschiedene Atome oder Reste, ausgewählt unter den Halogenatomen und den Resten Alkyl, Alkoxy, Amino, Alkylamino, Dialkylamino, Acylamino, Alkoxycarbonylamino und Trifluormethyl, und R₃ einen Phenylrest, substituiert durch ein oder mehrere, gleiche oder verschiedene Atome oder Reste, ausgewählt unter den Halogenatomen und den Resten Alkyl, Alkoxy, Amino, Alkylamino, Dialkylamino, Acylamino, Alkoxycarbonylamino, Nitro und Trifluormethyl, oder einen Rest 2-Thienyl oder 3-Thienyl oder 2-Furyl oder 3-Furyl oder 2-Thiazolyl oder 4-Thiazolyl oder 5-Thiazolyl bedeutet, gegebenenfalls substituiert durch ein oder mehrere, gleiche oder verschiedene Atome oder Reste, ausgewählt unter den Halogenatomen und den Resten Alkyl, Alkoxy, Amino, Alkylamino, Dialkylamino, Acylamino, Alkoxycarbonylamino und Trifluormethyl.

6. Verfahren zur Herstellung nach Anspruch 1 zur Herstellung eines Taxoides der allgemeinen Formel (I), in der R ein Wasserstoffatom oder einen Rest Acetyl oder Methoxyacetyl darstellt, Ar ein Phenylrest ist, R₁ einen Rest Benzoyl oder tert.-Butoxycarbonyl bedeutet und R₃ einen Rest 2-Fluorphenyl, 3-Fluorphenyl, 4-Fluorphenyl, Pentafluorphenyl, 4-Ethylphenyl, 4-tert.-Butylphenyl, 3-Nitrophenyl, 4-Nitrophenyl oder 4-Trifluormethylphenyl darstellt.

7. Das Produkt der allgemeinen Formel (II) 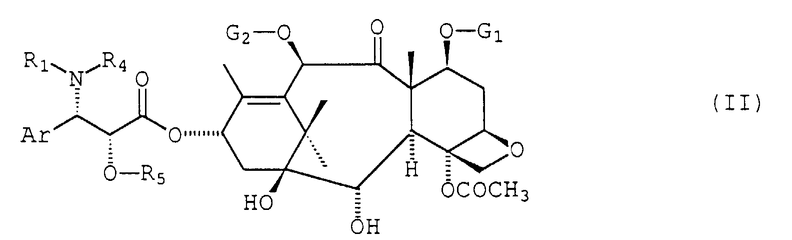 in der Ar und R₁ wie in Anspruch 1 definiert sind und R₄ entweder ein Wasserstoffatom darstellt und R₅ eine Schutzgruppe für die Hydroxyfunktion bedeutet, oder R₄ und R₅ zusammen einen gesättigten Heterocyclus mit 5 oder 6 Ringgliedern bilden, G₁ eine Schutzgruppe für die Hydroxyfunktion darstellt und G₂ einen Acetylrest oder eine Schutzgruppe für die Hydroxyfunktion bedeutet.

8. Neues Produkt nach Anspruch 7, dadurch gekennzeichnet, daß Ar und R₁ wie in Anspruch 1 definiert sind und, wenn R₄ ein Wasserstoffatom ist, R₅ vorzugsweise einen Rest Methoxymethyl, 1-Ethoxyethyl, Benzyloxymethyl, Trimethylsilyl, Triethylsilyl, (β-Trimethylsilylethoxy)-methyl oder Tetrahydropyranyl darstellt, und, wenn R₄ und R₅ zusammen einen Heterocyclus bilden, dieser ein Oxazolidinring ist, gegebenenfalls in Position -2 monosubstituiert oder geminal disubstituiert, G₁ einen Rest Trialkylsilyl, Dialkylarylsilyl, Alkyldiarylsilyl oder Triarylsilyl bedeutet, worin die Alkylteile 1 bis 4 Kohlenstoffatome enthalten und die Arylteile vorzugsweise Phenylreste sind, und G₂ ein Rest Alkoxyacetyl ist.

9. Verfahren zur Herstellung eines Produktes nach einem der Ansprüche 7 oder 8, dadurch gekennzeichnet, daß man eine elektrolytische Reduktion bei einem Produkt der allgemeinen Formel (VIII) 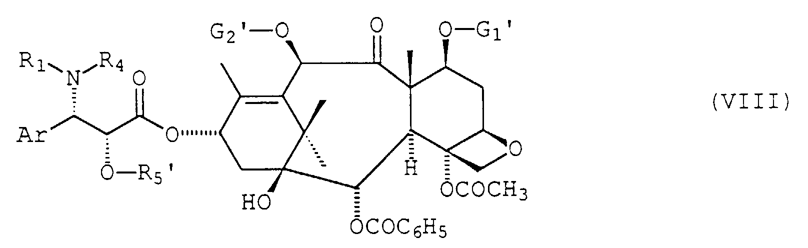 durchführt, in der Ar und R₁ wie in Anspruch 1 definiert sind, R₅' ein Wasserstoffatom oder eine Schutzgruppe für die Hydroxyfunktion bedeutet, R₄ ein Wasserstoffatom ist, G₁' ein Wasserstoffatom oder eine Schutzgruppe für die Hydroxyfunktion darstellt und G₂' ein Wasserstoffatom, einen Acetylrest oder eine Schutzgruppe für die Hydroxyfunktion bedeutet, wobei man in einem Elektrolyten arbeitet, der aus einem in dem organischen Lösungsmittel oder in einer wäßrig-organischen Mischung löslichen quaternären Ammoniumsalz besteht, sowie bei einem geregelten Potential.

10. Verfahren zur Herstellung eines Produktes nach einem der Ansprüche 7 oder 8, dadurch gekennzeichnet, daß man ein Produkt der allgemeinen Formel (XI) 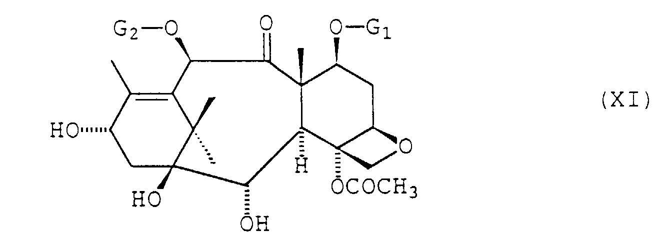 in der G₁ und G₂ wie in einem der Ansprüche 7 oder 8 definiert sind, mit Hilfe einer Säure der allgemeinen Formel (XII) 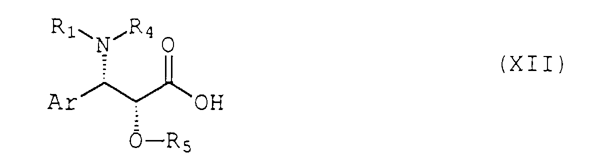 in der Ar, R₁, R₄ und R₅ wie in einem der Ansprüche 7 oder 8 definiert sind, oder einem Derivat dieser Säure, wie beispielsweise einem Halogenid, dem Anhydrid oder einem gemischten Anhydrid, verestert, wobei man in Anwesenheit eines Aktivierungsmittels wie einem Aminopyridin und gegebenenfalls eines Kondensationsmittels wie einem Imid sowie in einem organischen Lösungsmittel bei einer Temperatur zwischen 0 °C und 90 °C arbeitet.

11. Verfahren zur Herstellung eines Produktes der allgemeinen Formel (XI) wie in Anspruch 10 definiert, dadurch gekennzeichnet, daß man eine elektrolytische Reduktion bei einem Produkt der allgemeinen Formel (XIII) 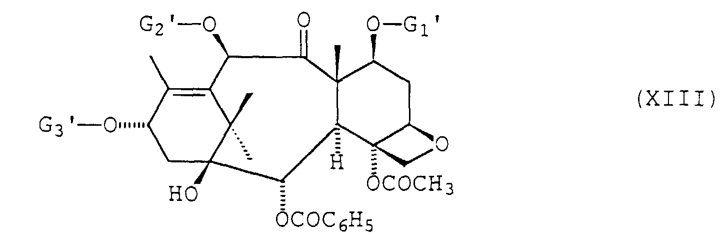 durchführt, in der G₁' und G₂' wie vorstehend definiert sind und G₃' ein Wasserstoffatom oder eine Schutzgruppe für die Hydroxyfunktion darstellt, die mit G₁' identisch ist, wobei man in einem Elektrolyten arbeitet, der aus einem in dem organischen Lösungsmittel oder in einer wäßrig-organischen Mischung löslichen quaternären Ammoniumsalz besteht, sowie bei einem geregelten Potential, und man anschließend je nach Fall selektiv die Schutzgruppe G₃' durch ein Wasserstoffatom ersetzt oder selektiv die Hydroxyfunktionen in den Positionen -7 und -10 schützt.

## Claims

1. Process for the preparation of taxoids of general formula: 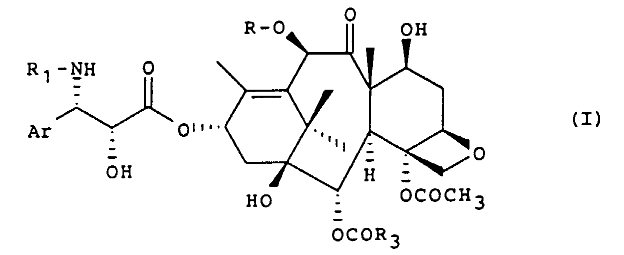 in which
Ar represents an aryl radical or an aromatic heterocyclic radical which is optionally substituted,
R represents a hydrogen atom or an acetyl or alkoxyacetyl radical,
R₁ represents a benzoyl radical or a radical R₂-O-CO- in which R₂ represents:
- a straight or branched alkyl radical containing 1 to 8 carbon atoms, an alkenyl radical containing 2 to 8 carbon atoms, an alkynyl radical containing 3 to 8 carbon atoms, a cycloalkyl radical containing 3 to 6 carbon atoms, a cycloalkenyl radical containing 4 to 6 carbon atoms or a bicycloalkyl radical containing 7 to 11 carbon atoms, these radicals optionally being substituted by one or a number of substituents chosen from halogen atoms and hydroxyl, alkyloxy, containing 1 to 4 carbon atoms, dialkylamino, in which each alkyl part contains 1 to 4 carbon atoms, piperidino, morpholino, 1-piperazinyl (optionally substituted in the 4-position by an alkyl radical containing 1 to 4 carbon atoms or by a phenylalkyl radical in which the alkyl part contains 1 to 4 carbon atoms), cycloalkyl, containing 3 to 6 carbon atoms, cycloalkenyl, containing 4 to 6 carbon atoms, phenyl, cyano, carboxyl or alkyloxycarbonyl, in which the alkyl part contains 1 to 4 carbon atoms, radicals,
- or a phenyl radical optionally substituted by one or a number of atoms or radicals chosen from halogen atoms and alkyl, containing 1 to 4 carbon atoms, or alkyloxy, containing 1 to 4 carbon atoms, radicals,
- or a saturated or unsaturated heterocyclyl radical containing 4 to 6 members and optionally substituted by one or a number of alkyl radicals containing 1 to 4 carbon atoms, and
R₃ represents
- a straight or branched alkyl radical containing 1 to 8 carbon atoms, an alkenyl radical containing 2 to 8 carbon atoms, an alkynyl radical containing 3 to 8 carbon atoms, a cycloalkyl radical containing 3 to 6 carbon atoms, a cycloalkenyl radical containing 4 to 6 carbon atoms or a bicycloalkyl radical containing 7 to 11 carbon atoms, these radicals optionally being substituted by one or a number of substituents chosen from halogen atoms and the following radicals: hydroxyl, alkyloxy, containing 1 to 4 carbon atoms, dialkylamino, in which each alkyl part contains 1 to 4 carbon atoms, piperidino, morpholino, 1-piperazinyl (optionally substituted in the 4-position by an alkyl radical containing 1 to 4 carbon atoms or by a phenylalkyl radical in which the alkyl part contains 1 to 4 carbon atoms), cycloalkyl, containing 3 to 6 carbon atoms, cycloalkenyl, containing 4 to 6 carbon atoms, phenyl, optionally substituted by one or a number of substituents chosen from cyano, carboxyl or alkyloxycarbonyl, in which the alkyl part contains 1 to 4 carbon atoms, radicals,
- or an aryl radical optionally substituted by one or a number of atoms or radicals, it being understood that R₃ cannot represent an unsubstituted phenyl radical,
- or a saturated or unsaturated heterocyclyl radical containing 4 to 6 members and optionally substituted by one or a number of atoms or radicals,
it being understood that the cycloalkyl, cycloalkenyl or bicycloalkyl radicals can optionally be substituted by one or a number of alkyl radicals containing 1 to 4 carbon atoms,
characterized in that a product of general formula: 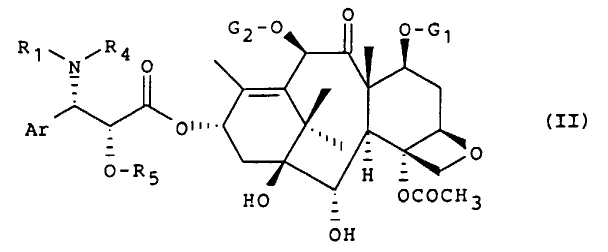 in which Ar and R₁ are defined as above, or either R₄ represents a hydrogen atom and R₅ represents a protective group of the hydroxyl functional group, or else R₄ and R₅ together form a saturated 5- or 6-membered heterocycle, G₁ represents a protective group of the hydroxyl functional group and G₂ represents an acetyl radical or a protective group of the hydroxyl functional group, is esterified by means of an acid of general formula:
R₃-CO-OH (III)
in which R₃ is defined as above, or of an activated derivative of this acid, to obtain a product of general formula: 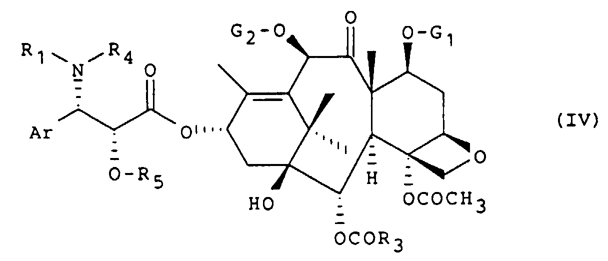 in which Ar, R₁, R₃, R₄, R₅, G₁ and G₂ are defined as above, the replacement of the protective groups R₅, when R₄ represents a hydrogen atom, or else R₄ and R₅, when R₄ and R₅ together form a saturated 5- or 6-membered heterocycle, G₁ and optionally G₂ of which by hydrogen atoms leads to the product of general formula (I), optionally passing, depending on the meanings of R₁, R₄ and R₅, through a product of general formula: 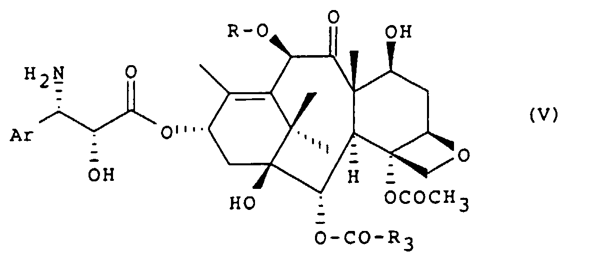 in which R is defined as above, which is acylated by means of benzoyl chloride or of a product of general formula:
R₂-O-CO-X (VI)
in which R₂ is defined as above and X represents a halogen atom or an -O-R₂ or -O-CO-O-R₂ residue.

2. Process according to claim 1, characterized in that the esterification of the product of general formula (II) is carried out by reacting the acid of general formula (III), preferably in the halide form, with the product of general formula (II) metallated beforehand by means of an alkali metal alkylide, the reaction being carried out in an inert organic solvent such as an ether at a temperature below -50°C.

3. Process according to claim 1, characterized in that replacement of the protective groups of hydroxyl functional groups by hydrogen atoms is carried out:
1) when R₄ represents a hydrogen atom, R₅ is defined as above and G₁ represents a silylated radical and G₂ represents an acetyl radical, by treating the product of general formula (IV) with an inorganic acid chosen from hydrochloric, sulphuric and hydrofluoric acids or an organic acid chosen from formic, acetic, methanesulphonic, trifluoromethanesulphonic and p-toluenesulphonic acids, used alone or as a mixture, the reaction being carried out in an organic solvent chosen from alcohols, ethers, esters, aliphatic hydrocarbons, halogenated aliphatic hydrocarbons, aromatic hydrocarbons or nitriles at a temperature between -10 and 60°C,
2) when R₄ and R₅ together form a saturated heterocycle of general formula: 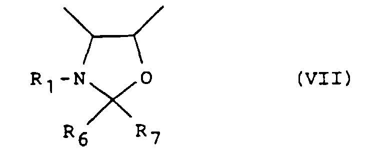 in which R₁ is defined as in one of claims 1, 2 or 3, R₆ and R₇, which are identical or different, represent a hydrogen atom or an alkyl radical containing 1 to 4 carbon atoms, or an aralkyl radical in which the alkyl part contains 1 to 4 carbon atoms and the aryl part preferably represents a phenyl radical optionally substituted by one or a number of alkoxy radicals containing 1 to 4 carbon atoms, or an aryl radical preferably representing a phenyl radical optionally substituted by one or a number of alkoxy radicals containing 1 to 4 carbon atoms, or else R₆ represents an alkoxy radical containing 1 to 4 carbon atoms or a trihalomethyl radical or a phenyl radical substituted by a trihalomethyl radical and R₇ represents a hydrogen atom, or else R₆ and R₇ form, together with the carbon atom to which they are bonded, a ring having 4 to 7 members, G₁ represents a silylated radical and G₂ represents an acetyl radical, in the following way, depending on the meanings of R₁, R₆ and R₇:
a) when R₁ represents a t-butoxycarbonyl radical, R₆ and R₇, which are identical or different, represent an alkyl radical or an aralkyl or aryl radical, or else R₆ represents a trihalomethyl radical or a phenyl radical substituted by a trihalomethyl radical and R₇ represents a hydrogen atom, or else R₆ and R₇ together form a ring having 4 to 7 members, by treating a product of general formula (IV) with an inorganic or organic acid, optionally in an organic solvent such as an alcohol, to obtain a product of general formula (V) which is acylated by means of a product of general formula (VI).
b) when R₁ represents a benzoyl radical or a radical R₂-O-CO- in which R₂ is defined as above, R₆ represents a hydrogen atom or an alkoxy radical containing 1 to 4 carbon atoms or a phenyl radical substituted by one or a number of alkoxy radicals containing 1 to 4 carbon atoms and R₇ represents a hydrogen atom, by treating a product of general formula (IV) in the presence of a catalytic or stoichiometric amount of an inorganic acid chosen from hydrochloric and sulphuric acids or an organic acid chosen from acetic, methanesulphonic, trifluoromethanesulphonic and p-toluenesulphonic acids, used alone or as a mixture, in an organic solvent chosen from alcohols, ethers, esters, aliphatic hydrocarbons, halogenated aliphatic hydrocarbons and aromatic hydrocarbons at a temperature between -10 and and 60°C.
3) when G₁ represents a silylated radical, G₂ represents an alkoxyacetyl radical and R₄ and R₅ are defined as in point 1) above, by first replacing the protective groups G₁ and R₅ by hydrogen atoms, the reaction being carried out under the acidic conditions described in point 1 above, and then by replacing the protective group G₂ by a hydrogen atom by treatment in alkaline medium by means of ammonia, under conditions which do not affect the remainder of the molecule, or by means of a zinc halide in methanol,
4) when G₁ represents a silylated radical, G₂ represents an alkoxyacetyl radical and R₄ and R₅ are defined as in point 2-a) above, by first replacing the protective group G₁ by treatment in acidic medium under conditions which do not affect the remainder of the molecule, by then optionally replacing the protective group G₂ by a hydrogen atom under the conditions described in point 3) above, and then by treating the product of general formula (V) obtained under the deprotection and acylation conditions described in point 2-a) above.
5) when G₁ represents a silylated radical, G₂ represents an alkoxyacetyl radical and R₄ and R₅ are defined as in point 2-b) above, by first replacing the protective group G₁ by a treatment in acidic medium under conditions which do not affect the remainder of the molecule, by then optionally replacing the protective group G₂ by a hydrogen atom under the conditions described in point 3) above, and then by treating the product obtained under the conditions described in point 2-b) above.

4. Preparation process according to claim 1 for the preparation of a taxoid of general formula (I) in which, R₁ and R₂ being defined as in claim 1, the aryl radicals represented by Ar and R₃ are phenyl or α- or β-naphthyl radicals optionally substituted by one or a number of atoms or radicals chosen from halogen atoms (fluorine, chlorine, bromine, iodine) and alkyl, alkenyl, alkynyl, aryl, arylalkyl, alkoxy, alkylthio, aryloxy, arylthio, hydroxyl, hydroxyalkyl, mercapto, formyl, acyl, acylamino, aroylamino, alkoxycarbonylamino, amino, alkylamino, dialkylamino, carboxyl, alkoxycarbonyl, carbamoyl, dialkylcarbamoyl, cyano, nitro and trifluoromethyl radicals, it being understood that the alkyl radicals and the alkyl portions of the other radicals contain 1 to 4 carbon atoms, that the alkenyl and alkynyl radicals contain 2 to 8 carbon atoms and that the aryl radicals are phenyl or α- or β-naphthyl radicals, and that the R₃ radical cannot represent an unsubstituted phenyl radical, and the heterocyclic radicals represented by Ar and R₃ are aromatic heterocyclic radicals having 5 members and containing one or a number of atoms, which are identical or different, chosen from nitrogen, oxygen or sulphur atoms, optionally substituted by one or a number of substituents, which are identical or different, chosen from halogen atoms (fluorine, chlorine, bromine, iodine) and alkyl, containing 1 to 4 carbon atoms, aryl, containing 6 to 10 carbon atoms, alkoxy, containing 1 to 4 carbon atoms, aryloxy, containing 6 to 10 carbon atoms, amino, alkylamino, containing 1 to 4 carbon atoms, dialkylamino, in which each alkyl part contains 1 to 4 carbon atoms, acylamino, in which the acyl part contains 1 to 4 carbon atoms, alkoxycarbonylamino, containing 1 to 4 carbon atoms, acyl, containing 1 to 4 carbon atoms, arylcarbonyl, in which the aryl part contains 6 to 10 carbon atoms, cyano, nitro, trifluoromethyl, carboxyl, carbamoyl, alkylcarbamoyl, in which the alkyl part contains 1 to 4 carbon atoms, dialkylcarbamoyl, in which each alkyl part contains 1 to 4 carbon atoms, or alkoxycarbonyl, in which the alkoxy part contains 1 to 4 carbon atoms, radicals.

5. Preparation process according to claim 1 for the preparation of a taxoid of general formula (I) in which, R₁ and R₂ being defined as in claim 1, Ar represents a phenyl, 2- or 3-thienyl or 2- or 3-furyl or 2- or 4- or 5-thiazolyl radical, optionally substituted by one or a number of atoms or radicals, which are identical or different, chosen from halogen atoms and alkyl, alkoxy, amino, alkylamino, dialkylamino, acylamino, alkoxycarbonylamino and trifluoromethyl radicals, and R₃ represents a phenyl radical substituted by one or a number of atoms or radicals, which are identical or different, chosen from halogen atoms and alkyl, alkoxy, amino, alkylamino, dialkylamino, acylamino, alkoxycarbonylamino, nitro and trifluoromethyl radicals or a 2-or 3-thienyl or 2- or 3-furyl or 2- or 4- or 5-thiazolyl radical, optionally substituted by one or a number of atoms or radicals, which are identical or different, chosen from halogen atoms and alkyl, alkoxy, amino, alkylamino, dialkylamino, acylamino, alkoxycarbonylamino and trifluoromethyl radicals.

6. Preparation process according to claim 1 for the preparation of a taxoid of general formula (I) in which R represents a hydrogen atom or an acetyl or methoxyacetyl radical, Ar represents a phenyl radical, R₁ represents a benzoyl or tert-butoxycarbonyl radical and R₃ represents a 2-fluorophenyl, 3-fluorophenyl, 4-fluorophenyl, pentafluorophenyl, 4-ethylphenyl, 4-tert-butylphenyl, 3-nitrophenyl, 4-nitrophenyl or 4-(trifluoromethyl)phenyl radical.

7. The product of general formula: 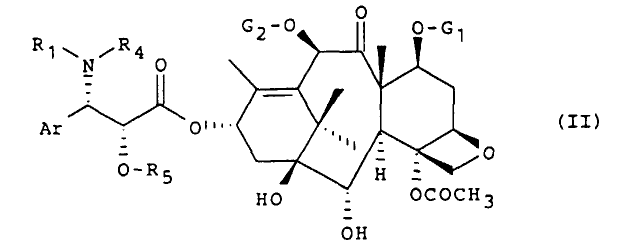 in which Ar and R₁ are defined as in claim 1, and either R₄ represents a hydrogen atom and R₅ represents a protective group of the hydroxyl functional group or R₄ and R₅ together form a saturated 5- or 6-membered heterocycle, G₁ represents a protective group of the hydroxyl functional group and G₂ represents an acetyl radical or else a protective group of the hydroxyl functional group.

8. New product according to claim 7, characterized in that, Ar and R₁ being defined as in claim 1, when R₄ represents a hydrogen atom, R₅ preferably represents a methoxymethyl, 1-ethoxyethyl, benzyloxymethyl, trimethylsilyl, triethylsilyl, (β-trimethylsilylethoxy)methyl or tetrahydropyranyl radical and, when R₄ and R₅ together form a heterocycle, the latter is an oxazolidine ring optionally monosubstituted or gem-disubstituted in the 2-position and G₁ represents a trialkylsilyl, dialkylarylsilyl, alkyldiarylsilyl or triarylsilyl radical in which the alkyl parts contain 1 to 4 carbon atoms and the aryl parts are preferably phenyl radicals and G₂ represents an alkoxyacetyl radical.

9. Process for the preparation of a product according to either of claims 7 and 8, characterized in that an electrolytic reduction of a product of general formula: 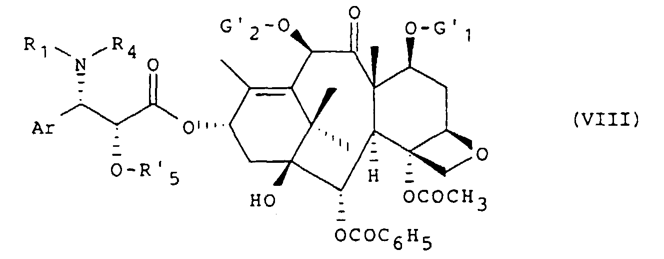 in which Ar and R₁ are defined as in claim 1, R'₅ represents a hydrogen atom or a protective group of the hydroxyl functional group, R₄ representing a hydrogen atom, G'₁ represents a hydrogen atom or a protective group of the hydroxyl functional group and G'₂ represents a hydrogen atom, an acetyl radical or a protective group of the hydroxyl functional group, is carried out, the electrolytic reduction taking place in an electrolyte consisting of a quaternary ammonium salt which is soluble in the organic solvent or in an aqueous/organic mixture at a controlled potential.

10. Process for the preparation of a product according to either of claims 7 and 8, characterized in that a product of general formula: 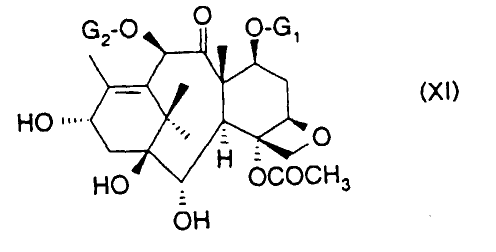 in which G₁ and G₂ are defined as in either of claims 7 and 8, is esterified by means of an acid of general formula: 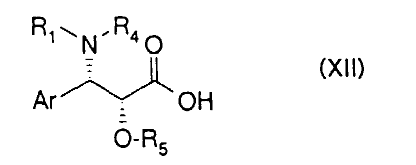 in which Ar, R₁, R₄ and R₅ are defined as in either of claims 7 and 8, or of a derivative of this acid such as a halide, the anhydride or a mixed anhydride in the presence of an activating agent such as an aminopyridine and optionally of a condensation agent such as an imide, the reaction being carried out in an organic solvent at a temperature between 0 and 90°C.

11. Process for the preparation of a product of general formula (XI) as defined in claim 10, characterized in that the electrolytic reduction of a product of general formula: 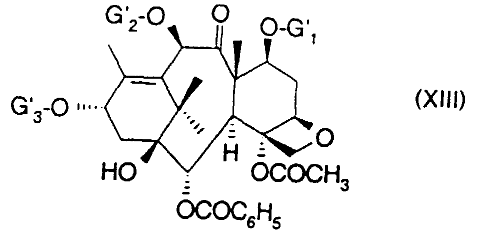 in which G'₁ and G'₂ are defined as above and G'₃ represents a hydrogen atom or a protective group of the hydroxyl functional group identical to G'₁, is carried out, the electrolytic reduction taking place in an electrolyte consisting of a quaternary ammonium salt which is soluble in the organic solvent or in an aqueous/organic solvent at a controlled potential and then, depending on the situation, the protective group G'₃ is selectively replaced by a hydrogen atom or the hydroxyl functional groups in the 7- and 10-positions are selectively protected.
